(19) Europäisches Patentamt
European Patent Office
Office européen des brevets

(11) EP 3 945 092 A1

(12) EUROPEAN PATENT APPLICATION

(43) Date of publication:
**02.02.2022 Bulletin 2022/05**

(21) Application number: **20305860.7**

(22) Date of filing: **27.07.2020**

(51) International Patent Classification (IPC):
***C07D 401/06*** *(2006.01)* ***C07D 401/14*** *(2006.01)*
***C07D 409/06*** *(2006.01)* ***C07D 417/06*** *(2006.01)*
***A61K 31/44*** *(2006.01)* ***A61K 31/425*** *(2006.01)*
***A61K 31/433*** *(2006.01)* ***A61P 25/00*** *(2006.01)*

(52) Cooperative Patent Classification (CPC):
**C07D 401/06; C07D 401/14; C07D 409/06; C07D 417/06**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Etat Français représenté par la Direction Centrale Du Service de Santé des Armées 75015 Paris (FR)**
- **Centre National de la Recherche Scientifique 75016 Paris (FR)**
- **Universite de Rouen Normandie 76130 Mont-Saint-Aignan (FR)**
- **Institut National Des Sciences Appliquées De Rouen 76800 Saint-Etienne-du-Rouvray (FR)**

(72) Inventors:
- **PROBST, Nicolas 76000 Rouen (FR)**
- **BRAÏKI, Anissa 92330 Sceaux (FR)**
- **JEAN, Ludovic 76821 Mont-Saint-Aignan Cedex (FR)**
- **RENARD, Pierre-Yves 75004 Paris (FR)**
- **DIAS, José 91223 Brétigny-sur-Orge (FR)**
- **NACHON, Florian 91223 Brétigny-sur-Orge (FR)**

(74) Representative: **Gevers & Orès**
**Immeuble le Palatin 2**
**3 Cours du Triangle**
**CS 80165**
**92939 Paris La Défense Cedex (FR)**

# (54) CARBALDEHYDE OXIMES AS BUTYRYLCHOLINESTERASE REACTIVATORS

(57) The present invention relates to compounds for their use in the reactivation of butyrylcholinesterase. Such compounds are useful in the treatment or prevention of the intoxication with at least one organophosphorus nerve agent. The invention also relates to pharmaceutical compositions and kits comprising said compounds, and compounds *per se*.



Printed by Jouve, 75001 PARIS (FR)

## Description

The present invention relates to compounds for their use in the reactivation of butyrylcholinesterase. Such compounds are useful in the treatment or prevention of the intoxication with at least one organophosphorus nerve agent. The invention also relates to pharmaceutical compositions and kits comprising said compounds, and compounds *per se.*

Organophosphorous nerve agents (OPNA) are extremely toxic compounds that comprise chemical warfare agents (CWA) including sarin, soman, cyclosarin, tabun, VX and pesticides such as paraoxon, parathion and tetraethyl pyrophosphate (TEPP). Their acute toxicity results from the irreversible inhibition of acetylcholinesterase (AChE) through phosphylation of its catalytic serine, which results in the inability of the enzyme to hydrolyze acetylcholine (ACh). Accumulation of this neurotransmitter at cholinergic synapses occurs, leading to a permanent saturation of the muscarinic and nicotinic receptors which ultimately will result in a cholinergic toxidrome including seizures and respiratory distress. Depending on the class of OPNA and on the administrated dose, death can occur within a few minutes to a day.

Due to the similarity between the chemical precursors of CWA and pesticides, and to the relatively simple chemistry involved in their synthesis, efforts to control the proliferation of these agents have proved of limited success. Illustrative examples include the terrorist attack in the Tokyo subway in 1995, the bombing of Kurd civilians during the Iraq-Iran war in 1988, and that of civilians in Syria, as reported in August 2013. Additionally, despite the international efforts aimed at regulating and lessening the use of these environmentally toxic compounds, ca. 100 different OPNA are still used intensively as pest control agents, with only anecdotal monitoring. This results in about 3,000,000 acute intoxications per year, 200,000 of which lead to death. Moreover, intoxications may also occur during the destruction of chemical weapons stockpiles. Therefore, the development of effective measures to counteract OPNA poisoning remains a challenging issue to protect and treat both civilian and military populations.

The current treatment for OPNA poisoning consists in the administration of a combination of atropine (antimuscarinic agent) and diazepam (anticonvulsant drug), to limit convulsions, and of a standard pyridinium oxime (pralidoxime, trimedoxime, HI-6, obidoxime, or HLo-7) to reactivate AChE. Oximes exert their action on OPNA-inhibited AChE by attacking the phosphorous atom of the phosphylated serine, leading to the removal of the phosphylate and restoration of the enzyme catalytic activity.

These oximes work in the peripheral nervous system, but they have a limited bioavailability in the central nervous system due to their poor blood-brain barrier permeability.

Butyrylcholinesterase (BChE) is a circulating plasma enzyme that is also targeted by OPNAs. BChE is an enzyme that is not very selective and is capable of reacting irreversibly with OPNAs very rapidly, neutralizing them regardless of their nature. It has therefore been developed as a bioscavenger of OPNAs to protect against the effects of acute intoxication. However, in spite of its recognized effectiveness, BChE is a stoichiometric bioscavenger (an equivalent of enzyme is needed to neutralize an equivalent of OPNAs) and therefore requires the injection of a large quantity of enzyme to effectively protect a subject. Despite progress made to improve its production by transgenic organisms or to improve its purification from human plasma, the cost of using this enzyme, especially on a large scale, remains high.

Surprisingly, the inventors have now discovered that compounds of the invention are able to reactivate BChE, thus regenerate the phosphylated enzyme, while standard pyridinium oximes are poorly efficient to reactivate said BChE.

Notably, the compounds of the invention may be used as antidotes against OPNA intoxications or as detoxifying agents against organophosphorus compounds, thanks to their effective and fast reactivation of BChE, alone or in combination with the use of BChE.

The present invention thus concerns a compound of following formula (I):

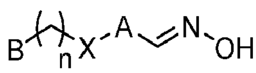 (I)

wherein:

n is an integer from 1 to 6;
at least one of the carbon atoms of

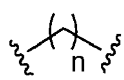

being optionally replaced by an atom chosen from nitrogen and oxygen;
X is a single bond or chosen from -O-, -S- and -NH-;

A is chosen from the group comprising arene diyles and 5 to 6 membered heteroarene diyles, said heteroarene being chosen from the group comprising pyridine, thiophene, thiadiazole, oxathiazole, in particular pyridine;
A is optionally substituted by at least one group R chosen from -OH, $C_1$-$C_6$ alkyl, -O-$C_1$-$C_6$ alkyl, -halogen, notably -Cl, -Br, -F, in particular -OH;
B is chosen from heterocyclic groups with 4 to 10 carbon atoms comprising at least one nitrogen atom, and heteroaryl groups chosen from indole, pyrrazole, imidazole, oxazole, thiazole, oxadiazole and thiadiazole,
the cycle B is optionally fused with at least an arene, in particular a benzene, to form a polycycle B';
the cycle B or B' is optionally substituted by at least one group Z chosen from $C_1$-$C_6$ alkyl, in particular methyl or ethyl; O-$C_1$-$C_6$ alkyl, in particular -OMe; aryl, in particular phenyl; heteroaryl, in particular pyridyl, pyrimidinyl; benzyl; benzhydryl; and -$NR_aR_b$ groups, wherein Ra and Rb are independently chosen from H and $C_1$-$C_6$ alkyls, Ra and Rb being in particular H;
or a stereoisomeric form, a mixture of stereoisomeric forms or a pharmaceutically acceptable salt or solvate form thereof,
for use as a reactivator of human or animal butyrylcholinesterase, in particular human butyrylcholinesterase for the treatment or prevention of an intoxication with at least one organophosphorus nerve agent, said butyrylcholinesterase being prior to treatment or after prevention inhibited by at least one organophosphorus nerve agent.

Said BChE can be from any species. In a particular embodiment, the BChE is mammalian, particularly human.
Human BChE is for instance described in GenBank Gene ID: 590 and GenBank Accession Nos. NM_000055.2 and NP_000046.1 provide examples of amino acid and nucleotide sequences for human BChE.
For the purpose of the invention, the term "pharmaceutically acceptable" is intended to mean what is useful to the preparation of a pharmaceutical composition, and what is generally safe and non-toxic, for a pharmaceutical use.
The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.
The pharmaceutically acceptable salts comprise:

(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminum ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.
It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.
It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.
The term "($C_1$-$C_6$)alkyl", as used in the present invention, refers to a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

The term "aryl", as used in the present invention, refers to an aromatic hydrocarbon group comprising preferably 6 to 10 carbon atoms and comprising one or more, notably 1 or 2, fused rings, such as, for example, a phenyl or naphtyl group. Advantageously, it will be a phenyl group.

The term "heterocycle" as used in the present invention refers in particular to a saturated or unsaturated, more particularly saturated, hydrocarbon monocycle or polycycle (comprising fused, bridged or spiro rings), such as a bicycle, in which one or more, advantageously 1 to 4, and more advantageously 1 or 2, carbon atoms have each been replaced with a heteroatom selected from nitrogen, oxygen, sulphur and silicium atoms, and notably being a nitrogen atom. Advantageously, the heterocycle comprises 5 to 15, notably 5 to 10 atoms in the ring(s). The ring(s) of the heterocycle has/have advantageously 5 or 6 members.

By "heterocyclic groups comprising at least one nitrogen atom", it is in particular meant an azaheterocyclic group, in which, optionally, one or more, advantageously 1 to 3, and more advantageously 1 or 2, carbon atoms have each been further replaced with a heteroatom selected from oxygen, sulphur and silicium atoms.

According to a particular embodiment, the heterocycle is a saturated or unsaturated, more particularly saturated, hydrocarbon monocycle or bicycle (comprising fused, bridged or spiro rings, notably fused rings), each cycle having 5 or 6 members and 1 to 4, notably 1 or 2, carbon atoms having each been replaced with a nitrogen or oxygen atom, notably a nitrogen atom.

A heterocycle can be notably piperidine, piperazine, triazinane, morpholine, thiomorpholine, pyrrolidine, azepane, azocane, azonane, dihydropyridines, dihydropyrimidines (notably 1,2- dihydropyrimidine), dihydropyridazines, dihydropyrazines, dihydrotriazines, tetrahydropyridines, tetrahydropyrimidines, tetrahydropyridazines, tetrahydropyrazines, tetrahydrotriazines, etc.

The term "heteroaryl" as used in the present invention refers in particular to an aromatic heterocycle as defined above.

According to a particular embodiment, the heteroaryl is an aromatic hydrocarbon monocycle or bicycle (i.e. comprising fused rings), each cycle having 5 or 6 members, notably 6 members, and 1 to 4, notably 1 or 2, carbon atoms having each been replaced with a nitrogen or oxygen atom, notably a nitrogen atom.

A heteroaryl can be notably thiophene, furan, pyrrole, imidazole, pyrazole, oxazole, isoxazole, thiazole, isothiazole, triazoles (1,2,3-triazole and 1,2,4-triazole), benzofuran, indole, benzothiophene, benzimidazole, indazole, benzoxazole, benzisoxazole, benzothiazole, benzisothiazole, pyridine, pyrimidine, pyridazine, pyrazine, triazine, quinoline, isoquinoline, quinoxaline, quinazoline, etc.

The term "halogen", as used in the present invention, refers in particular to a fluorine, bromine, chlorine or iodine atom.

The present invention also concerns a compound of formula (I) as defined above for use as a reactivator of human or animal butyrylcholinesterase, in particular human butyrylcholinesterase for the treatment or prevention of a nervous and/or respiratory failure due to an intoxication with at least one organophosphorus nerve agent, said butyrylcholinesterase being prior to treatment of after prevention inhibited by at least one organophosphorus nerve agent.

The present invention also concerns a compound of formula (I) as defined above for use in the treatment or prevention of an blood intoxication with at least one organophosphorus nerve agent.

The present invention also concerns a compound of formula (I) as defined above or a pharmaceutical composition as defined below for use as an in vivo reactivator of human or animal butyrylcholinesterase, in particular human butyrylcholinesterase, said butyrylcholinesterase being prior to use inhibited by at least one organophosphorus nerve agent.

The present invention also concerns the use of a compound of formula (I) as defined above as an in vitro or ex vivo reactivator of human or animal butyrylcholinesterase, in particular human butyrylcholinesterase, said butyrylcholinesterase being prior to use inhibited by at least one organophosphorus nerve agent.

The present invention also concerns a method of treatment or prevention of an intoxication with at least one organophosphorus nerve agent comprising the administration of an effective dose of a compound of formula (I) as defined above or a pharmaceutical composition as defined below to a subject in need thereof.

Said administration can be performed prior to the intoxication, in particular prior to exposure to said organophosphorus nerve agent, or after exposure to said organophosphorus nerve agent but prior to intoxication, said method being in this case a method of prevention.

Said administration can be performed simultaneously to or after the intoxication, said method being in this case a method of treatment.

The present invention also concerns a method of treatment or prevention of a nervous and/or respiratory failure due to intoxication with at least one organophosphorus nerve agent comprising the administration of an effective dose of a compound of formula (I) as defined above or a pharmaceutical composition as defined below to a subject in need thereof.

"Effective dose" notably refers to an amount of a BChE, or BChE and AChE reactivation oxime or oxime containing composition for treatment purposes such that BChE, or BChE and AChE enzymes are reactivated in a

therapeutically meaningful outcome. Determining an effective amount of such an oxime or combination of oximes for administering to a subject in need thereof can be done based on in vitro and/or animal data using routine computational methods well-known in the medical arts. A skilled person in the medical arts can determine what amount is sufficient for a therapeutically meaningful outcome. In one embodiment, the effective amount contains between about 200 g and 0.1 mg of one or more of the disclosed oximes. In another embodiment, the effective amount contains between about 100 g and 500 mg of one or more of the disclosed oximes. In a further embodiment, the effective amount contains between about 50 g and 1 g of one or more of the disclosed oximes, and preferably about 1-5 g thereof. A person of skill in the art will understand that the effective amount will depend on the mass of the subject and the extent of the exposure to the OP. In a still further embodiment, atropine is co-administered with the one or more of the disclosed oximes.

As used herein, the term "patient" or "subject" refers to a warm blooded animal such as a mammal, preferably a human, or a human child, which is afflicted with, or has the potential to be afflicted with one or more diseases and conditions described herein.

A "subject" can also be an animal in need of veterinary treatment, e.g., companion animals (e.g., dogs, cats, and the like), farm animals (e.g., cows, sheep, pigs, horses, and the like) and laboratory animals (e.g., rats, mice, guinea pigs ("GP"), and the like).

A "subject in need" refers to a subject that is at risk for exposure to OPs or that in need of a medical assistance to treat, reverse, counteract, and/or prevent poisoning, damage, and/or other harmful effects of exposure to OPs, whether intentional or accidental.

"Treat" and "treatment," with respect to the exposure of a subject to an organophosphorus compound, refer to a medical intervention which attenuates, prevents, and/or counteracts the effects of such exposure. The foregoing terms can refer to the prophylactic administration of the present compounds and compositions, preferably in the form of a therapeutic composition comprising one or more of the disclosed oximes and one or more pharmaceutical carriers, to a subject at risk of exposure to an organophosphorus compound prior to an anticipated exposure, and/or can refer to the administration of the present compounds and compositions following such exposure.

In a particular embodiment, X represents a single bond.

In a particular embodiment, the residue

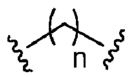

is such as none of its carbon atoms are replaced by an atom chosen from nitrogen and oxygen.

In a particular embodiment, at least one of the carbon atoms of

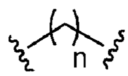

is optionally replaced by an atom chosen from nitrogen and oxygen, when X represents a single bond.

In a particular embodiment, n is 4 or 5.

In a particular embodiment, cycle B is chosen from pyrrolidine, piperidine, azepane, azocane, azonane, piperazine, thiomorpholine and morpholine, said cycle B being optionally substituted and/or fused as defined above, in particular substituted as defined above.

In a particular embodiment, cycle B is fused with a benzene, to form in particular a tetrahydroisoquinoline, more particularly an unsubstituted tetrahydroisoquinoline.

In a particular embodiment, cycle B is an indole, notably an unsubstituted indole.

In a particular embodiment, cycle B is chosen from pyrrazole, imidazole, oxazole, thiazole, oxadiazole and thiadiazole, said cycle B being optionally substituted and/or fused as defined above, in particular fused as defined above.

In a particular embodiment, the compound of formula (I) as defined above is of following formula (Ia):

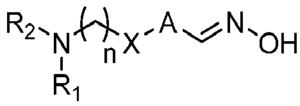

(Ia),

and in particular of following formula (Ib):

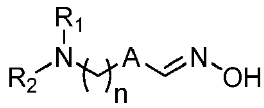 (Ib),

wherein n, X and A are as defined above and $R_1$ and $R_2$ form together with the nitrogen to which they are attached a cycle B chosen from the heterocyclic groups with 4 to 10 carbon atoms and indole.

In a particular embodiment, $-NR_1R_2$ represents a pyrrolidine, piperidine, azepane, azocane, azonane, piperazine, thiomorpholine or morpholine, being optionally substituted and/or fused as defined above, in particular substituted as defined above.

In a particular embodiment, the compound of formula (I) as defined above is of following formula (Ic):

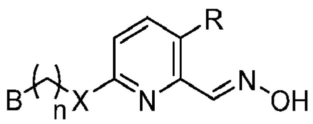 (Ic),

and in particular of following formula (Id):

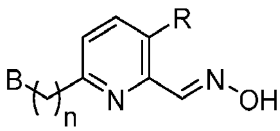 (Id),

wherein n, B, X and R are as defined above.

In a particular embodiment, the compound of formula (I) as defined above is of following formula (Ie):

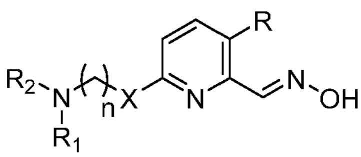 (Ie),

and in particular of following formula (If):

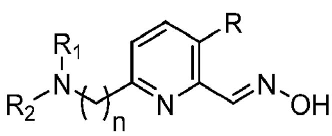 (If),

wherein X, R, $R_1$ and $R_2$ are as defined above, R being in particular -OH.

In a particular embodiment, more particularly about a compound of formula (Ia), (Ib), (Ie) or (If), $-NR_1R_2$ represents a residue chosen from:

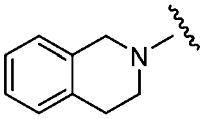 , 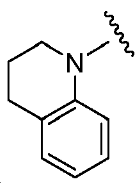 , 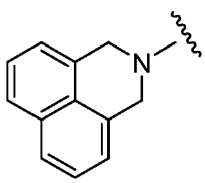 , 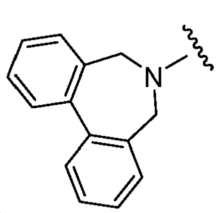 , 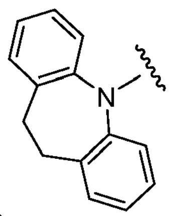 ,

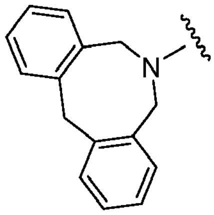, 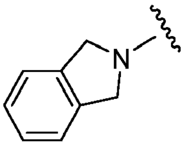, 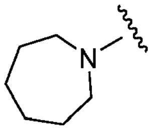, 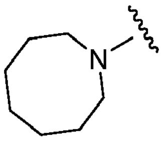, 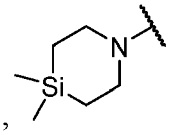, 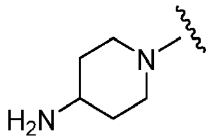,

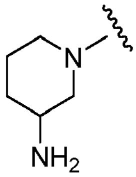, 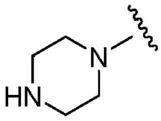, 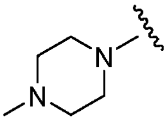, 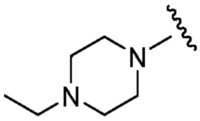, 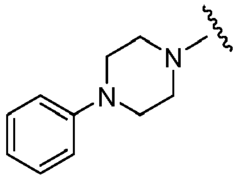,

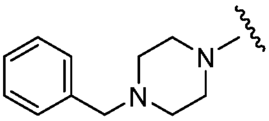, 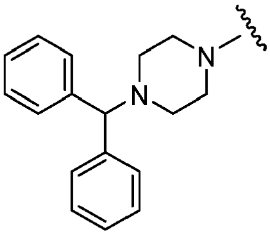, 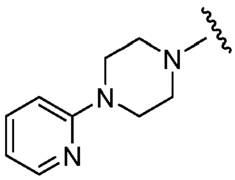 and 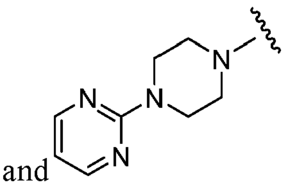.

In a particular embodiment, the compound of formula (I) of the invention is chosen from:

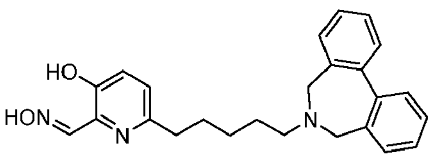, 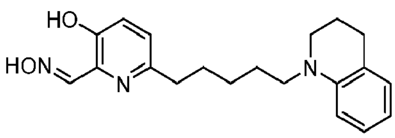,

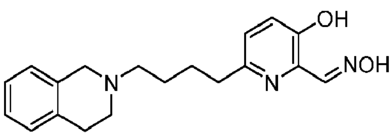, 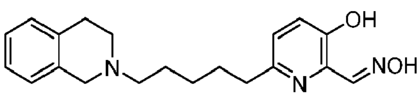,

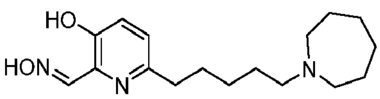,

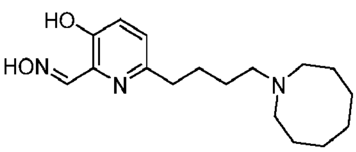, 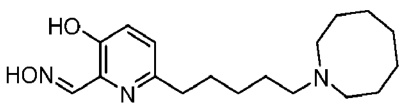,

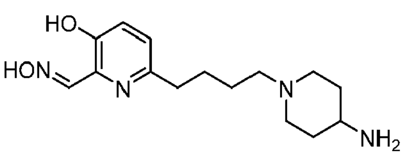, 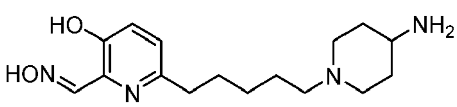,

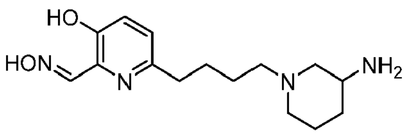,
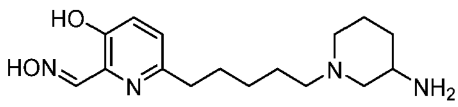,

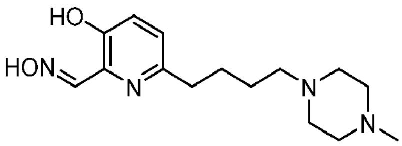,
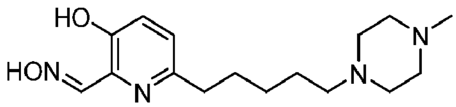,

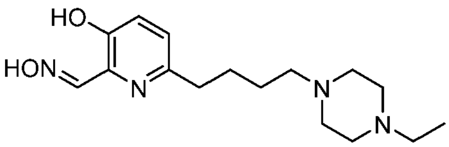,
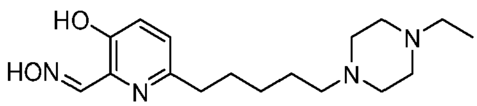,

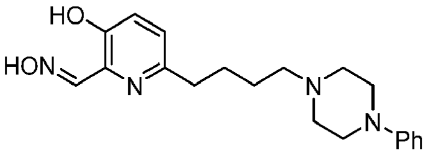,
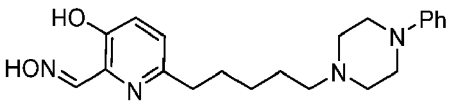,

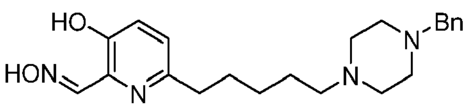,
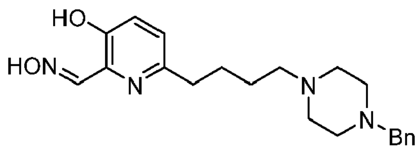,

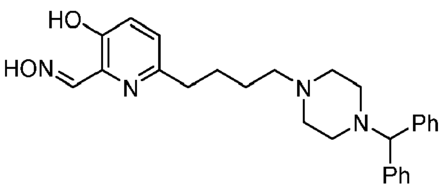,
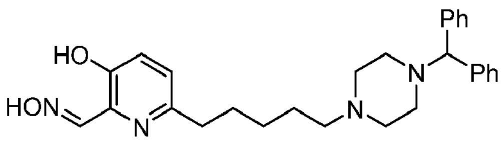,

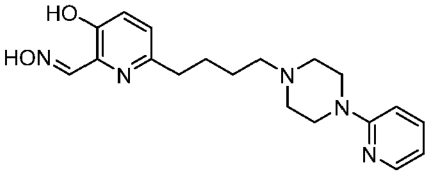,
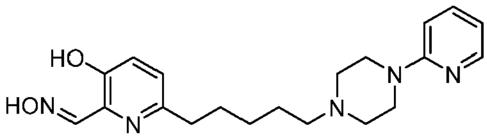,

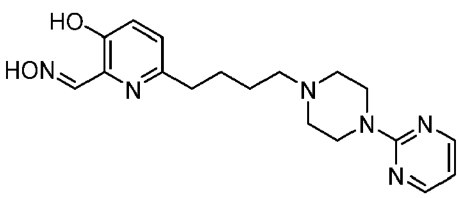,
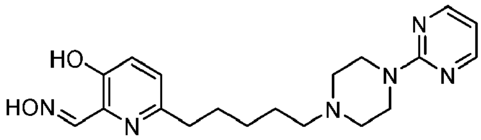,

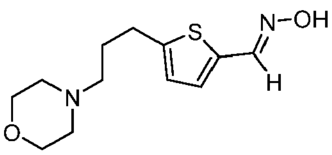,
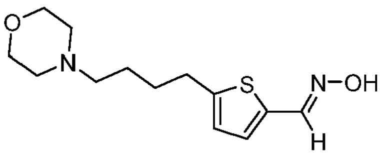,

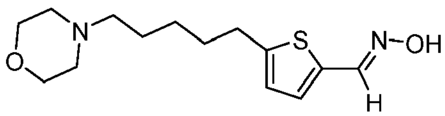,
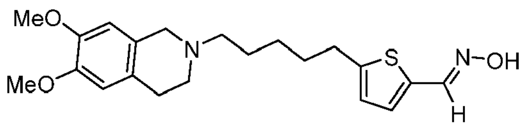,

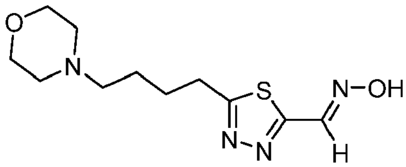,
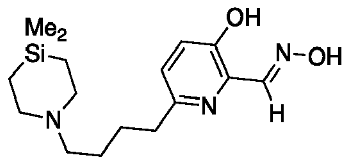,

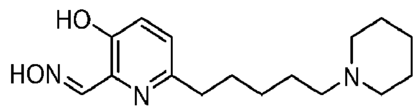,

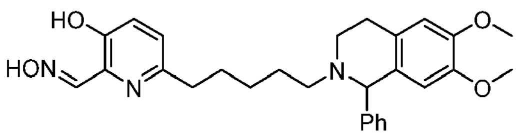,
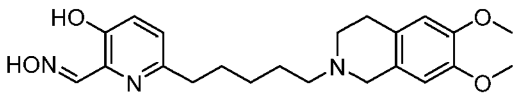,

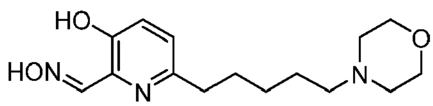,
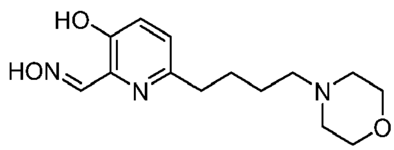,

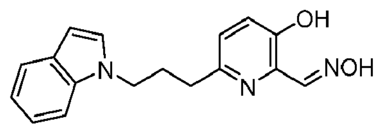.

In a particular embodiment, said organophosphorus nerve agent is selected from warfare agents such as Tammelin esters including *O*-ethyl-*S*-[2-(diisopropylamino)ethyl]methylphosphonothioate (VX), *O*-Ethyl-*S*-2-(diisopropylamino)ethylethylphosphonothiolate (VS), amiton (VG), 2-[ethoxy(ethyl)phosphoryl]sulfanyl-*N*,*N*-diethylethanamine (VE), edemo (VM), *N,N*-diethyl-2-(methyl-(2-methylpropoxy)phosphoryl)sulfanylethanamine (VR) and *O*-cyclopentyl *S*-(2-diethylaminoethyl) methylphosphonothiolate (EA-3148); tabun; sarin; cyclosarin; soman; Novichok agents; and pesticides such as paraoxon, parathion, tetraethyl pyrophosphate (TEPP), dichlorvos, phosmet, malathion, fenitrothion, methyl parathion, tetrachlorvinphos, chlorpyrifos, azamethiphos, diazinon, azinphos-methyl, terbufos.

In another aspect, the invention concerns a compound of following formula (II):

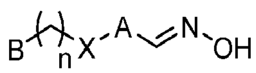 (II)

wherein:

n is an integer from 1 to 6;
at least one of the carbon atoms of

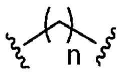

being optionally replaced by an atom chosen from nitrogen and oxygen;
X is a single bond or chosen from -O-, -S- and -NH-;
A is chosen from the group comprising arene diyles and 5 to 6 membered heteroarene diyles, said heteroarene being chosen from the group comprising pyridine, thiophene, thiadiazole, oxathiazole, in particular pyridine;
A is optionally substituted by a group R chosen from -OH, $C_1$-$C_6$ alkyl, -O- $C_1$-$C_6$ alkyl, -halogen, notably -Cl, -Br, -F, in particular -OH;
B is chosen from:

- -$NR_1R_2$ groups wherein $R_1$ and $R_2$ form together with the nitrogen to which they are attached a cycle B chosen from the heterocyclic groups with 4 to 10 carbon atoms; and
- heteroaryl groups chosen from pyrrazole, imidazole, oxazole, thiazole, oxadiazole and thiadiazole;

the cycle B is optionally fused with at least an arene, in particular a benzene, to form a polycycle B';
the cycle B or B' is optionally substituted by at least one group Z chosen from $C_1$-$C_6$ alkyl, in particular methyl or ethyl; O-$C_1$-$C_6$ alkyl, in particular -OMe; aryl, in particular phenyl; heteroaryl, in particular pyridyl, pyrimidinyl; benzyl; benzhydryl; and -$NR_aR_b$ groups, wherein Ra and Rb are independently chosen from H and $C_1$-$C_6$ alkyls, Ra and Rb being in particular H;
with the proviso that, in particular when A is a pyridine:

- cycle B is fused and/or substituted when said cycle B is a piperidine;
- cycle B' is not substituted by one or more O-$C_1$-$C_6$ alkyl groups when B' represents a tetrahydroisoquinoline;

or a stereoisomeric form, a mixture of stereoisomeric forms or a pharmaceutically acceptable salt or solvate form thereof,
for use in the treatment or prevention of an intoxication with at least one organophosphorus nerve agent.

The present invention also concerns a compound of formula (II) as defined above for use in the treatment or prevention of a nervous and/or respiratory failure due to intoxication with at least one organophosphorus nerve agent.
All embodiments mentioned above, for example related to formula (I), (Ia), (Ib), (Ic), (Id), (Ie) or (If), apply here as well, alone or in combination.
In a particular embodiment, the compound of formula (II) of the invention is chosen from:

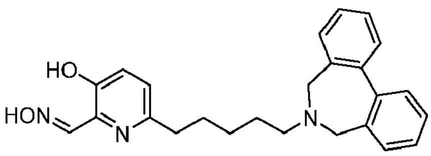,
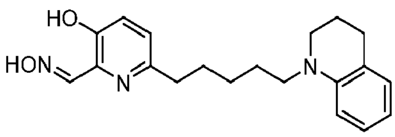,

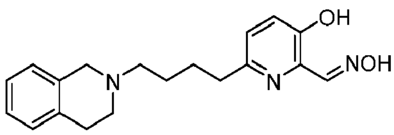,
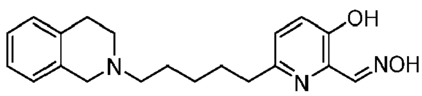,

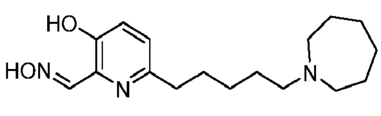,

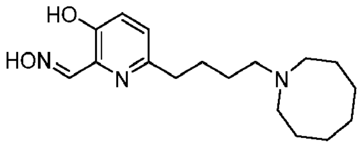, 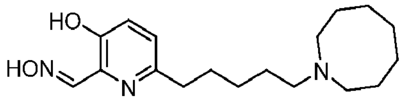,

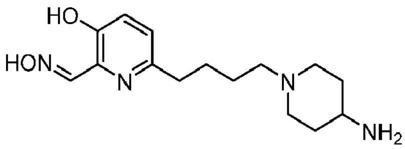, 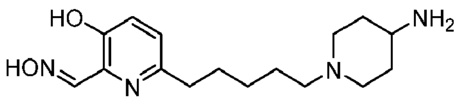,

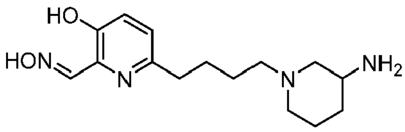, 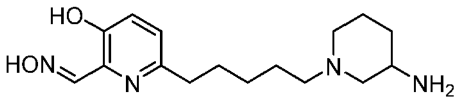,

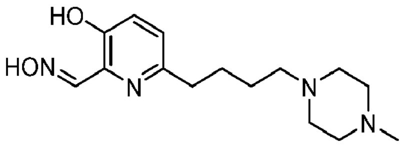, 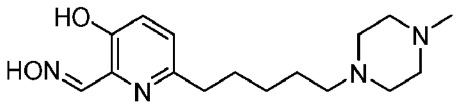,

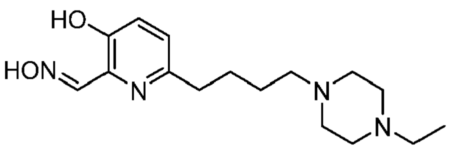, 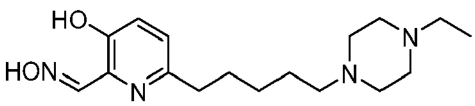,

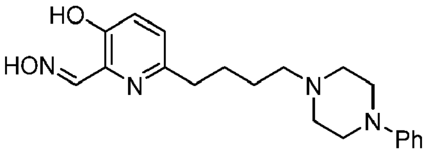, 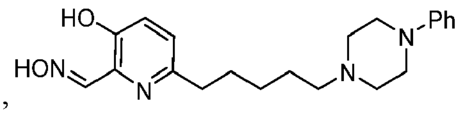,

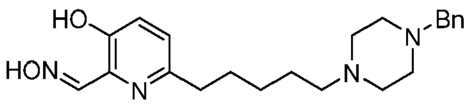, 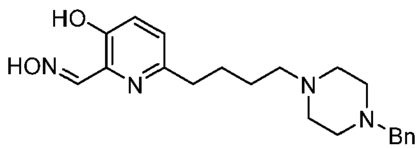,

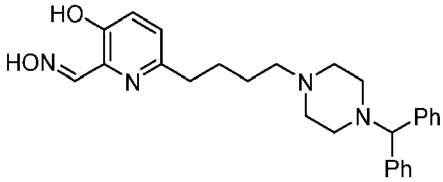, 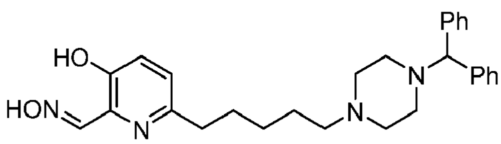,

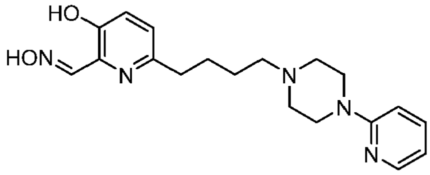, 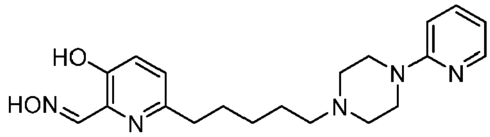,

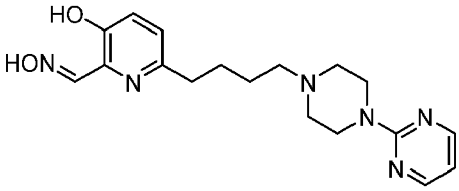, 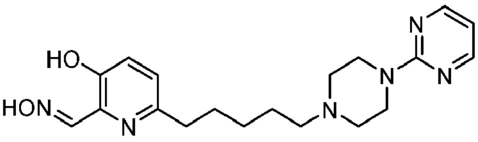,

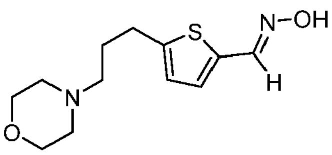, 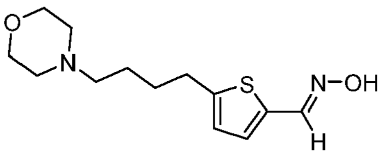,

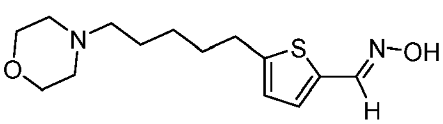, 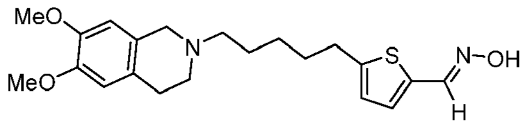,

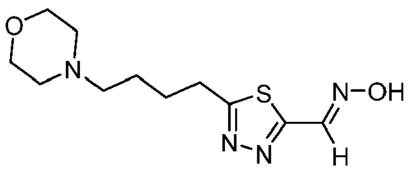, 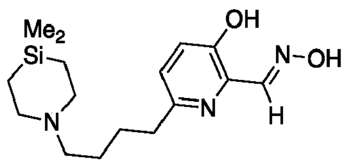.

The present invention also concerns a pharmaceutical composition comprising a compound of formula (II) as defined above in admixture with at least one pharmaceutically acceptable excipient.

All embodiments mentioned above, for example related to formula (I), (Ia), (Ib), (Ic), (Id), (Ie) or (If), apply here as well, alone or in combination.

The compound of formula (III) or the pharmaceutical composition of the present invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of the present invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavouring agents, solubilizers, lubricants, suspending agents, binders, or tablet disintegrating agents; it can also be an encapsulating material.

Tablets, powders, cachets, and capsules can be used as solid dosage forms suitable for oral administration.

Liquid form compositions include solutions, suspensions, and emulsions. For example, sterile water or propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavouring agents, stabilizers, and thickening agents as desired. Aqueous solutions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will according to one embodiment of the present invention include 0.05% to 99% weight (percent by weight), according to an alternative embodiment from 0.10 to 50% weight, of the compound of the present invention, all percentages by weight being based on total composition. A therapeutically effective amount for the practice of the present invention may be determined, by the use of known criteria including the age, weight and response of the individual patient, and interpreted within the context of the disease

which is being treated or which is being prevented, by one of ordinary skills in the art.

The present invention also concerns a compound of formula (II) as defined above.

All embodiments mentioned above, for example related to formula (I), (Ia), (Ib), (Ic), (Id), (Ie) or (If), apply here as well, alone or in combination.

The present invention also concerns a kit comprising a butyrylcholinesterase, in particular a human butyrylcholinesterase, notably selected from wild-type, recombinant, or synthetic butyrylcholinesterase enzymes, and their variants, including peptidomimetics, and a compound of formula (I) or a pharmaceutical composition as defined above.

The present invention also concerns a kit comprising a butyrylcholinesterase, in particular a human butyrylcholinesterase, and a compound of formula (I) or a pharmaceutical composition as defined above, for simultaneous, sequential or separate use in the treatment or prevention of a nervous and/or respiratory failure due to intoxication with at least one organophosphorus nerve agent.

The BChE may be purified and/or concentrated from natural sources (e.g., blood) or may be expressed recombinantly. In a particular embodiment, the BChE is expressed recombinantly. In a particular embodiment, the BChE is isolated. In a particular embodiment, the BChE is purified over a procainamide or huprine affinity column.

For example, the BChE is purified according to the procedure mentioned in WO2015077317 or EP2556057.

By "variant" is in particular meant any protein that has 90, 91, 92, 93, 94, 95, 96, 97, 98, 99% or more of identity with Human BChE, as for instance described in GenBank Gene ID: 590.

Said variants can optionally include nonnatural aminoacids, being in that case peptidomimetics while still defined as variants.

## Definitions

The following terms and expressions contained herein are defined as follows:

As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

## Synthesis

The compounds of the present invention may be prepared in a number of methods well known to those skilled in the art, including, but not limited to those described below, or through modifications of these methods by applying standard techniques known to those skilled in the art of organic synthesis. The appropriate modifications and substitutions will be readily apparent and well known or readily obtainable from the scientific literature to those skilled in the art. In particular, such methods can be found in R.C. Larock, Comprehensive Organic Transformations, Wiley-VCH Publishers, 1999.

All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

The reagents and starting materials are commercially available, or readily synthesized by well-known techniques by one of ordinary skill in the arts.

In particular, the compounds defined above are obtained according to the following general procedure:

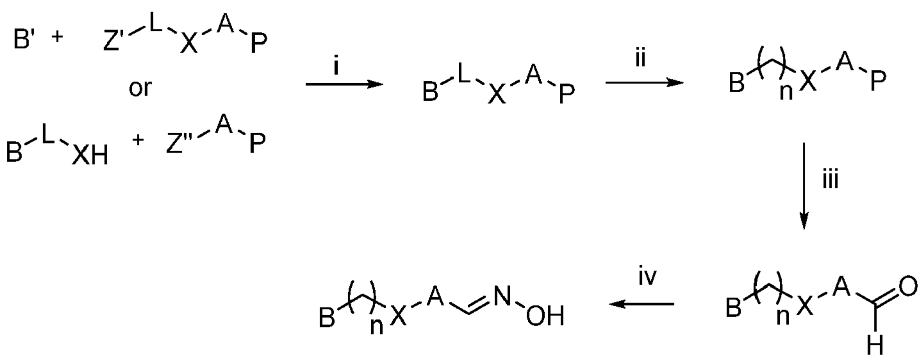


wherein:

- B' is a precursor of the -B group, B' being for example BH;
- Z' and Z" are independently chosen from the leaving groups known from the skilled in the art;
- L is a precursor of the group that links B to X in the final compound;
- P is a protective group of an aldehyde or a precursor of an aldehyde;
- Step iv can be performed using AcONa and $NH_2OH.HCl$.

**EXAMPLES**

**Example 1: Synthesis of compounds of the invention**

**General procedure**

**Sonogashira Cross-coupling reaction - Procedure A**

To an argon degassed solution of bromo aryl (1 equiv.) in dry dichloromethane/triethylamine (2/1, v/v, 0.1 M) and alkyne (0.9-1.1 equiv) were poured $Pd(PPh_3)_4$ (0.05 equiv.) CuI (0.1 equiv.) and the solution was stirred under argon at rt overnight in absence of light. The reaction mixture was concentrated under reduced pressure and purified by flash chromatography (Petroleum ether/EtOAc) to give the desired cross-coupling product.

**Mesyl activation - Procedure B**

Primary alcohol (1 equiv.) was dissolved in dry dichloromethane (0.1 M) and the resulting mixture was cooled down to 0°C. Triethylamine (3 equiv.) followed by Mesyl chloride (1.5 equiv.) were added dropwise in this order. The resulting mixture was stirred 1 to 3 hours at rt. The crude mixture is diluted in dichloromethane, washed with water and brine. Organic layer was dried over magnesium sulfate and concentated under reduced pressure. The mesylate was directly used in following step without further purification.

**Tosyl activation - Procedure C**

Primary alcohol (1 equiv.) was dissolved in dry dichloromethane (0.1 M) under argon atmosphere and the resulting mixture was cooled down to 0 °C. Triethylamine (3 equiv.) followed by tosyl chloride (1.5 equiv.) were added in this order. The resulting mixture was stirred overnight at rt. The crude mixture was concentrated under reduced pressure and purified using flash chromatography (Petroleum ether/EtOAc).

**Nucleophilic substitution - Procedure D**

Activated alcohol (1.0-1.1 equiv) was dissolved in dry MeCN (0.1 M) under argon atmosphere and the resulting mixture was cooled down to 0°C. Amine (1 equiv.) followed by potassium carbonate (1.5 equiv.) were added in this order. The resulting heterogenous mixture was reflux overnight. Salt were removed by filtration and solvent was evaporated under reduced pressure. The crude product was purified using flash chromatography on silica gel.

**Hydrogenation - Procedure E**

Dry benzyl ether was dissolved in MeOH or EtOAc (0.05 M) and the homogenous solution was degassed 20 min using argon. Pearlman's catalyst or Pd/C (20% w/w) was added. After 10 min of degassing using argon, balloon of hydrogen gas was bubbled and the mixture was stirred under hydrogen atmosphere at rt until completion. The crude mixture was filtered over celite®, solvents were removed under vacuum. If needed, the crude can be purified by flash chromatography on silica gel (dichloromethane/MeOH).

**Aldehyde generation (protection -reduction- deprotection) - Procedure F**

Unprotected 3-hydroxypyridine-2-methyl ester was dissolved in dry dichloromethane (0.1 M), then triethylamine (2.2 equiv.) and TBSOTf (1.1 equiv.) were added dropwise at 0 °C. The mixture was stirred at rt until completion (30 min to 3 h). Resulting mixture was diluted in dichloromethane, washed with water, brine and dried under sodium sulfate. Dichloromethane was removed under vacuum giving the crude silyl ether that was dissolved in dry dichloromethane (0.1 M) and cooled down to -78 °C. DIBAL-H (n equiv. = 2 + heteroatom number on ligand*0.5) was added dropwise and the mixture was stirred at -78 °C during 15 min. Methanol was added and the crude mixture was heat up to rt. After dilution with dichloromethane, the organic layer was washed using aqueous NaOH (1 M), water and brine. After drying under sodium sulfate or magnesium sulfate, dichloromethane was removed under vacuum giving the crude silyl ether aldehyde used without further purification in the next step. Crude product was dissolved in dry THF and cooled down to 0 °C. TBAF (1.05-1.1 equiv., 1.0 M in THF) was added dropwise and the mixture was stirred at 0 °C during 30 min. THF was removed under vacuum and the crude product was purified by flash chromatography on silica gel.

**Oxime generation - Procedure G**

Aldehyde was dissolved in dry MeOH under argon. Hydroxylamine hydrochloride (1.1-3 equiv.) and sodium acetate (1.2-3.1 equiv.) were added and the mixture was stirred at rt until completion (3-4 h). Methanol was removed under vacuum and the crude oxime was purified using either normal or reverse phase chromatography.

**Analytic HPLC methods**

**Method A:** Analytic HPLC (Thermo Hypersil GOLD C18 column, 5 μm, 2.1 × 100 mm) with MeOH and trifluoroacetic acid (aq. 0.1%; pH 2.0) as eluents [100% aq. TFA (5 min), then linear gradient from 0% to 100% (45 min) MeOH, then 100% MeOH (5 min)] at a flow rate of 0.25 mL/min. UV/Vis detection (220- 400 nm) was achieved with "Max Plot" (i.e., chromatogram at absorbance maximum for each compound) mode.

**Method B:** Analytic HPLC (Thermo Hypersil GOLD C18 column, 5 μm, 2.1 × 100 mm) with MeCN and trifluoroacetic acid (aq. 0.1%; pH 2.0) as eluents [100% aq. TFA (5 min), then linear gradient from 0% to 100% (45 min) MeCN, then 100% MeCN (5 min)] at a flow rate of 0.25 mL/min. UV/Vis detection (220- 700 nm) was achieved with "Max Plot".

**Method C:** Analytic HPLC (Thermo Hypersil GOLD C18 column, 5 μm, 2.1 × 100 mm) with MeCN and water as eluents [100% water (5 min), then linear gradient from 0% to 100% (45 min) MeCN, then 100% MeCN (5 min)] at a flow rate of 0.25 mL/min. UV/Vis detection (220-700 nm) was achieved with "Max Plot" (i.e., chromatogram at absorbance maximum for each compound) mode.

**Preparative HPLC and SFC methods**

**Method D:** Automated flash purification (Biotage, Interchim - Puriflash® 430, PF-15C18HP/120G) with aq. 0.1% TFA as aq. mobile phase [0% MeCN (5 min), then linear gradient from 0 to 50% (70 min) MeCN] at a flow rate of 35.0 mL/min. Dual UVdetection was achieved at 254 and 330 nm.

**Method E:** Semi-preparative HPLC (Thermo scientific Syncronis C18 250*21mm, 5μm) with isocratic mobile phase (water/MeCN [80/20]), at a flow rate of 19 ml/min. UVdetection was achieved at 290 nm

**Method F:** Supercritic flash chromatography (Princeton SFC-60A, 2 Ethylpyridine, 250*10 mm, 5 μm) was performed with $CO_2$ +10% MeOH as mobile phase at 120 bars and at flow rate of 15.0 mL/min. UVdetection was achieved at 271 nm.

**Methyl 3-hydroxypicolinate - 8**

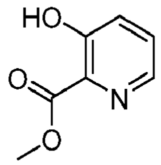

To a suspension of 3-hydroxypicolinic acid (10 g, 72 mmol, 1 equiv.) in methanol (150 mL, 0.5 M) was added dropwise at 0 °C concentrated sulfuric acid (12 mL, 216 mmol, 3 equiv.). The obtained solution was stirred at reflux 6 h. The reaction mixture was concentrated under reduced pressure. The pH was adjusted to 8.5 with an aqueous solution of saturated $NaHCO_3$ and solid $NaHCO_3$. The aqueous layer was extracted with EtOAc and the combined organic layers were washed with brine, dried over magnesium sulfate and concentrated under reduced pressure to give 8 as a white solid (10.9 g, 99%). *Rf* = 0.3 (Petroleum ether/EtOAc 1/1, v/v). m.p. = 74 °C. $^1H$ NMR (300 MHz, $CDCl_3$): δ (ppm) 4.06 (s, 3H, Me), 7.34 (dd, *J* = 3.9, 8.4 Hz, 1H), 7.38 (dd, *J* = 1.5, 8.4 Hz, 1H), 8.38 (dd, *J* = 1.5, 3.9 Hz, 1H), 10.64 (s, 1H). $^{13}C$ NMR (75 MHz, $CDCl_3$) δ (ppm) 53.2, 126.3, 129.8, 130.2, 141.6, 158.9, 169.9. MS (ESI+): *m/z* (%): 154 (100) $[M+H]^+$.

**Methyl 6-bromo-3-hydroxypicolinate** - **9a**

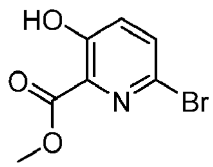

To a solution of methyl 3-hydroxypicolinate **8** (10 g, 65.36 mmol) in osmosed water (0.1 M) with crushed ice at 0 °C, was added portionwise bromine (4 x 1.02 mL every 30 min, 78.4 mmol, 1.2 equiv.) under vigorous stirring. The mixture was vigorously stirred at 0 °C for 1-2 h. The solution was extracted by dichloromethane and the combined organic layers were washed with brine, dried over magnesium sulfate and concentrated under reduced pressure to give **9a** as a white-off solid (15.08 g, 99%). *Rf* = 0.3 (Petroleum ether/EtOAc 6/4, v/v). $^1H$ NMR (300 MHz, $CDCl_3$) δ (ppm) 4.07 (s, 3H), 7.29 (d, *J* = 8.7 Hz, 1H), 7.58 (dd, *J* = 0.3, 8.7 Hz, 1H), 10.72 (br s, 1H). $^{13}C$ NMR (75 MHz, $CDCl_3$): δ (ppm) 53.5, 129.5, 130.0, 130.7, 134.5, 158.5, 169.1. MS (ESI+): *m/z* (%): 234 (85) and 232 (100) $[M+H]^+$.

**Methyl 3-(benzyloxy)-6-bromopicolinate - 4a**

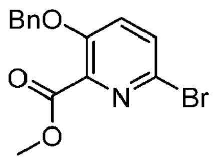

To a solution of methyl 3-hydroxy-6-bromopicolinate **9a** (11.6 g, 49.8 mmol), in acetone (200 mL, 0.25 M) was added successively potassium carbonate (21 g, 149 mmol, 3 equiv.) and benzyl bromide (12 mL, 100 mmol, 2.0 equiv.). The heterogeneous reaction mixture was reflux overnight. Salts were removed by filtration and the crude product was concentrated under reduced pressure. Purification by flash chromatography on silica gel (Petroleum ether/EtOAc 95/5 to 60/40, v/v) afforded the desired product **4a** as a white solid (15.2 g, 95%). *Rf* = 0.4 (Petroleum ether/EtOAc 8/2, v/v). $^1H$ NMR (300 MHz, $CDCl_3$): δ (ppm) 3.97 (s, 3H), 5.21 (s, 2H), 7.24 (d, *J* = 8.7 Hz, 1H), 7.47-7.34 (m, 5H), 7.51 (d, *J* = 8.7 Hz, 1H). $^{13}C$ NMR (75 MHz, $CDCl_3$): δ (ppm) 52.9, 71.2, 125.0, 126.9, 128.4, 128.8, 131.2, 131.4, 135.2, 139.8, 154.0, 164.0. MS (ESI+): *m/z* (%): 324 (85) and 322 (100) $[M+H]^+$.

**3-(benzyloxy)-6-bromopicolinaldehyde - 84**

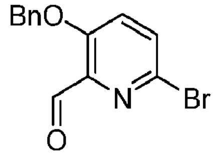

Compound **4a** (1.43 g, 4.46 mmol, 1 equiv.) was dissolved in dry dichloromethane (30 mL), the solution was cooled down to -78 °C, DIBAL (8.92 mL, 8.92 mmol, 2 equiv.) was slowly added. This mixture was stirred at -78 °C for 15 min. MeOH (9 mL) was slowly added, then the mixture was allowed to warm to rt. The reaction mixture was diluted with dichloromethane, washed with aqueous NaOH (1 M), water and brine. Organic layer was dried over magnesium sulfate and evaporated under reduced pressure to give **84** as a white powder (1.3 g, quant.). $^{1}$H NMR (300 MHz, $CDCl_3$) δ (ppm): 10.18 (s, 1H), 7.49 (d, J = 8.8 Hz, 1H), 7.39 - 7.22 (m, 6H), 5.19 (s, 2H). $^{13}$C NMR (75 MHz, $CDCl_3$) δ (ppm): 189.2, 157.0, 143.6, 139.8, 134.7, 129.0, 128.8, 127.3, 126.1, 124.6, 71.1. MS (ESI+): *m/z* (%): 293 [M+H].

**3-(benzyloxy)-6-bromo-2-(1,3-dioxolan-2-yl)pyridine** - **85**

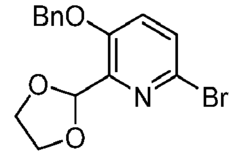

A bed of molecular sieves previously activated was put in a round bottom flask under argon. dichloromethane (80 mL) was added and **84** (1.3 g, 4.45 mmol, 1.0 equiv.) was dissolved. After cooling at 0 °C, ethylene glycol (1.23 mL, 22.25 mmol, 5 equiv.), then dropwise $BF_3.Et_2O$ (1.1 mL, 8.9 mmol, 2 equiv.) were added in this order. The mixture was allowed to warm at rt overnight. Molecular sieves were filtered off and the filtrate was washed with water. Organic layer was dried over magnesium sulfate and evaporated to give **85** as a white powder (1.42 g, 95%). *Rf* = 0.5 (Petroleum ether/EtOAc, 8/2, v/v). $^{1}$H NMR (300 MHz, $CDCl_3$): δ (ppm) 7.39 - 7.21 (m, 7H), 7.05 (d, J = 8.7 Hz, 1H), 6.21 (s, 1H), 5.04 (s, 2H), 4.24 - 4.06 (m, 2H), 4.06 - 3.91 (m, 2H). $^{13}$C NMR (75 MHz, $CDCl_3$): δ (ppm) 65.9, 71.0, 99.9, 123.3, 127.5, 128.5, 128.8, 128.9, 131.4, 135.6, 147.5, 152.9. MS (ESI+): *m/z* (%): 336 (85) and 338 (100) $[M+H]^+$.

**But-3-yn-1-yl 4-methylbenzenesulfonate - 62b**

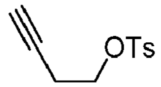

But-3-yn-1-yl 4-methylbenzenesulfonate was synthetized following Procedure B using but-3-yn-1-ol and 62b was isolated after purification by flash chromatography on silica gel (Petroleum ether/EtOAc, 90/10, v/v) as a colorless liquid (*quant.). Rf* = 0.4 (Petroleum ether/EtOAc 9/1, v/v). $^{1}$H NMR (300 MHz, $CDCl_3$) δ (ppm) 7.80 (d, *J* = 8.3 Hz, 2H), 7.35 (d, *J* = 8.0 Hz, 2H), 4.10 (t, *J* = 7.1 Hz, 2H), 2.55 (td, *J* = 7.1, 2.7 Hz, 2H), 2.45 (s, 3H), 1.97 (t, *J* = 2.7 Hz, 1H). $^{13}$C NMR (75 MHz, $CDCl_3$) δ (ppm) 144.9, 133.0, 129.9, 128.1, 82.2, 69.6, 68.8, 27.8, 21.8, 14.8.

**1-(but-3-yn-1-yl)-1,2,3,4-tetrahydroquinoline - 95**

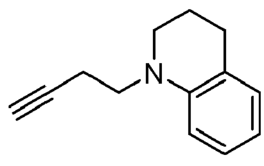

Tosylate **62b** (1.12g, 5 mmol, 1.0 equiv) was dissolved in dry MeCN (80 mL, 0.05 M) under argon atmosphere

and the resulting mixture was cooled down to 0 °C. 1,2,3,4-tetrahydroquinoline (660 μL, 5.05 mmol, 1.01 equiv.) followed by potassium carbonate (800 mg, 5.7 mmol, 1.2 equiv.) were added in this order. The resulting heterogenous mixture was reflux overnight. Salt were removed by filtration and solvent was evaporated under reduced pressure. The crude product was purified by flash chromatography on silica gel (Petroleum ether/EtOAc, 100/0 to 96/4, v/v) to give the 95 as a yellow oil (86 mg, 9%). $^1$H NMR (300 MHz, $CDCl_3$): δ (ppm) 7.13 (t, *J* = 7.1 Hz, 1H), 7.02 (d, *J* = 6.7 Hz, 1H), 6.65 (dd, *J* = 6.7, 7.1 Hz, 2H), 3.56 (d, *J* = 6.2 Hz, 2H), 3.41 (m, 2H), 2.82 (m, 2H), 2.53 (m, 2H), 2.15 - 1.90 (m, 3H). $^{13}$C NMR (75 MHz, $CDCl_3$): δ (ppm) 144.45, 129.48, 127.30, 122.59, 116.14, 110.49, 82.52, 69.63, 50.55, 49.75, 28.20, 22.27, 16.06.

**Methyl 3-(benzyloxy)-6-(4-(3,4-dihydroquinolin-1(2H)-yl)but-1-yn-1-yl)picolinate** - **99**

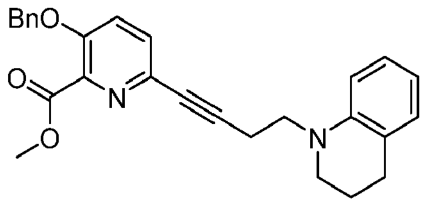

To an degassed solution of bromo pyridine **4a** (149 mg, 0.46 mmol, 1 equiv.) in dry dichloromethane/triethylamine (2/1, v/v, 0.1 M) and **95** (86 mg, 0.46 mmol, 1 equiv.) were poured $Pd(PPh_3)_4$ (27 mg, 0.023 mmol, 0.05 equiv.) and CuI (9.5 mg, 0.046 mmol, 0.1 equiv.). The solution was stirred under argon at rt overnight in absence of light. The reaction mixture was concentrated under reduced pressure and purified by flash chromatography (Petroleum ether/EtOAc, 100/0 to 70/30, v/v) to give 99 as an orange oil (35 mg, 14%). *Rf* = 0.30 (Petroleum ether/EtOAc, 80/20, v/v). $^1$H NMR (300 MHz, $CDCl_3$): δ (ppm) 7.36 (m, 7H), 7.05 (m, 1H), 6.93 (m, 1H), 6.60 (m, 2H), 5.21 (s, 2H), 3.96 (s, 3H), 3.57 (m, 2H), 3.34 (m, 2H), 2.74 (m, 2H), 2.66 (m, 2H), 1.95 (m, 2H). MS (ESI+): *m*/*z* (%): 427 $[M+H]^+$.

**Methyl 3-(benzyloxy)-6-(4-hydroxybut-1-yn-1-yl)picolinate** - **14b**

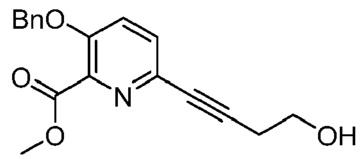

To a solution of methyl 3-(benzyloxy)-6-bromopicolinate **4a** (2.0 g, 6.20 mmol, 1 equiv) in dry dichloromethane (53 mL) and triethylamine (27 mL) was added 4-pentyn-1-ol (485 μL, 6.21 mmol, 1 equiv). The resulting mixture was degassed for 20 min with argon. CuI (200 mg, 0.1 equiv.) and $Pd(PPh_3)_4$ (330 mg, 0.05 equiv.) were then poured and the solution was stirred under argon at rt overnight. The reaction mixture was concentrated under reduced pressure. Purification by flash chromatography (Petroleum ether/EtOAc 7/3 to 3/7, v/v) afforded the desired product **14b** as a yellow oil that crystallized upon standing (1.8 g, 94%). *Rf* = 0.3 (Petroleum ether/EtOAc 4/6, v/v). $^1$H NMR (300 MHz, $CDCl_3$) δ (ppm) 2.66 (t, J = 6.6 Hz, 2H), 3.00 (br s, 1H), 3.82 (t, J = 6.6 Hz, 2H), 3.96 (s, 3H), 4.29-4.35 (m, 1H), 5.18 (s, 2H), 7.28-7.44 (m, 7H). 13C NMR (75 MHz, CDCl3) δ (ppm) 23.8, 52.7, 60.7, 70.9, 80.6, 87.4, 121.9, 126.9, 128.3, 128.8, 130.1, 135.0, 135.5, 139.9, 153.2, 164.8. MS (ESI+): *m*/*z* (%): 312 (100) $[M+H]^+$.

**methyl 3-(benzyloxy)-6-(4-(tosyloxy)but-1-yn-1-yl)picolinate - 59b**

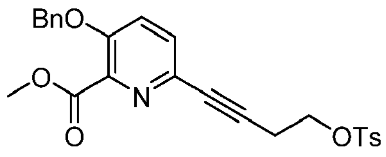

Methyl 3-(benzyloxy)-6-(4-(tosyloxy)but-1-yn-1-yl)picolinate 59b was synthetized following Procedure B using 14b and was isolated after purification by flash chromatography on silica gel (Petroleum ether/EtOAc, 60/40, v/v) as a yellow oil (yield: *quant.). Rf* = 0.42 (Petroleum ether/EtOAc, 60/40, v/v). $^{1}H$ NMR (300 MHz, $CDCl_3$) δ (ppm) 7.84 (d, *J* = 8.3 Hz, 2H), 7.56 - 7.19 (m, 9H), 5.22 (s, 2H), 4.21 (t, *J* = 6.9 Hz, 2H), 3.98 (s, 3H), 2.80 (t, *J* = 6.9 Hz, 2H), 2.44 (s, 3H). $^{13}C$ NMR (75 MHz, $CDCl_3$) δ (ppm) 164.5, 152.9, 152.9, 144.9, 142.8, 141.1. 139.9, 135.2, 134.4, 134.1, 132.4, 129.9, 129.7, 129.5, 128.4, 127.9, 127.6, 126.7, 125.0, 121.4, 83.6, 80.9, 70.4, 67.1, 52.4, 21.3, 20.0.

**Methyl 3-(benzyloxy)-6-(5-(3,4-dihydroquinolin-1(2H)-yl)pent-1-yn-1-yl)picolinate - 100**

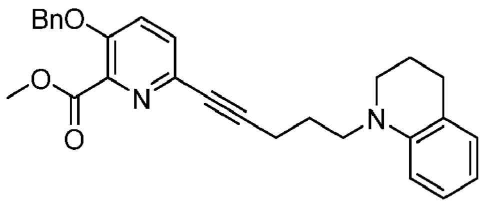

Tosylate **59b** (993 mg, 2.07 mmol, 1.0 equiv) was dissolved in dry MeCN (20 mL, 0.1 M) under Argon atmosphere and the resulting mixture was cooled down to 0 °C. 1,2,3,4-tetrahydroquinoline (300 μL, 2.5 mmol, 1.2 equiv.) followed by potassium carbonate (575 mg, 5.7 mmol, 2 equiv.) were added in this order. The resulting heterogenous mixture was reflux overnight. Salt were removed by filtration and solvent was evaporated under reduced pressure. The crude product was purified by flash chromatography on silica gel (Petroleum ether/EtOAc, 100/0 to 50/50, v/v) to give the 95 as an orange oil (212 mg, 24%). *Rf* = 0.5 (Petroleum ether/EtOAc, 50/50, v/v). $^{1}H$ NMR (300 MHz, $CDCl_3$): δ (ppm) 7.49 - 7.28 (m, 7H), 7.03 (td, *J* = 8.3, 1.7 Hz, 1H), 6.94 (dd, *J* = 7.3, 1.4 Hz, 1H), 6.62 (d, *J* = 8.2 Hz, 1H), 6.56 (td, *J* = 7.3, 1.0 Hz, 1H), 5.20 (s, 2H), 3.97 (s, 3H), 3.46 - 3.35 (m, 2H), 3.33 - 3.24 (m, 2H), 2.75 (t, *J* = 6.4 Hz, 2H), 2.48 (t, *J* = 7.0 Hz, 2H), 2.01 - 1.80 (m, 4H). $^{13}C$ NMR (75 MHz, $CDCl_3$): δ (ppm) 164.90, 152.99, 145.26, 140.21, 135.59, 135.44, 129.99, 129.25, 128.80, 128.29, 127.14, 126.97, 122.41, 121.84, 115.63, 110.63, 89.73, 79.86, 70.89, 52.73, 50.48, 49.79, 28.20, 25.18, 22.31, 17.13. MS (ESI+): *m/z* (%): 441 $[M+H]^+$.

**Methyl 6-(5-(3,4-dihydroquinolin-1(2H)-yl)pentyl)-3-hydroxypicolinate -101**

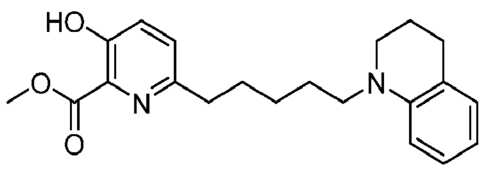

Benzyl ether **100** (210 mg, 0.5 mmol, 1.0 equiv.) was dissolved in MeOH (0.05 M) and the homogenous solution was degassed 20 min using argon. Pearlman's catalyst (42 mg, 20% w/w) was added. After 10 min of degassing using Argon, balloon of hydrogen gas was bubbled and the mixture was stirred under hydrogen atmosphere at rt until completion. The crude mixture was filtered over celite®, solvents were removed under vacuum to give **101** as a red oil (171 mg, 96%). $^{1}H$ NMR (300 MHz, $CDCl_3$): δ (ppm) 7.34 - 7.25 (m, 2H), 7.04 (td, *J* = 8.2, 1.7 Hz, 1H), 6.99 - 6.87 (m, 1H), 6.58 - 6.51 (m, 2H), 4.05 (s, 3H), 3.25 (dd, *J* = 13.1, 6.9 Hz, 4H), 2.86 - 2.71 (m, 4H), 1.99 - 1.88 (m, 2H), 1.70 (ddt, *J* = 23.0, 11.7, 7.6 Hz, 6H), 1.47 - 1.35 (m, 2H). $^{13}C$ NMR (75 MHz, $CDCl_3$): δ (ppm) 170.18, 157.20, 153.95, 145.28, 129.17, 129.14, 128.84, 127.04, 126.64, 122.19, 115.28, 110.44, 53.14, 51.34, 49.47, 37.62, 29.99, 28.21, 26.92, 26.09, 22.26. MS (ESI+): *m/z* (%): 355 $[M+H]^+$.

**6-(5-(3,4-dihydroquinolin-1(2H)-yl)pentyl)-3-hydroxypicolinaldehyde - 102**

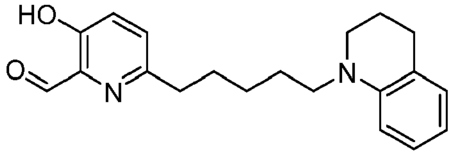

Compound **101** (169 mg, 0.48 mmol, 1.0 equiv.) was dissolved in dry dichloromethane (10 mL, 0.05 M), then triethylamine (192 μL, 1.2 mmol, 2.5 equiv.) and TBSOTf (143 μL, 0.53 mmol, 1.1 equiv.) were added dropwise at 0°C. The mixture was stirred at rt 1 h. Resulting mixture was diluted in dichloromethane, washed with water, brine and dried under sodium sulfate. Dichloromethane was removed under vacuum giving the crude silyl ether that was dissolved in dry dichloromethane (10 mL, 0.05 M) and cooled down to - 78°C. DIBAL-H (1.2 mL, 1.2 mmol, 2.5 equiv.) was added dropwise and the mixture was stirred at -78°C during 15 min. Methanol (1.2 mL) was added and the crude mixture was heat up to rt. After dilution with dichloromethane, the organic layer was washed using aqueous NaOH (1M), water and brine. After drying under sodium sulfate, dichloromethane was removed under vacuum giving the crude silyl ether aldehyde moiety that was dissolved in dry THF (20 mL) and cooled down to 0°C. TBAF (350 μL, 1 equiv.) was added dropwise and the mixture was stirred at 0 °C during 30 min. THF was removed under vacuum and the crude was purified by flash chromatography on silica gel (Petroleum ether/EtOAc, 80/20, v/v) to give the title product as a red oil (81 mg, 52% over 3 steps). *Rf* = 0.62 (Petroleum ether/EtOAc, 80/20, v/v). $^1$H NMR (300 MHz, $CDCl_3$): δ (ppm) 10.08 (s, 1H), 7.32 (d, *J* = 1.9 Hz, 2H), 7.07 (t, *J* = 7.7 Hz, 1H), 6.97 (d, *J* = 7.7 Hz, 1H), 6.68 - 6.53 (m, 2H), 3.29 (dd, *J* = 12.5, 5.5 Hz, 4H), 2.82 (dt, *J* = 12.2, 6.8 Hz, 4H), 1.97 (dt, *J* = 12.6, 6.2 Hz, 2H), 1.83 (dt, *J* = 15.6, 7.9 Hz, 2H), 1.76 - 1.60 (m, 2H), 1.56 - 1.38 (m, 2H). MS (ESI+) *m/z* = 325 $[M+H]^+$.

**AB-392 - 93**

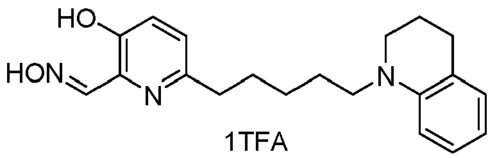

1TFA

Aldehyde **102** (81 mg, 0.25 mmol, 1.0 equiv.) was dissolved in dry MeOH under argon. Hydroxylamine hydrochloride (19 mg, 0.26 mmol, 1.1 equiv.) and sodium acetate (25 mg, 0.3 mmol, 1.2 equiv.) were added and the mixture was stirred at rt during 4h. Methanol was removed under vacuum and the crude oxime was purified using reverse phase chromatography (method D) to give the TFA salt of the title compound as a white-off solid (81.6 mg, 72%). $^1$H NMR (300 MHz, $CDCl_3$): δ (ppm) 10.57 (br s, 3H), 8.37 (s, 1H), 8.15 (d, *J* = 3.7 Hz, 1H), 7.47 (dd, *J* = 11.3, 4.2 Hz, 1H), 7.14 (d, *J* = 8.4 Hz, 1H), 6.96 (d, *J* = 8.4 Hz, 1H), 6.67 - 6.55 (m, 2H), 3.65-3.50 (m, 4H), 2.77-2.61 (m, 6H), 2.51 - 2.39 (m, 2H), 1.76 - 1.53 (m, 4H), 1.42-1.28 (m, 2H). $^{13}$C NMR (75 MHz, $CDCl_3$): δ (ppm) 159.25, 153.13, 152.56, 147.82, 137.79, 135.30, 124.52, 123.68, 113.74, 107.45, 58.40, 52.63, 44.62, 37.02, 29.89, 27.04, 25.8, 22.78. HRMS (ESI+): *m/z* calcd for C20H26N3O2 340.2025; found: 340.2027. HPLC (method B): $t_R$ = 15.70 min, purity = 98.34%. MS (ESI+): *m/z* (%): 340 $[M+H]^+$.

**2-(but-3-yn-1-yl)-1,2,3,4-tetrahydroisoquinoline -104**

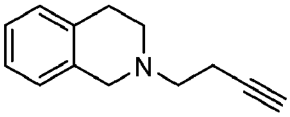

But-3-yn-1-yl methanesulfonate (740 mg, 5 mmol, 2.0 equiv.) was dissolved in dry MeCN (50 mL, 0.1 M) under argon atmosphere and the resulting mixture was cooled down to 0 °C. 1,2,3,4-tetrahydroisoquinoline (330 μL, 2.5 mmol, 1 equiv.) followed by potassium carbonate (766 mg, 5.5 mmol, 2.2 equiv.) were added in this order. The resulting heterogenous mixture was reflux overnight. Salt were removed by filtration and solvent was evaporated under reduced pressure. The crude product was purified by flash chromatography (Petroleum ether/EtOAc, 80/20, v/v) to give the title compound as a yellow oil (408.4 mg, 88%). *Rf* = 0.36 (Petroleum ether/EtOAc, 80/20, v/v). $^1$H NMR (300 MHz, $CDCl_3$):

δ (ppm) 7.24 - 7.06 (m, 3H), 7.06 - 6.97 (m, 1H), 3.69 (s, 2H), 2.91 (t, *J* = 5.8 Hz, 2H), 2.85 - 2.70 (m, 4H), 2.56 - 2.43 (m, 2H), 2.00 (t, *J* = 2.7 Hz, 1H). $^{13}C$ NMR: (75 MHz, $CDCl_3$) δ (ppm) 134.60, 134.24, 128.80, 126.71, 126.30, 125.76, 82.85, 69.24, 56.85, 55.85, 50.82, 29.10, 17.34. MS (ESI+): *m/z* (%): 186 $[M+H]^+$.

**Methyl 3-(benzyloxy)-6-(4-(3,4-dihydroisoquinolin-2(1H)-yl)but-1-yn-1-yl)picolinate -105**

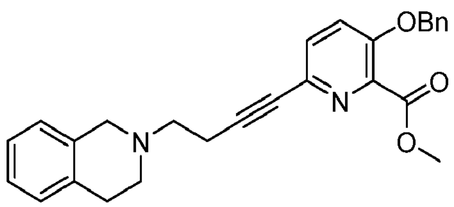

To an argon degassed solution of **4a** (509 mg, 1.58 mmol, 1 equiv.) in dry dichloromethane/triethylamine (2/1, v/v, 0.1M) and alkyne 104 (293 mg, 1.58 mmol, 1 equiv.) were poured $Pd(PPh_3)_4$ (91 mg, 0.079 mmol, 0.05 equiv.) CuI (30 mg, 0.158 mmol, 0.1 equiv.) and the solution was stirred under argon at rt overnight in absence of light. The reaction mixture was concentrated under reduced pressure and purified by flash chromatography (Petroleum ether/EtOAc, 40/60, v/v) to give the desired cross-coupling product **105** as an orange oil (300 mg, 45 %, trace of $PPh_3$ remaining). *Rf* = 0.25 (Petroleum ether/EtOAc, 1/1, v/v). MS (ESI+): *m/z* = 427 $[M+H]^+$. $^1H$ NMR (300 MHz, $CDCl_3$): δ (ppm) 7.51 - 7.31 (m, 7H), 7.18 - 6.97 (m, 4H), 5.20 (s, 2H), 3.96 (s, 3H), 3.70 (s, 2H), 2.95 - 2.76 (m, 5H), 2.76 - 2.63 (m, 2H), 1.26 (t, *J* = 7.1 Hz, 1H). $^{13}C$ NMR (75 MHz, $CDCl_3$): δ (ppm) 164.90, 153.06, 140.23, 135.62, 135.43, 134.57, 134.19, 130.05, 128.83, 128.76, 128.33, 127.00, 126.68, 126.26, 125.73, 121.86, 88.50, 80.16, 70.94, 56.63, 55.78, 52.76, 50.80, 29.14, 18.18.

**Methyl 6-(4-(3,4-dihydroisoquinolin-2(1H)-yl)butyl)-3-hydroxypicolinate** - **106**

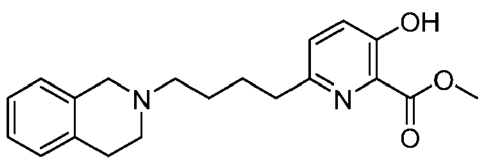

Compound **105** (279 mg, 0.63 mmol, 1.0 equiv.) was dissolved in MeOH (13 mL, 0.05 M) and the homogenous solution was degassed 20 min using argon. Pearlman's catalyst (56 mg, 20% w/w) was added. After 10 min of degassing using Argon, balloon of hydrogen gas was bubbled and the mixture was stirred under hydrogen atmosphere at rt overnight. The crude mixture was filtered over celite®, solvents were removed under vacuum to give compound **106** as a brown oil (203 mg, 97%). MS (ESI+): *m/z* (%): 341 $[M+H]^+$. $^1H$ NMR (300 MHz, $CDCl_3$): δ (ppm) 10.59 (s, 1H), 7.34 - 7.27 (m, 2H), 7.15 - 6.98 (m, 4H), 4.02 (s, 3H), 3.48 (s, 2H), 2.99 - 2.68 (m, 6H), 2.59 (s, 2H), 1.83 - 1.62 (m, 4H). $^{13}C$ NMR (75 MHz, $CDCl_3$): δ (ppm) 169.69, 156.77, 153.33, 133.60, 133.54, 131.72, 131.59, 128.82, 128.43, 128.24, 126.26, 126.20, 125.92, 125.36, 57.43, 55.37, 52.70, 50.36, 36.96, 28.22, 27.46, 26.04.

**6-(4-(3,4-dihydroisoquinolin-2(1H)-yl)butyl)-3-hydroxypicolinaldehyde - 107**

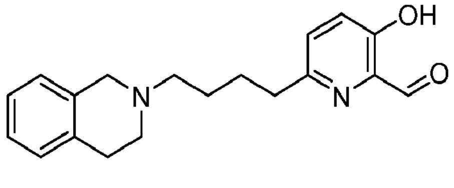

Compound **106** (690 mg, 2.03 mmol, 1 equiv.) was dissolved in dry dichloromethane (20 mL, 0.1 M), then triethylamine (850 μL, 6.09 mmol, 3 equiv.) and TBSOTf (550 μL, 3.05, 2.5 equiv.) were added dropwise at 0°C. The

mixture was stirred at rt 3 h. Resulting mixture was diluted in dichloromethane, washed with water, brine and dried under sodium sulfate. Dichloromethane was removed under vacuum giving the crude silyl ether. $^1$H NMR (300 MHz, $CDCl_3$) δ (ppm) 7.18 - 6.98 (m, 6H), 3.92 (s, 3H), 3.60 (s, 2H), 2.89 (t, *J* = 5.8 Hz, 2H), 2.85 - 2.77 (m, 2H), 2.58 - 2.46 (m, 4H), 1.85 - 1.58 (m, 4H), 1.02 - 0.97 (m, 9H), 0.22 - 0.18 (m, 6H).

The crude silyl ether (880 mg, 2.03 mmol, 1.0 equiv.) was dissolved in dry dichloromethane (20 mL, 0.1 M) and cooled down to -78°C. DIBAL-H (5.07 mL, 5.07 mmol, 2.5 equiv.) was added dropwise and the mixture was stirred at -78°C during 15 min. Methanol (5 mL) was added and the crude mixture was heat up to rt. After dilution with dichloromethane, the organic layer was washed using aqueous NaOH (1 M), water and brine. After drying under sodium sulfate, dichloromethane was removed under vacuum giving the crude silyl ether aldehyde moiety used without purification in the next step. $^1$H NMR (300 MHz, $CDCl_3$): δ (ppm) 10.33 (s, 1H), 7.18 (d, *J* = 8.5 Hz, 2H), 7.12 - 6.98 (m, 4H), 3.60 (s, 2H), 2.92 - 2.82 (m, 4H), 2.71 (t, *J* = 5.9 Hz, 2H), 2.57 - 2.48 (m, 2H), 1.85 - 1.62 (m, 4H), 1.01 (s, 9H), 0.25 (s, 6H).

Crude product was dissolved in dry THF (20 mL, 0.1 M) and cooled down to 0 °C. TBAF (2.44 mL, 2.44 mmol, 1.2 equiv.) was added dropwise and the mixture was stirred at 0 °C during 30 min. THF was removed under vacuum and the crude was purified using silica gel flash chromatography (dichloromethane/MeOH, 95/5, v/v) to give the title compound as an orange oil (180 mg, 28%). *Rf* = 0.28 (dichloromethane/MeOH, 95/5, v/v). $^1$H NMR (300 MHz, $CDCl_3$): δ (ppm) 10.02 (s, 1H), 7.31 - 7.20 (m, 2H), 7.15 - 6.94 (m, 4H), 3.61 (s, 2H), 2.94 - 2.76 (m, 4H), 2.72 (t, *J* = 5.9 Hz, 2H), 2.61 - 2.50 (m, 2H), 1.87 - 1.60 (m, 4H). $^{13}$C NMR (75 MHz, $CDCl_3$): δ (ppm) 198.59, 156.87, 154.65, 135.63, 134.61, 134.17, 129.64, 128.54, 126.47, 126.25, 126.03, 125.51, 58.01, 56.08, 50.88, 37.06, 28.95, 27.47, 26.64. MS (ESI+): *m*/*z* (%): 311 $[M+H]^+$.

**AB-558** - **90**

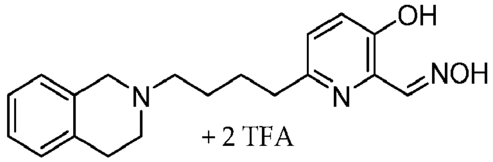

Aldehyde **107** (179 mg, 0.58 mmol, 1.0 equiv.) was dissolved in dry methanol (5.8 mL, 0.1 M). Hydroxylamine hydrochloride (84 mg, 1.2 mmol, 2 equiv.) and sodium acetate (123 mg, 1.5 mmol, 2.5 equiv.) were added and the mixture was stirred at rt during 3 h. Concentration until dryness and subsequent reverse phase purification (method D) gave TFA salt of title compound as a white-off solid (242 mg, 75%). $^{13}$C NMR (75 MHz, $CD_3OD$) δ (ppm) 161.13 (q, J = 37 Hz), 154.26, 152.30, 148.51, 135.30, 132.10, 129.86, 129.46, 129.10, 128.85, 128.27, 127.85, 126.62, 56.96, 54.20, 51.14, 35.37, 27.72, 26.33, 24.64. HPLC (method B): $t_R$ = 18.5 min (> 99%). MS (ESI+): *m*/*z* (%): 326 (100) $[M+H]^+$. HRMS (ESI+): *m*/*z* calcd for C19H24N3O2 326.1869; found: 326.1863.

**Methyl 3-(benzyloxy)-6-(5-(tosyloxy)pent-1-yn-1-yl)picolinate** - **59a**

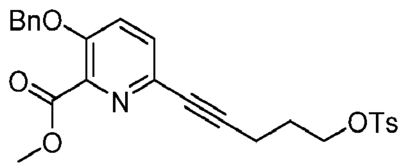

Methyl 3-(benzyloxy)-6-(5-((methylsulfonyl)oxy)pent-1-yn-1-yl)picolinate was synthetized following **Procedure B** using **14a** and **59a** was isolated after purification by flash chromatography on silica gel (Petroleum ether/EtOAc, 60/40, v/v) as a yellow oil (yield: *quant.*). *Rf* = 0.4 (Petroleum ether/EtOAc, 60/40, v/v). $^1$H NMR (300 MHz, $CDCl_3$) δ (ppm) 7.79 (d, *J* = 8.3 Hz, 2H), 7.47 - 7.27 (m, 9H), 5.21 (s, 2H), 4.17 (t, *J* = 6.1 Hz, 2H), 3.96 (s, 3H), 2.47 (t, *J* = 7.0 Hz, 2H), 2.39 (s, 3H), 1.98 - 1.84 (m, 2H).

**Methyl 3-(benzyloxy)-6-(5-(3,4-dihydroisoquinolin-2(1H)-yl)pent-1-yn-1-yl)picolinate - 108**

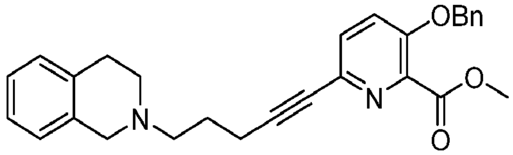

Tosylate **59a** (992 mg, 2.07 mmol, 1.0 equiv) was dissolved in dry MeCN (20 mL, 0.1 M) under Argon atmosphere and the resulting mixture was cooled down to 0°C. 1,2,3,4-tetrahydroisoquinoline (300 μL, 2.28 mmol, 1.1 equiv.) followed by potassium carbonate (575 g, 4.14 mmol, 2 equiv.) were added in this order. The resulting heterogenous mixture was reflux overnight. Salt were removed by filtration and solvent was evaporated under reduced pressure. The crude product was purified by chromatography on silica gel (dichloromethane/MeOH, 98/2, v/v) to afford **108** as a yellow oil (662 mg, 72%). *Rf* = 0.24 (dichloromethane/MeOH, 98/2, v/v). $^1H$ NMR (300 MHz, $CDCl_3$): δ (ppm) 7.45 - 7.24 (m, 6H), 7.14-6.99 (m, 4H), 5.20 (s, 2H), 3.96 (s, 3H), 3.64 (s, 2H), 2.90 (t, *J* = 5.9 Hz, 2H), 2.74 (t, *J* = 5.9 Hz, 2H), 2.67 - 2.59 (m, 2H), 2.51 (dd, *J* = 12.1, 4.9 Hz, 2H), 1.96 - 1.84 (m, 2H). $^{13}C$ NMR (75 MHz, $CDCl_3$): δ (ppm) 164.94, 152.98, 140.18, 135.65, 134.93, 134.46, 132.10, 130.06, 128.61, 127.01, 126.67, 126.17, 125.66, 121.90, 90.26, 79.58, 70.95, 57.37, 56.28, 52.76, 51.02, 29.24, 26.10, 17.50

**Methyl 6-(5-(3,4-dihydroisoquinolin-2(1H)-yl)pentyl)-3-hydroxypicolinate - 109**

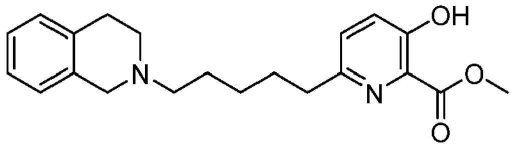

Compound **108** (660 mg, 1.5 mmol, 1.0 equiv.) was dissolved in MeOH (30 mL, 0.05 M) and the homogenous solution was degassed 20 min using argon. Pearlman's catalyst (132 mg, 20% w/w) was added. After 10 min of degassing using Argon, balloon of hydrogen gas was bubbled and the mixture was stirred under hydrogen atmosphere at rt until completion. The crude mixture was filtered over celite®, solvents were removed under vacuum to afford the product as orange oil (510 mg, 97%). $^1H$ NMR (300 MHz, $CDCl_3$): δ (ppm) 7.25 (d, *J* = 4.8 Hz, 2H), 7.11 - 6.95 (m, 4H), 4.01 (s, *J* = 6.3 Hz, 3H), 3.61 (s, 2H), 2.88 (t, *J* = 5.8 Hz, 2H), 2.83 - 2.75 (m, 2H), 2.71 (t, *J* = 5.9 Hz, 2H), 2.54 - 2.46 (m, 2H), 1.80 - 1.69 (m, 2H), 1.68 - 1.58 (m, 2H), 1.48 - 1.35 (m, 2H). $^{13}C$ NMR (75 MHz, $CDCl_3$): δ (ppm) 170.01, 157.04, 153.86, 134.47, 134.08, 129.01, 128.58, 126.45, 125.99, 125.47, 58.07, 55.97, 52.97, 50.73, 37.46, 29.87, 28.83, 27.13, 26.82.

**6-(5-(3,4-dihydroisoquinolin-2(1H)-yl)pentyl)-3-hydroxypicolinaldehyde - 110**

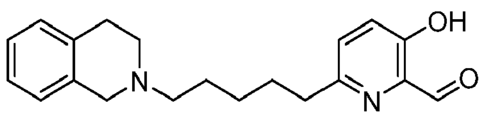

Compound **109** (462 mg, 1.65 mmol, 1.0 equiv.) was dissolved in dry dichloromethane (0.1 M), then triethylamine (500μL, 3.5 mmol, 2.1 equiv.) and TBSOTf (360μL, 1.98 mmol, 1.2 equiv.) were added dropwise at 0 °C. The mixture was stirred at rt during 4 h. Resulting mixture was diluted in dichloromethane, washed with water, brine and dried under sodium sulfate. dichloromethane was removed under vacuum giving the crude silyl ether that was dissolved in dry dichloromethane (0.1 M) and cooled down to -78°C. DIBAL-H (4.2 mL, 4.2 mmol, 2.5 equiv.) was added dropwise and the mixture was stirred at -78°C during 15 min. Methanol was added and the crude mixture was heat up to rt. After dilution with dichloromethane, the organic layer was washed using aqueous NaOH (1 M), water and brine. After drying under sodium sulfate or magnesium sulfate, dichloromethane was removed under vacuum giving the crude silyl ether aldehyde moiety used without purification in the next step. Crude reduced silyl ether was dissolved in dry THF (0.1 M)

and cooled down to 0°C. TBAF (1.66 mL, 1.66 mmol, 1.2 equiv.) was added dropwise and the mixture was stirred at 0°C during 60 min. THF was removed under vacuum and the crude was purified using flash chromatography (dichloromethane/MeOH, 95/5, v/v) to afford the product as a pale-yellow oil (322 mg, 60 % over 3 steps). *Rf* = 0.43 (dichloromethane/MeOH, 95/5, v/v). $^1H$ NMR (300 MHz, $CDCl_3$): δ (ppm) 10.02 (s, 1H), 7.30 - 7.21 (m, 2H), 7.14 - 6.91 (m, 4H), 3.62 (s, 2H), 2.89 (t, *J* = 5.8 Hz, 2H), 2.83 - 2.66 (m, 4H), 2.55 - 2.46 (m, 2H), 1.84 - 1.58 (m, 4H), 1.50 - 1.34 (m, 2H). $^{13}C$ NMR (75 MHz, $CDCl_3$): δ (ppm) 198.7, 156.94, 154.91, 135.71, 134.70, 134.25, 129.71, 128.62, 126.32, 126.31, 126.10, 125.58, 58.22, 56.16, 50.91, 37.26, 29.62, 29.00, 27.19, 26.97.

**AB-746** - **91**

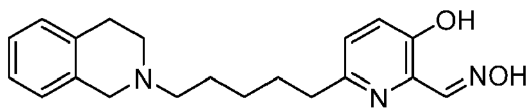

2.05 TFA

Aldehyde **110** (322 mg, 0.99 mmol, 1 equiv.) was dissolved in dry MeOH (10 mL) under argon. Hydroxylamine hydrochloride (75 mg, 1.08 mmol, 1.1 equiv.) and sodium acetate (110 mg, 1.35 mmol, 1.5 equiv.) were added and the mixture was stirred at 4h. Methanol was removed under vacuum and the crude oxime was purified using reverse phase chromatography (method D) to give the title compound as a yellow solid (227.1 mg, 64%). $^1H$ NMR (300 MHz, $CD_3OD$): δ (ppm) 8.37 (s, 1H), 7.84 (d, *J* = 8.8 Hz, 1H), 7.61 (d, *J* = 8.8 Hz, 1H), 7.33 - 7.02 (m, 4H), 5.60 (s, 3H), 4.54 (d, *J* = 15.1 Hz, 1H), 4.26 (d, *J* = 15.1 Hz, 1H), 3.73 (s, 1H), 3.45-3.15 (m, 4H), 3.12 - 2.81 (m, 3H), 2.00 - 1.60 (m, 4H), 1.56-1.38 (m, 2H). $^{13}C$ NMR (75 MHz, $CD_3OD$): δ (ppm) 162.74 (q, *J* = 34.9 Hz, $CF_3$), 154.41, 151.33, 143.78, 133.32, 132.48, 132.01, 129.70, 129.26, 128.76, 128.10, 127.72, 123.78, 119.90, 116.02, 112.14, 57.03, 53.99, 50.95, 49.00, 33.97, 30.05, 26.86, 26.23, 24.71. HPLC (method B): $t_R$ = 19.07 min (95%). MS (ESI+): *m/z* (%): 340 (100) $[M+H]^+$. HRMS (ESI+): *m/z* calcd for C20H26N3O2 340.2029; found: 340.2025.

**Methyl 3-(benzyloxy)-6-(5-((methylsulfonyl)oxy)pent-1-yn-1-yl)picolinate** - **57a**

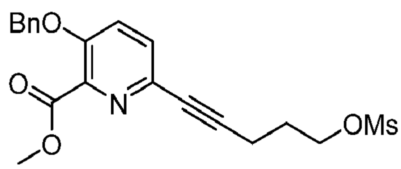

Methyl 3-(benzyloxy)-6-(4-((methylsulfonyl)oxy)pent-1-yn-1-yl)picolinate was synthetized following Procedure A using 14a and 57a was used without further purification in the next step (orangeous oil, yield: *quant.*). Caution: must be used within 12 h. $^1H$ NMR (300 MHz, $CDCl_3$) δ (ppm) 7.46 - 7.26 (m, 7H), 5.20 (s, 2H), 4.39 (t, *J* = 6.1 Hz, 2H), 3.96 (s, 3H), 3.04 (s, 3H), 2.58 (t, *J* = 6.9 Hz, 2H), 2.14 - 1.97 (m, 2H).

**Methyl 6-(5-(azepan-1-yl)pent-1-yn-1-yl)-3-(benzyloxy)picolinate - 121**

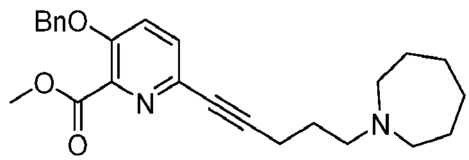

Mesylate **57a** (581 mg, 1.8 mmol, 1.03 equiv) was dissolved in dry MeCN (20 mL, 0.1 M) under Argon atmosphere and the resulting mixture was cooled down to 0°C. Azepane (200 μL, 1.74 mmol, 1.0 equiv.) followed by potassium carbonate (745 mg, 5.4 mmol, 1.5 equiv.) were added in this order. The resulting heterogenous mixture was reflux

overnight. Salt were removed by filtration and solvent was evaporated under reduced pressure. The crude product was purified by flash chromatography (dichloromethane/MeOH, 95/5, v/v) to afford the desired product as a brown oil (438 mg, 62%). *Rf* = 0.27 (dichloromethane/MeOH, 95/5, v/v). $^1$H NMR (300 MHz, $CDCl_3$): δ (ppm) 7.54 - 7.14 (m, 7H), 5.18 (s, 2H), 3.94 (s, 3H), 2.78 - 2.50 (m, 6H), 2.44 (t, *J* = 7.2 Hz, 2H), 1.85 - 1.68 (m, 2H), 1.59 (s, 8H). $^{13}$C NMR (75 MHz, $CDCl_3$): δ (ppm) 164.74, 152.68, 139.88, 135.45, 129.85, 128.56, 128.05, 126.78, 121.69, 90.34, 79.20, 70.65, 57.01, 55.33, 52.48, 28.07, 26.89, 26.40, 17.18.

**Methyl 6-(5-(azepan-1-yl)pentyl)-3-hydroxypicolinate - 122**

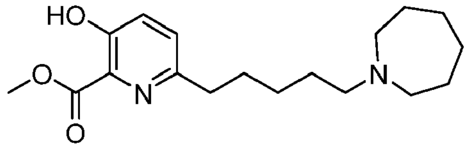

Compound **121** (369 mg, 0.91 mmol, 1 equiv.) was dissolved in MeOH (18 mL, 0.05 M) and the homogenous solution was degassed 20 min using argon. Pearlman's catalyst (147 mg, 20% w/w) was added. After 10 min of degassing using Argon, balloon of hydrogen gas was bubbled and the mixture was stirred under hydrogen atmosphere at rt until completion. The crude mixture was filtered over celite®, solvents were removed under vacuum to give **122** as an orangeous oil (291 mg, quant.). $^1$H NMR (300 MHz, $CDCl_3$): δ (ppm) 8.97 (br s, 1H), 7.25 (s, 2H), 4.01 (s, 3H), 2.83 - 2.69 (m, 2H), 2.69 - 2.53 (m, 4H), 2.54 - 2.39 (m, 2H), 1.75 - 1.44 (m, 12H), 1.40 - 1.26 (m, 2H). $^{13}$C NMR (75 MHz, $CDCl_3$): δ (ppm) 170.16, 157.18, 154.00, 129.18, 128.78, 126.63, 57.94, 55.25, 53.14, 37.56, 29.91, 27.15, 27.01, 26.72.

**6-(5-(azepan-1-yl)pentyl)-3-hydroxypicolinaldehyde - 123**

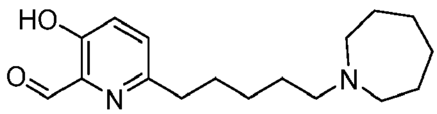

Unprotected 3-hydroxypyridine-2-methyl ester **122** (86 mg, 0.28 mmol, 1.0 equiv.) was dissolved in dry dichloromethane (10 mL, 0.2 M), then triethylamine (118 μL, 0.84, 3 equiv.) and TBSOTf (77 μL, 0.42 mmol, 1.5 equiv.) were added dropwise at 0 °C. The mixture was stirred at rt overnight. Resulting mixture was diluted in dichloromethane, washed with water, brine and dried under sodium sulfate. dichloromethane was removed under vacuum giving the crude silyl ether that was dissolved in dry dichloromethane (0.1 M) and cooled down to -78 °C. DIBAL-H (685 μL, 685 μmol, 2.5 equiv.) was added dropwise and the mixture was stirred at -78 °C during 15 min. Methanol was added and the crude mixture was heat up to rt. After dilution with dichloromethane, the organic layer was washed using aqueous NaOH (1 M), water and brine. After drying under sodium sulfate or magnesium sulfate, dichloromethane was removed under vacuum giving the crude silyl ether aldehyde moiety used without purification in the next step. Crude reduced silyl ether was dissolved in dry THF and cooled down to 0°C. TBAF (1.05- 1.1 equiv.) was added dropwise and the mixture was stirred at 0°C during 30 min. THF was removed under vacuum and the crude was purified using silica gel flash chromatography (dichloromethane/MeOH, 95/5 to 90/10, v/v) to give the title compound as a brown oil (56 mg, 68% over 3 step, with trace of TBAF, 1 aldehyde for 0.4 TBAF). *Rf* = 0.5 (dichloromethane/MeOH, 90/10, v/v). $^1$H NMR (300 MHz, $CDCl_3$) δ (ppm) 10.04 (s, 1H), 7.36 (d, *J* = 8.7 Hz, 1H), 7.32 (d, *J* = 8.5 Hz, 1H), 3.58 - 3.42 (br s, 2H), 3.06 - 2.83 (m, 4H), 2.69 (t, *J* = 7.6 Hz, 2H), 2.25 - 2 (m, 2H), 1.96 - 1.59 (m, 12H). $^{13}$C NMR (75 MHz, $CDCl_3$) δ (ppm) 198.68, 157.10, 154.29, 135.76, 130.01, 126.57, 57.48, 54.39, 36.64, 26.93, 26.30, 24.16, 23.84, 23.38. HRMS (ESI+): *m/z* calcd. for C17H27N2O2 291.2079; found 291.2073.

**AB-152** - **112 & 112a**

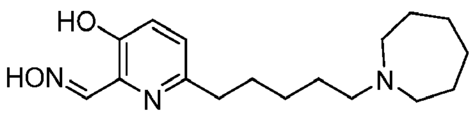

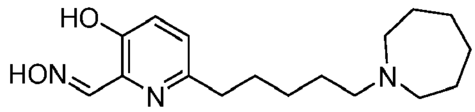

1.95 TFA

Aldehyde **123** (60 mg, 0.21 mmol, 1 equiv.) was dissolved in dry MeOH under argon. Hydroxylamine hydrochloride (15 mg, 1.1 equiv.) and sodium acetate (21 mg, 1.2 equiv) were added and the mixture was stirred at rt during 4 h. Methanol was removed under vacuum and the crude oxime was purified using normal phase purification (dichloromethane/MeOH, 90/10, v/v) to give **112** (38 mg, 60%) as a brown oil. *Rf* = 0.26 (dichloromethane/MeOH, 90/10, v/v). $^{1}$H NMR (300 MHz, $CD_3OD$): δ (ppm) 8.28 (s, 1H), 7.26 (d, *J* = 8.5 Hz, 1H), 7.14 (d, *J* = 8.5 Hz, 1H), 2.92 - 2.78 (m, 4H), 2.72 (s, 2H), 2.68 - 2.59 (m, 2H), 1.75 - 1.61 (m, 12H), 1.49 - 1.35 (m, 2H). $^{13}$C NMR (75 MHz, $CD_3OD$): δ (ppm) 154.36, 152.79, 136.40, 126.15, 125.33, 59.08, 56.37, 37.69, 30.98, 30.76, 27.98, 27.82, 26.97, 24.79. HPLC (method A): tR= 17.9 min (purity: 97%). HRMS (ESI+): *m/z* calcd. for C17H28N3O2 306.2177; found 306.2182.

Aldehyde **123** (295 mg, 1.017 mmol, 1.0 equiv.) was dissolved in dry MeOH under argon. Hydroxylamine hydrochloride (87 mg, 1.2 equiv.) and sodium acetate (120 mg, 1.4 equiv.) were added and the mixture was stirred at rt during 4 h. Methanol was removed under vacuum and the crude oxime was purified using reverse phase purification (method D) to give **112** as a brown oil (98 mg, 18%). $^{1}$H NMR (300 MHz, $CDCl_3$): δ (ppm) 11.67 (s, 2H), 8.35 (s, 1H), 7.16 (d, *J* = 8.5 Hz, 1H), 6.98 (d, *J* = 8.5 Hz, 1H), 3.58 - 3.42 (br s, 2H), 3.06 - 2.83 (m, 4H), 2.69 (t, *J* = 7.6 Hz, 2H), 2.25 - 2 (m, 2H), 1.96 - 1.59 (m, 12H). $^{13}$C NMR (75 MHz, $CDCl_3$): δ (ppm) 162.05 (q, J = 35 Hz), 153.18, 152.64, 152.52, 135.09, 124.67, 123.90, 57.48, 54.39, 36.64, 26.93, 26.30, 24.16, 23.84, 23.38. HPLC (method B): $t_R$ = 17.57 min (96.5%)

**Methyl 6-(4-(azocan-1-yl)but-1-yn-1-yl)-3-(benzyloxy)picolinate- 125**

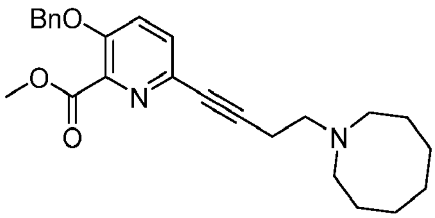

Mesylate **57a** (1.12 g, 2.90 mmol, 1.2 equiv.) was dissolved in dry MeCN (30 mL, 0.1 M) under Argon atmosphere and the resulting mixture was cooled down to 0°C. Azocane (315 μL, 2.40 mmol, 1.0 equiv.) followed by potassium carbonate (666 mg, 4.80 mmol, 2.0 equiv.) were added in this order. The resulting heterogenous mixture was reflux overnight. Salt were removed by filtration and solvent was evaporated under reduced pressure. The crude product was purified by flash chromatography (dichloromethane/MeOH, 100/0 to 95/5, v/v) to give the title compound as a brown oil (336 mg, 29 %, with trace of $PPh_3$). *Rf* = 0.31 (dichloromethane/MeOH, 96/4, v/v). TLC-MS (ESI+): *m/z* = 407 [M+H]+, 429 [M+Na]+. $^{1}$H NMR (300 MHz, $CDCl_3$): δ (ppm) 7.50 - 7.12 (m, 8H), 5.07 (s, 2H), 3.83 (s, 3H), 2.69 (t, *J* = 7.4 Hz, 2H), 2.50 (br s, 3H), 2.44 (t, *J* = 7.4 Hz, 2H), 1.49 (t, *J* = 8.7 Hz, 11H). $^{13}$C NMR (75 MHz, $CDCl_3$): δ (ppm) 164.78 (q, J = 36 Hz), 152.74, 139.92, 135.47, 135.42, 133.05, 132.00, 131.90, 131.87, 131.67, 129.81, 128.59, 128.50, 128.34, 128.08, 126.81, 121.72, 89.24, 79.69, 70.70, 57.12, 53.39, 52.50, 27.85, 27.25, 26.09, 18.59.

**Methyl 6-(4-(azocan-1-yl)butyl)-3-hydroxypicolinate -126**

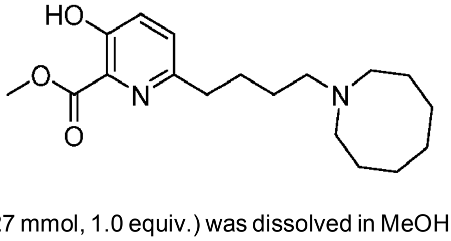

Compound **125** (336 mg, 0.827 mmol, 1.0 equiv.) was dissolved in MeOH (83 mL, 0.01 M) and the homogenous solution was degassed 20 min using argon. Pearlman's catalyst (150 mg, 40% w/w) was added. After 10 min of degassing

using Argon, balloon of hydrogen gas was bubbled and the mixture was stirred under hydrogen atmosphere at rt overnight. The crude mixture was filtered over celite®, solvents were removed under vacuum. The crude was purified by flash chromatography on silica gel (dichloromethane/MeOH, 90/10, v/v) to afford the title product (39 mg, 15%). *Rf* = 0.50 (dichloromethane/MeOH, 90/10, v/v). TLC-MS (ESI+): *m/z* = 321 [M+H]+, 344 [M+Na]+. $^1$H NMR (300 MHz, $CDCl_3$): δ (ppm) 10.56 (s, 1H), 7.32 (s, 2H), 4.04 (s, 3H), 3.22 (br s, 4H), 3.06 - 2.90 (m, 2H), 2.90 - 2.71 (m, 2H), 2.06 - 1.86 (m, 6H), 1.86 - 1.71 (m, 3H), 1.67 (br s, 5H). $^{13}$C NMR (75 MHz, $CDCl_3$): δ (ppm) 170.46, 157.86, 153.10, 129.88, 129.35, 127.45, 55.63, 53.63, 50.80, 37.00, 27.50, 26.79, 24.87, 24.30, 22.85.

**AB-643 -127**

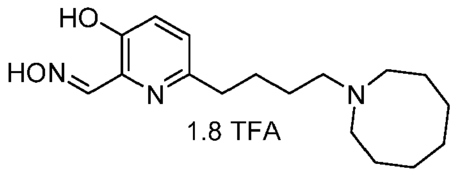

1.8 TFA

Compound **126** (39 mg, 0.122 mmol, 1.0 equiv.) was dissolved in dry dichloromethane (10 mL, 0.1 M), then triethylamine (52 μL, 0.366 mmol, 3 equiv.) and TBSOTf (43 μL, 0.188 mmol, 3 equiv.) were added dropwise at 0°C. The mixture was stirred at rt overnight. Resulting mixture was diluted in dichloromethane, washed with water, brine and dried under sodium sulfate. Dichloromethane was removed under vacuum giving the crude silyl ether that was dissolved in dry dichloromethane (10 mL, 0.1 M) and cooled down to -78°C. DIBAL-H (305 μL, 0.305 mmol, 2.5 equiv.) was added dropwise and the mixture was stirred at -78°C during 15 min. Methanol (305 μL) was added and the crude mixture was heat up to rt. After dilution with dichloromethane, the organic layer was washed using aqueous NaOH (1 M), water and brine. After drying under sodium sulfate, dichloromethane was removed under vacuum giving the crude silyl ether aldehyde moiety used without purification in the next step. Crude reduced silyl ether was dissolved in dry THF (20 mL, 0.05 M) and cooled down to 0°C. TBAF (150 μL, 0.150 mmol, 1.2 equiv.) was added dropwise and the mixture was stirred at 0°C during 30 min. THF was removed under vacuum and the crude was purified using silica gel flash chromatography (dichloromethane/MeOH, 100/0 to 90/10, v/v) to afford the aldehyde as a yellow oil (16.2 mg, 46 % over 3 steps - trace of TBAF). TLS-MS (ESI+) : *m/z* = 291 $[M+H]^+$, 313 $[M+Na]^+$; *Rf* = 0.18 (dichloromethane/MeOH, 90/10, v/v).

The crude aldehyde was dissolved in MeOH (5 mL, 0.02 M) under argon. Hydroxylamine hydrochloride (10 mg, 0.140 mmol, 2.5 equiv.) and sodium acetate (14 mg, 0.168 mmol, 3 equiv.) were added and the mixture was stirred at rt overnight. Methanol was removed under vacuum and the crude oxime was purified using reverse phase chromatography (method D) to give the title compound as brown oil (4.98 mg, 8% over 4 steps). $^1$H NMR (300 MHz, $CD_3OD$): δ (ppm) 8.34 (s, 1H), 7.46 (d, *J* = 8.6 Hz, 1H), 7.32 (d, *J* = 8.6 Hz, 1H), 3.55 - 3.40 (m, 2H), 3.27 - 3.09 (m, 4H), 2.95 - 2.80 (m, 2H), 2.10 - 1.93 (m, 2H), 1.90-1.70 (m, 10H), 1.70-1.50 (br s, 3H). $^{13}$C NMR (75 MHz, $CD_3OD$): δ (ppm) 152.75, 151.32, 148.49, 134.32, 126.67, 124.77, 55.89, 51.34, 34.45, 26.35, 25.16, 24.03, 23.53, 22.38. HRMS (ESI+): *m/z* calcd. for C17H28N3O2 306.2182; found 306.2184. HPLC (method A): $t_R$ = 17.90 min (purity = 95.1%).

**Methyl 6-(5-(azocan-1-yl)pent-1-yn-1-yl)-3-(benzyloxy)picolinate** - **128**

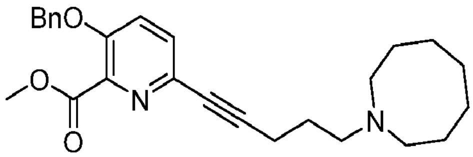

Tosylate **59a** (1.02 g, 2.13 mmol, 1.0 equiv) was dissolved in dry MeCN (0.1 M) under Argon atmosphere and the resulting mixture was cooled down to 0 °C. Azocane (305 μL, 2.35 mmol, 1.1 equiv.) followed by potassium carbonate (590 mg, 4.26 mmol, 2 equiv.) were added in this order. The resulting heterogenous mixture was reflux overnight. Salt were removed by filtration and solvent was evaporated under reduced pressure. The crude product was purified using Silica gel flash chromatography (dichloromethane/MeOH, 95/5 to 90/10, v/v) to give the title compound as a brown oil (780 mg, 85 %). *Rf* = 0.43 (dichloromethane/MeOH, 90/10, v/v). $^1$H NMR (300 MHz, $CDCl_3$): δ (ppm) 7.54 - 7.23 (m, 7H), 5.19 (s, 2H), 3.95 (s, 3H), 2.65 (t, *J* = 6.6 Hz, 5H), 2.50 (t, *J* = 7.2 Hz, 2H), 1.93 -1.73 (m, 2H), 1.61 (s, 9H). $^{13}$C

NMR (75 MHz, $CDCl_3$): δ (ppm) 164.66, 152.58, 139.77, 135.33, 135.19, 129.83, 128.43, 127.93, 126.68, 121.62, 89.86, 79.30, 70.52, 57.30, 53.41, 52.35, 26.89, 26.68, 26.25, 25.93, 16.96.

**Methyl 6-(5-(azocan-1-yl)pentyl)-3-hydroxypicolinate -129**

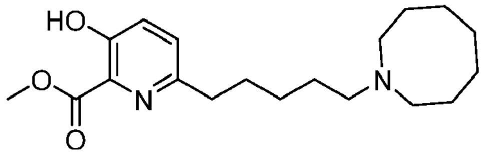

Compound **128** (780 mg, 1.86 mmol, 1 equiv.) was dissolved in MeOH (186 mL, 0.01 M) and the homogenous solution was degassed 20 min using argon. Pearlman's catalyst (150 mg, 20% w/w) was added. After 10 min of degassing using Argon, balloon of hydrogen gas was bubbled and the mixture was stirred under hydrogen atmosphere at rt overnight. The crude mixture was filtered over celite®, solvents were removed under vacuum. The crude was purified by flash chromatography on silica gel (dichloromethane/MeOH, 90/10, v/v) to afford the title product (621 mg, 100%). *Rf* = 0.52 (dichloromethane/MeOH, 90/10, v/v). TLC-MS (ESI+) *m/z* = 335 $[M+H]^+$, 357 $[M+Na]^+$. $^1H$ NMR (300 MHz, $CDCl_3$): δ (ppm) 7.11 (d, *J* = 8.7 Hz, 1H), 7.07 (d, *J* = 8.4 Hz, 1H), 3.80 (s, 3H), 3.17 (br s, 2H), 3.03 (br s, 3H), 2.77 (br s, 2H), 2.56 (t, *J* = 7.6 Hz, 2H), 1.72 (br s, 6H), 1.48 (d, *J* = 19.1 Hz, 7H), 1.18 (br s, 3H). $^{13}C$ NMR (75 MHz, $CDCl_3$): δ (ppm) 169.79, 156.91, 153.18, 129.17, 128.59, 126.53, 55.58, 52.91, 50.55, 36.82, 29.06, 26.13, 25.85, 24.24, 24.03, 22.39.

**AB-436** - **114**

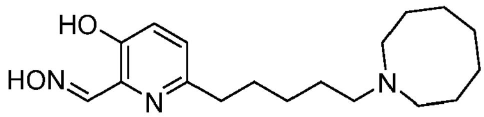

1.81 TFA

Compound **129** (621, 1.86 mmol, 1 equiv.) was dissolved in dry dichloromethane (20 mL, 0.1 M), then triethylamine (783μL, 5.58, 3 equiv.) and TBSOTf (510 μL, 2.79 mmol, 1.5 equiv.) were added dropwise at 0°C. The mixture was stirred at rt until overnight. Resulting mixture was diluted in dichloromethane, washed with water, brine and dried under sodium sulfate. dichloromethane was removed under vacuum giving the crude silyl ether that was dissolved in dry dichloromethane (20 mL, 0.1 M) and cooled down to - 78°C. DIBAL-H (3.29 mL, 3.29 mmol, 2.5 equiv.) was added dropwise and the mixture was stirred at -78°C during 15 min. Methanol was added and the crude mixture was heat up to rt. After dilution with dichloromethane, the organic layer was washed using aqueous NaOH (1 M), water and brine. After drying under sodium sulfate or magnesium sulfate, dichloromethane was removed under vacuum giving the crude silyl ether aldehyde moiety used without purification in the next step. Crude reduced silyl ether was dissolved in dry THF (40 mL) and cooled down to 0 °C. TBAF (1.9 mL, 1.9 mmol, 1.02 equiv.) was added dropwise and the mixture was stirred at 0 °C during 30 min. THF was removed under vacuum and the crude was purified through a silica gel plug (dichloromethane/MeOH, 95/5, v/v) to give the crude deprotected aldehyde (105,4 mg, 0.347 mmol, 1 equiv.) that was dissolved in dry MeOH under argon. Hydroxylamine hydrochloride (30 mg, 0.417 mmol, 1.1 equiv.) and sodium acetate (36 mg,0.417 mmol, 1.2 equiv.) were added and the mixture was stirred overnight at rt. Methanol was removed under vacuum and the crude oxime was purified using reverse phase chromatography to afford the title compound as light orange oil (5.42 mg, 0.9 % over 4 steps). $^1H$ NMR (300 MHz, $CD_3OD$): δ (ppm) 7.46 (d, *J* = 8.7 Hz, 1H), 7.40 (d, *J* = 8.7 Hz, 1H), 3.54 - 3.37 (m, 2H), 3.28 - 3.05 (m, 3H), 2.86 - 2.71 (m, 2H), 2.10 - 1.90 (m, 2H), 1.90 - 1.54 (m, 13H), 1.51 - 1.36 (m, 2H). $^{13}C$ NMR (75 MHz, $CD_3OD$): δ (ppm) 170.40 (q, 38 Hz), 158.07, 154.94, 130.81, 130.03, 128.50, 57.61, 53.36, 52.78, 37.20, 30.48, 27.07, 26.56, 25.47, 25.29, 23.83, 9.18. HRMS (ESI+): *m/z* calcd. for C18H30N3O2 320.2338; found 320.2332. HPLC (method B): $t_R$ = 18.58 min (purity = 95.2%).

**N-(tert-Butoxycarbonyl)-N-(4-piperidyl)amine** - **132**

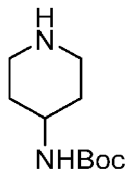

1-benzylpiperidin-4-amine (4 mL, 19.05 mmol, 1.0 equiv.) and DIPEA (8.3 mL, 45.82 mmol, 2.4 equiv.) were dissolved in dry THF (40 mL) at 0°C and $Boc_2O$ (4.97 g, 22.86 mmol, 1.2 equiv.) in dry THF (23 mL) was added dropwise. The mixture was stirred overnight at rt. After concentration until dryness, a crude white powder was obtained and used without purification (5.33 g, 97 %). The product obtained 5.33 g, 18.40 mmol, 1 equiv.) was dissolved in degassed methanol (100 mL). Pd/C (533 mg, 10% w/w) was poured into this solution. After 10 min of degassing using argon, balloon of hydrogen gas was bubbled and the mixture was stirred under hydrogen atmosphere (1 atm) at rt overnight. The crude mixture was filtered over celite®, solvents were removed under vacuum to give the title compound as a white solid (3.68 g, 97 % over 1 steps). Mp = 164 °C (dec). MS (ESI+): *m/z* = 201 $[M+H]^+$, 223 $[M+Na]^+$. $^1H$ NMR (300 MHz, $CDCl_3$): δ (ppm) 4.88 (br s, 1H), 3.36 (br s, 1H), 2.94 (dt, *J* = 12.4, 3.0 Hz, 2H), 2.53 (td, *J* = 12.4, 2.3 Hz, 2H), 1.80 (d, *J* = 10.1 Hz, 2H), 1.63 - 1.44 (m, 1H), 1.28 (d, *J* = 30.7 Hz, 9H), 1.16 (ddd, *J* = 15.4, 11.7, 4.0 Hz, 2H). $^{13}C$ NMR (75 MHz, $CDCl_3$): δ (ppm) 155.00, 78.72, 47.99, 45.25, 33.75, 28.22.

**Methyl 3-(benzyloxy)-6-(4-((methylsulfonyl)oxy)but-1-yn-1-yl)picolinate** - **57b**

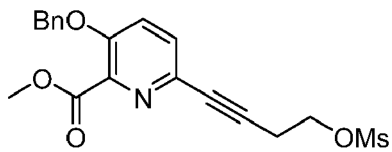

Methyl 3-(benzyloxy)-6-(5-((methylsulfonyl)oxy)but-1-yn-1-yl)picolinate was synthetized folowing **Procedure A** using **14b** and **57b** was used without purification in the next step (orangeous oil, yield: *quant.).* Caution: must be used within 6 h. *Rf* = 0.5 (dichloromethane/MeOH, 1/1, v/v). $^1H$ NMR (300 MHz, $CDCl_3$) δ (ppm) 7.51 - 7.27 (m, 7H), 5.21 (s, 2H), 3.96 (s, 3H), 3.82 (q, *J* = 6.3 Hz, 2H), 2.69 (t, *J* = 6.3 Hz, 2H), 1.56 (s, 3H).

**Methyl 3-(benzyloxy)-6-(4-(4-((*tert*-butoxycarbonyl)amino)piperidin-1-yl)but-1-yn-1-yl)picolinate - 133**

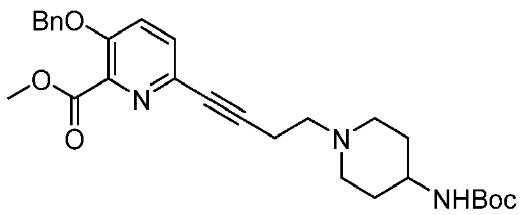

Mesylate **57b** (973 mg, 2.5 mmol, 1.0 equiv.) was dissolved in dry MeCN (30 mL, 0.08 M) under argon atmosphere and the resulting mixture was cooled down to 0 °C. Compound **132** (500 mg, 2.5 mmol, 1.0 equiv.) followed by potassium carbonate (700 g, 5 mmol, 2.0 equiv.) were added in this order. The resulting heterogenous mixture was reflux overnight. Salt were removed by filtration and solvent was evaporated under reduced pressure. The crude product was purified by flash chromatography (dichloromethane/MeOH, 94/6 to 90/10, v/v) to give the title compound **136** as an orange oil (389 g, 32 %). *Rf* = 0.25 (dichloromethane/MeOH, 95/5, v/v). MS (ESI+): *m/z* = 494 $[M+H]^+$. $^1H$ NMR (300 MHz, $CDCl_3$): δ (ppm) 7.43 - 7.04 (m, 7H), 5.10 (s, 2H), 4.56 (br s, 1H), 3.85 (s, 3H), 3.45 - 3.26 (m, 1H), 2.76 (d, *J* = 11.8 Hz, 2H), 2.63 - 2.52 (m, 2H), 2.52 - 2.43 (m, 2H), 2.06 (t, *J* = 10.5 Hz, 2H), 1.83 (d, *J* = 10.4 Hz, 2H), 1.35 (s, 10H). $^{13}C$ NMR (75 MHz, $CDCl_3$): δ (ppm) 172.81, 164.68, 155.13, 152.88, 139.81, 135.38, 135.06, 132.00, 131.93, 131.90, 131.87, 129.91, 128.62, 128.52, 128.36, 128.12, 126.80, 121.71, 88.28, 79.89, 79.06, 70.65, 56.59, 52.58, 51.94, 32.29, 28.32, 17.61.

**Methyl 6-(4-(4-((tert-butoxycarbonyl)amino)piperidin-1-yl)butyl)-3-hydroxypicolinate - 134**

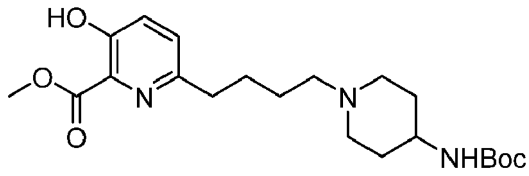

Compound **133** (388 mg, 0.788 mmol, 1.0 equiv.) was ether was dissolved in MeOH or EtOAc (0.05 M) and the homogenous solution was degassed 20 min using argon. Pearlman's catalyst (77 mg, 20% w/w) was added. After 10 min of degassing using Argon, balloon of hydrogen gas was bubbled and the mixture was stirred under hydrogen atmosphere at rt until completion (4 days). The crude mixture was filtered over celite®, solvents were removed under vacuum to give the title compound as a brown solid (321 mg, quant.). MS (ESI+): *m*/*z* = 409 $[M+H]^+$. $^1H$ NMR (300 MHz, $CDCl_3$): δ (ppm) 7.22 (s, 2H), 4.86 (br s, 1H), 3.94 (s, 3H), 3.41 (br s, 1H), 2.88 (d, *J* = 10.7 Hz, 2H), 2.71 (t, *J* = 7.4 Hz, 2H), 2.49 - 2.30 (m, 2H), 2.12 (t, *J* = 8.7 Hz, 2H), 1.88 (d, *J* = 13.8 Hz, 3H), 1.73 - 1.42 (m, 6H), 1.34 (s, 10H). $^{13}C$ NMR (75 MHz, $CDCl_3$): δ (ppm) 169.92, 157.05, 155.21, 153.45, 132.00, 131.95, 131.87, 129.14, 128.66, 128.55, 128.39, 126.62, 79.08, 57.91, 53.02, 52.15, 37.06, 31.58, 28.32, 27.67, 25.85.

**Tert-butyl (1-(4-(6-formyl-5-hydroxypyridin-2-yl)butyl)piperidin-4-yl)carbamate-135**

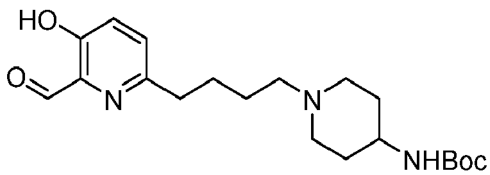

Compound **134** (321 mg, 0.788 mmol, 1 equiv.) in dry dichloromethane (10, mL, 0.08 M), then triethylamine (280 μL, 1.97 mmol, 2.5 equiv.) and TBSOTf (220 μL, 0.946 mmol, 1.2 equiv.) were added dropwise at 0 °C. The mixture was stirred at rt overnight. Resulting mixture was diluted in dichloromethane, washed with water, brine and dried under sodium sulfate. Dichloromethane was removed under vacuum giving the crude silyl ether that was dissolved in dry dichloromethane (10 mL, 0.08 M) and cooled down to - 78 °C. DIBAL-H (2.36 mL, 2.36 mmol, 3.0 equiv.) was added dropwise and the mixture was stirred at -78 °C during 15 min. Methanol was added and the crude mixture was heat up to rt. After dilution with dichloromethane, the organic layer was washed using aqueous NaOH (1 M), water and brine. After drying under sodium sulfate or magnesium sulfate, dichloromethane was removed under vacuum giving the crude silyl ether aldehyde moiety used without purification in the next step. Crude reduced silyl ether was dissolved in dry THF 20 mL and cooled down to 0 °C. TBAF (790 μL, 0.790 mmol, 1 equiv.) was added dropwise and the mixture was stirred at 0°C during 30 min. THF was removed under vacuum and the crude was purified using silica gel flash chromatography (dichloromethane/MeOH,90/10, v/v) to give the title compound as a yellow oil (168 mg, 57% over 3 steps). TLC-MS (ESI+): *m*/*z* = 378 $[M+H]^+$; *Rf* = 0.33 (dichloromethane/MeOH, 95/5, v/v). $^1H$ NMR (300 MHz, $CDCl_3$): δ (ppm) 9.95 (s, 1H), 7.24 (d, *J* = 8.7 Hz, 1H), 7.20 (d, *J* = 8.6 Hz, 1H), 4.42 (s, 1H), 3.39 (s, 1H), 2.87 - 2.66 (m, 4H), 2.40 - 2.23 (m, 2H), 2.00 (t, *J* = 10.8 Hz, 2H), 1.86 (d, *J* = 11.6 Hz, 2H), 1.77 - 1.58 (m, 2H), 1.52 (dt, *J* = 23.2, 13.0 Hz, 4H), 1.37 (s, 9H). $^{13}C$ NMR (75 MHz, $CDCl_3$): δ (ppm) 198.83, 157.08, 155.28, 154.75, 135.76, 129.86, 126.49, 77.16, 58.44, 52.55, 37.19, 32.48, 28.49, 27.69, 26.65.

**AB-740 - 115**

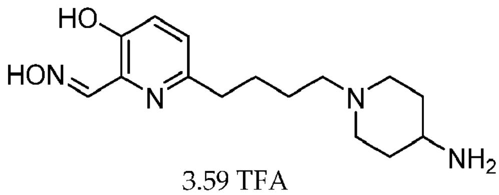

3.59 TFA

Aldehyde **135** (138 mg, 0.445 mmol, 1 equiv.) was dissolved in dry MeOH (20 mL, 0.02 M) under argon. Hydroxylamine hydrochloride (47 mg, 1.5 equiv.) was added and the mixture was stirred at rt 10 min. Then TFA (5 mL) was added at 0 °C and the mixture was stirred during 10 min. Volatiles were removed under vacuum and the crude oxime was purified using reverse phase chromatography to give the title compound as golden white off solid (177 mg, 57%). MS (ESI+): *m*/*z*: = 293 (100) $[M+H]^+$, 315 (15) $[M+Na]^+$. $^1H$ NMR (300 MHz, $CD_3OD$): δ (ppm) 8.40 (s, 1H), 7.73 (d, *J* = 8.7 Hz, 1H), 7.53 (d, *J* = 8.7 Hz, 1H), 5.19 (s, 11H), 3.68 (d, *J* = 9.9 Hz, 2H), 3.48 (s, 1H), 3.17 (s, 4H), 2.96 (d, *J* = 6.6 Hz, 2H), 2.29 (d, *J* = 12.7 Hz, 2H), 2.02 (dd, *J* = 12.9, 9.8 Hz, 2H), 1.82 (t, *J* = 11.6 Hz, 4H). $^{19}F$ NMR (282 MHz, $CD_3OD$): δ (ppm) -76.84. $^{13}C$ NMR (75 MHz, $CD_3OD$): δ (ppm) 162.95 (q, *J* = 35.0 Hz), 154.52, 151.06, 145.03, 134.10, 131.60, 127.45, 123.79, 119.92, 116.05, 112.17, 57.51, 51.78, 49.00, 46.75, 34.02, 28.46, 27.51, 24.43. HRMS (ESI+): *m*/*z* calcd. for C15H25N4O2 293.1978; found 293.1974. HPLC (method B): $t_R$ = 9.70 min (purity = 95.35%).

**Methyl 3-(benzyloxy)-6-(5-(4-((tert-butoxycarbonyl)amino)piperidin-1-yl)pent-1-yn-1-yl)picolinate** - **136**

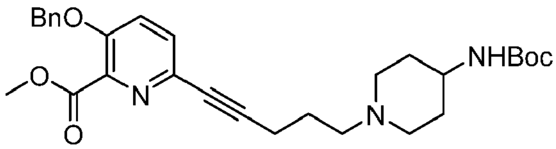

Tosylate **59a** (2.0 g, 4.2 mmol, 1.0 equiv) was dissolved in dry MeCN (42 mL, 0.1 M) under Argon atmosphere and the resulting mixture was cooled down to 0 °C. Compound **132** (840 mg, 4.32 mmol, 1.0 equiv.) followed by potassium carbonate (1.7 g, 4.2 mmol, 3.0 equiv.) were added in this order. The resulting heterogenous mixture was reflux overnight. Salt were removed by filtration and solvent was evaporated under reduced pressure. The crude product was purified using silica gel flash chromatography (dichloromethane/MeOH, 94/6 to 90/10, v/v) to give the title compound **136** as a yellow oil (2.1 g, 98 %). $^1H$ NMR (300 MHz, $CDCl_3$): δ (ppm) 7.40 -7.15 (m, 7H), 5.11 (s, 2H), 4.42 (s, 1H), 3.87 (s, 3H), 3.37 (s, 1H), 2.76 (d, *J* = 11.5 Hz, 2H), 2.36 (dd, *J* = 13.4, 6.6 Hz, 4H), 2.00 (t, *J* = 11.0 Hz, 2H), 1.84 (d, *J* = 11.1 Hz, 2H), 1.75 - 1.57 (m, 2H), 1.36 (s, 12H). $^{13}C$ NMR (75 MHz, $CDCl_3$): δ (ppm) 164.85, 155.23, 152.90, 135.56, 135.48, 129.97, 128.74, 128.24, 126.93, 121.83, 89.94, 79.55, 77.16, 70.85, 57.44, 52.67, 52.40, 32.51, 28.44, 25.83, 17.41. TLS-MS: *Rf* = 0.26 (dichloromethane/MeOH, 94/6, v/v) *m*/*z* = 508 [M+H]+.

**Methyl 6-(5-(4-((tert-butoxycarbonyl)amino)piperidin-1-yl)pentyl)-3-hydroxypicolinate** - **137**

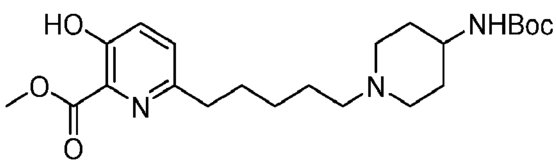

Compound **136** (2.1 g, 4.15 mmol, 1.0 equiv.) was dissolved in MeOH (400 mL, 0.01 M) and the homogenous solution was degassed 20 min using argon. Pearlman's catalyst (350 mg, 20%w/w) was added. After 10 min of degassing using Argon, balloon of hydrogen gas was bubbled and the mixture was stirred under hydrogen atmosphere at rt overnight. The crude mixture was filtered over celite®, solvents were removed under vacuum to give compound **137** as an orangeous oil (1.75 g, quant.). $^1H$ NMR (300 MHz, $CDCl_3$): δ (ppm) 10.59 (s, 1H), 7.28 (s, 2H), 4.51 (s, 1H), 4.04 (s, 3H), 3.53 (s, 1H), 3.08 (s, 2H), 2.89 - 2.64 (m, 2H), 2.53 (s, 2H), 2.33 (d, *J* = 8.7 Hz, 2H), 2.01 (d, *J* = 8.3 Hz, 3H), 1.88 - 1.55 (m, 6H), 1.53 - 1.17 (m, 13H). $^{13}C$ NMR (75 MHz, $CDCl_3$): δ (ppm) 170.10, 157.18, 155.21, 153.70, 129.17, 128.80, 126.66, 125.88, 79.46, 57.95, 53.13, 52.08, 46.95, 37.38, 31.15, 29.68, 28.38, 26.91, 25.68.

**Tert-butyl (1-(5-(6-formyl-5-hydroxypyridin-2-yl)pentyl)piperidin-4-yl)carbamate - 138**

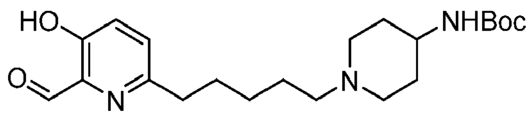

Compound **137** (1.75 g, 4.15 mmol, 1.0 equiv.) was dissolved in dry dichloromethane (40 mL, 0.1 M), then triethylamine (900 μL, 6.22 mmol, 1.5 equiv.) and TBSOTf (1.10 mL, 4.98 mmol, 1.2 equiv.) were added dropwise at 0°C. The mixture was stirred at rt during 3h. Resulting mixture was diluted in dichloromethane, washed with water, brine and dried over sodium sulfate. Dichloromethane was removed under vacuum giving the crude silyl ether that was dissolved in dry dichloromethane (40 mL, 0.1 M) and cooled down to - 78°C. DIBAL-H (8.2 mL, 8.2 mmol, 2.0 equiv.) was added dropwise and the mixture was stirred at -78°C during 15 min. Methanol (8.2 mL) was added and the crude mixture was heat up to rt. After dilution with dichloromethane, the organic layer was washed using aqueous NaOH (1M), water and brine. After dring under sodium sulfate or magnesium sulfate, dichloromethane was removed under vacuum giving the crude silyl ether aldehyde moiety used without purification in the next step. Crude reduced silyl ether was dissolved in dry THF (100 mL) and cooled down to 0°C. TBAF (4.2 mL, 4.2 mmol, 1.01 equiv.) was added dropwise and the mixture was stirred at 0°C during 30 min. THF was removed under vacuum and the crude was purified using silica gel flash chromatography (dichloromethane/MeOH, 100/0 to 90/10, v/v) to give the title product as a white off foam (316 mg, 30% over 3 steps). *Rf* = 0.5 (dichloromethane/MeOH, 90/10). MS (ESI+): *m/z* (%) = 392 (100) $[M+H]^+$. $^1H$ NMR (300 MHz, $CDCl_3$): δ (ppm) 10.46 (br s, *J* = 67.7 Hz, 1H), 10.05 (s, 1H), 7.32 (s, 2H), 4.63 (br s, 1H), 3.57 (br s, 1H), 3.10 (br s, 2H), 2.81 (t, *J* = 7.7 Hz, 2H), 2.57 (br s, 2H), 2.35 (br s, 2H), 2.16 - 1.92 (m, 2H), 1.75 (dt, *J* = 25.1, 12.6 Hz, 5H), 1.60 - 1.05 (m, 11H). $^{13}C$ NMR (75 MHz, $CDCl_3$): δ (ppm) 198.85, 157.10, 155.30, 154.72, 135.82, 129.87, 126.50, 79.64, 58.12, 52.26, 47.11, 37.15, 31.31, 29.79, 29.40, 28.49, 26.98, 25.82.

**AB-737** - **116**

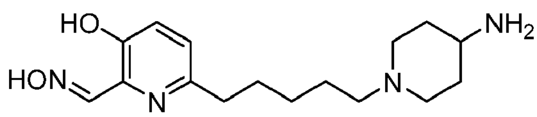

2.82 TFA

Same procedure as for **AB-740.** Yellow solid (298 mg, 65%). $^1H$ NMR (300 MHz, $CD_3OD$): δ (ppm) 8.42 (s, 1H), 7.84 (d, *J* = 8.8 Hz, 1H), 7.61 (d, *J* = 8.8 Hz, 1H), 5.48 (br s, 7H), 3.70 (d, *J* = 10.8 Hz, 2H), 3.49 (s, 1H), 3.12 (s, 4H), 3.01 - 2.82 (m, 2H), 2.31 (d, *J* = 12.9 Hz, 2H), 2.16 - 1.90 (m, 2H), 1.77 (t, *J* = 10.8 Hz, 4H), 1.59 - 1.29 (m, 2H). $^{19}F$ NMR (282 MHz, $CD_3OD$): δ (ppm) -76.66. $^{13}C$ NMR (75 MHz, $CD_3OD$): δ (ppm) 162.87 (q, *J* = 35.0 Hz), 154.52, 151.17, 143.34, 133.18, 132.92, 127.85, 123.76, 119.88, 116.01, 112.13, 57.73, 51.74, 46.81, 33.75, 29.96, 28.48, 26.79, 24.60. HRMS (ESI+): *m/z* calcd. for C16H27N4O2 307.2134; found 307.2133. HPLC (method B): $t_R$ = 14.51 min (97.37%).

**Methyl 3-(benzyloxy)-6-(4-(3-((tert-butoxycarbonyl)amino)piperidin-1-yl)but-1-yn-1-yl)picolinate - 140**

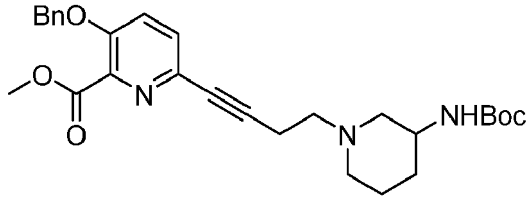

Same procedure as **133,** using commercially available 3-(Boc-amino)piperidine and methyl 3-(benzyloxy)-6-(4-((methylsulfonyl)oxy)but-1-yn-1-yl)picolinate as starting material. Yellow oil (461 mg, 38 %). *Rf* = 0.25 (dichloromethane/MeOH, 95/5). MS (ESI+): *m/z* = 494 $[M+H]^+$. $^1H$ NMR (300 MHz, $CDCl_3$): δ (ppm) 7.47 - 7.05 (m, 7H), 5.10 (s, 2H), 4.55 (s, 1H), 3.85 (s, 3H), 3.36 (s, 1H), 2.76 (d, *J* = 11.8 Hz, 2H), 2.52 (qd, *J* = 6.6, 3.7 Hz, 4H), 2.06 (t, *J* = 10.5 Hz, 2H), 1.93 - 1.68 (m, 3H), 1.35 (s, 13H). $^{13}C$ NMR (75 MHz, $CDCl_3$): δ (ppm) 172.81, 164.68, 155.13, 152.88, 139.81, 135.38, 135.06, 132.00, 131.93, 131.90, 131.87, 129.91, 129.04, 128.62, 128.52, 128.36, 128.12, 126.80, 121.71, 88.28, 79.89, 79.06, 70.65, 56.59, 52.58, 51.94, 47.51, 32.29, 28.32, 17.61.

**Methyl 6-(4-(3-((tert-butoxycarbonyl)amino)piperidin-1-yl)butyl)-3-hydroxypicolinate - 141**

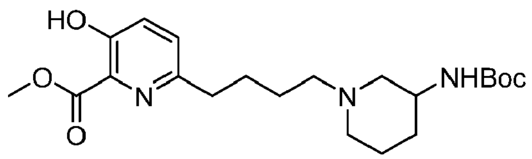

Same procedure as for compound **137,** using compound **140** as starting material. Light orange oil (374 mg, 98%). $^1H$ NMR (300 MHz, $CDCl_3$): δ (ppm) 10.51 (br s, 1H), 7.23 (s, 2H), 5.05 (br s, 1H), 3.97 (s, 3H), 3.68 (br s, 1H), 2.86 - 2.66 (m, 2H), 2.55 - 2.07 (m, 5H), 1.62 (dd, *J* = 15.7, 7.9 Hz, 3H), 1.56 - 1.41 (m, 4H), 1.36 (s, 10H). $^{13}C$ NMR (75 MHz, $CDCl_3$): δ (ppm) 170.16, 157.24, 155.22, 153.86, 129.21, 128.80, 126.72, 79.05, 58.54, 58.35, 53.75, 53.19, 46.22, 37.40, 29.68, 28.47, 27.87, 26.19, 22.12. MS (ESI+): *m/z* = 408 $[M+H]^+$.

**Tert-butyl (1-(4-(6-formyl-5-hydroxypyridin-2-yl)butyl)piperidin-3-yl)carbamate-142**

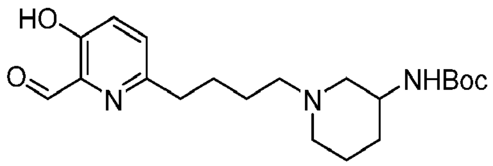

Same procedure as for **138,** using **141** as starting material. Yellow oil (189 mg, 55% over 3 steps). *Rf* = 0.5 (dichloromethane/MeOH, 90/10). MS (ESI+): m/z = 378 $[M+H]^+$. $^1H$ NMR (300 MHz, $CDCl_3$): δ (ppm) 9.96 (s, 1H), 7.24 (s, 2H), 4.98 (s, 1H), 3.66 (s, 1H), 3.46 - 3.16 (m, 1H), 2.81 - 2.60 (m, 2H), 2.44 (s, 1H), 2.39 - 2.16 (m, 4H), 1.63 (dt, *J* = 15.6, 8.0 Hz, 4H), 1.53 - 1.41 (m, 4H), 1.36 (s, 10H). $^{13}C$ NMR (75 MHz, $CDCl_3$): δ (ppm) 198.64, 156.98, 155.16, 154.75, 135.70, 129.74, 126.41, 78.94, 58.98, 58.62, 58.29, 53.75, 46.30, 37.09, 29.67, 28.43, 27.49, 26.27, 24.12, 22.27, 19.76, 13.69.

**AB-738 - 117**

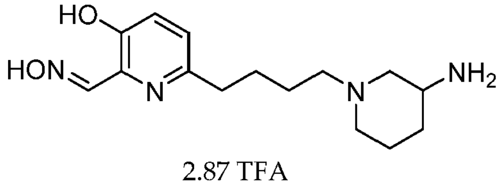

2.87 TFA

Same procedure as for **136,** using **142** as starting material. White off solid (199 mg, 65%). $^1H$ NMR (300 MHz, $CD_3OD$): δ (ppm) 8.41 (s, 1H), 7.87 - 7.74 (m, 1H), 7.61 (t, *J* = 8.9 Hz, 1H), 5.10 (br s, 9H), 3.78 (d, *J* = 10.2 Hz, 1H), 3.70 - 3.41 (m, 2H), 3.20 (dd, *J* = 9.7, 6.5 Hz, 2H), 3.14 - 2.80 (m, 4H), 2.34 - 2.00 (m, 2H), 2.00 - 1.58 (m, 4H), 1.46 (dt, *J* = 15.0, 7.7 Hz, 2H). $^{19}F$ NMR (282 MHz, $CD_3OD$): δ (ppm) -76.80. $^{13}C$ NMR (75 MHz, $CD_3OD$): δ (ppm) 162.93 (q, *J* = 35.0 Hz), 154.51, 151.31, 143.64, 133.46, 132.60, 129.07, 127.78, 123.76, 119.89, 116.01, 112.14, 58.25, 53.67, 52.96, 46.45, 33.95, 30.02, 27.30, 26.78, 24.53. HRMS (ESI+): *m/z* calcd. for C15H25N4O2 293.1978; found 293.1972.

HPLC (method B): $t_R$ = 12.87 min (95%).

**Methyl 3-(benzyloxy)-6-(5-(3-((tert-butoxycarbonyl)amino)piperidin-1-yl)pent-1-yn-1-yl)picolinate -143**

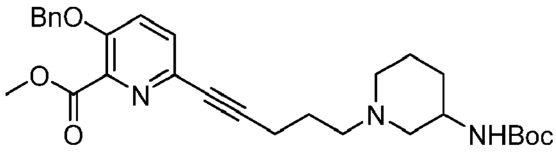

Same procedure as for compound **136,** using commercially available 3-(Boc-amino)piperidine and methyl 3-(benzyloxy)-6-(5-(tosyloxy)pent-1-yn-1-yl)picolinate **59a** as starting materials. Yellow oil (873 mg, 83%). *Rf* = 0.36 (dichloromethane/MeOH, 90/10, v/v). MS (ESI+): *m/z* = 508 [M+H]+. $^1$H NMR (300 MHz, $CDCl_3$): δ (ppm) 7.53 - 7.11 (m, 7H), 5.16 (s, 2H), 5.01 (br s, 1H), 3.92 (s, 3H), 3.70 (br s, 1H), 2.48 (d, *J* = 7.9 Hz, 1H), 2.39 (dd, *J* = 14.7, 7.4 Hz, 5H), 2.25 (d, *J* = 4.6 Hz, 2H), 1.81 - 1.59 (m, 3H), 1.42 (s, 12H). $^{13}$C NMR (75 MHz, $CDCl_3$): δ (ppm) 164.74, 155.07, 152.73, 139.97, 135.46, 135.37, 129.87, 128.59, 128.36, 128.30, 128.08, 127.04, 126.81, 121.71, 89.96, 79.39, 78.81, 70.69, 58.67, 57.29, 53.59, 52.50, 46.28, 29.65, 28.36, 25.52, 22.25, 17.18.

**Tert-butyl (1-(4-(6-formyl-5-hydroxypyridin-2-yl)butyl)piperidin-3-yl)carbamate** - **144**

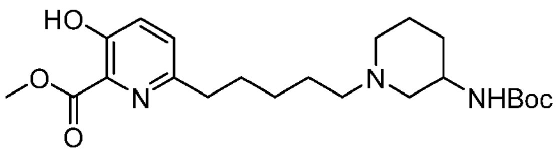

Same procedure as for compound **137** using compound **143** as starting material. Yellow oil (666 mg, 92%). MS (ESI+): *m/z* = 422 $[M+H]^+$. $^1$H NMR (300 MHz, $CDCl_3$): δ (ppm) 10.14 (s, 1H), 7.18 (s, 2H), 5.08 (s, 1H), 3.91 (s, 3H), 3.61 (s, 1H), 3.31 (s, 1H), 2.84 - 2.53 (m, 2H), 2.42 (s, 1H), 2.33 - 2.05 (m, 4H), 1.57 (d, *J* = 7.4 Hz, 4H), 1.45 - 1.17 (m, 15H). $^{13}$C NMR (75 MHz, $CDCl_3$): δ (ppm) 169.86, 156.90, 155.00, 153.77, 128.94, 128.49, 126.40, 78.63, 58.41, 58.29, 53.49, 52.85, 49.85, 46.10, 37.30, 29.73, 29.59, 28.20, 26.95, 26.30, 22.12.

***Tert-butyl* (1-(5-(6-formyl-5-hydroxypyridin-2-yl)pentyl)piperidin-3-yl)carbamate** - **145**

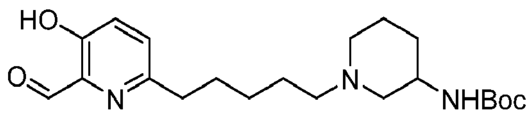

Same procedure as for compound **138,** using compound **144** as starting material Yellow oil (230 mg, 76%). *Rf* = 0.5 (dichloromethane/MeOH, 90/10, v/v). MS (ESI+): *m/z* = 392 $[M+H]^+$; $^1$H NMR (300 MHz, $CDCl_3$): δ (ppm) 10.25 (s, 1H), 9.95 (s, 1H), 7.21 (s, 2H), 4.95 (s, 1H), 3.64 (s, 1H), 2.79 - 2.63 (m, 2H), 2.42 (s, 1H), 2.23 (dd, *J* = 21.7, 14.6 Hz, 5H), 1.63 (dd, *J* = 15.3, 7.6 Hz, 4H), 1.50 - 1.21 (m, 16H). $^{13}$C NMR (75 MHz, $CDCl_3$): δ (ppm) 198.66, 157.00, 155.18, 154.77, 135.72, 129.77, 126.43, 77.16, 59.00, 58.64, 58.31, 53.77, 46.32, 37.11, 28.45, 27.51, 26.29, 24.14, 19.78, 13.71.

**AB-739 - 118**

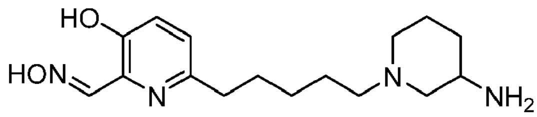

2.38 TFA

Same procedure as for **AB-737** using compound 145 as starting material. Yellow solid (259 mg, 76%). $^{1}H$ NMR (300 MHz, $CD_3OD$) δ (ppm): 8.31 (s, 1H), 7.66 (d, *J* = 8.7 Hz, 1H), 7.47 (d, *J* = 8.7 Hz, 1H), 5.12 (br s, 7H), 3.68 (d, *J* = 11.1 Hz, 1H), 3.53 (dd, *J* = 24.5, 12.3 Hz, 2H), 3.28 - 3.08 (m, 2H), 3.08 - 2.77 (m, 4H), 2.21 - 1.92 (m, 2H), 1.92 - 1.44 (m, 7H). $^{13}C$ NMR (75 MHz, $CD_3OD$) δ (ppm): 162.89 (q, *J* = 35.3 Hz), 154.55, 150.90, 144.62, 134.04, 131.85, 127.54, 123.74, 119.87, 116.00, 112.13, 57.99, 53.72, 53.00, 46.45, 33.89, 27.44, 27.29, 24.31. HPLC (method B): $t_R$ = 14.97 (97.31%). HRMS (ESI+): *m/z* calcd. for C16H27N4O2 307.2134; found 307.2129.

**Methyl 3-(benzyloxy)-6-(4-(4-methylpiperazin-1-yl)but-1-yn-1-yl)picolinate - 160**

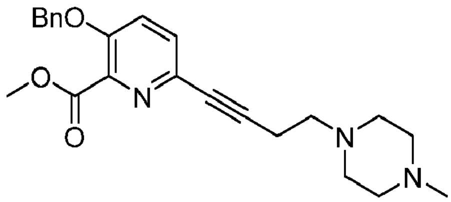

Mesylate **57b** (1.28 g, 3.28 mmol, 1.1 equiv) was dissolved in dry MeCN (33 mL, 0.1 M) under Argon atmosphere and the resulting mixture was cooled down to 0°C. 1-methylpiperazine (340μL, 2.98 mmol, 1.0 equiv.) followed by potassium carbonate (820 mg, 5.96 mmol, 2.0 equiv.) were added in this order. The resulting heterogenous mixture was reflux overnight. Salt were removed by filtration and solvent was evaporated under reduced pressure. The crude product was purified using Silica gel flash chromatography (dichloromethane/MeOH, 95/5, v/v) to give the title compound as a brown oil (509 mg, 43%). *Rf* = 0.2 (dichloromethane/MeOH, 95/5, v/v). MS (ESI+): *m/z* = 394 $[M+H]^+$, 416 [M+Na]+, 426 [M+K]+.. $^{1}H$ NMR (300 MHz, $CDCl_3$): δ (ppm) 7.53 - 7.24 (m, 7H), 5.20 (s, 2H), 3.97 (s, 3H), 2.81 - 2.66 (m, 2H), 2.67 - 2.41 (m, 10H), 2.34 (s, 3H). $^{13}C$ NMR (75 MHz, $CDCl_3$): δ (ppm) 164.57, 152.67, 139.86, 135.30, 134.97, 129.74, 128.46, 127.96, 126.68, 121.55, 88.05, 79.83, 70.53, 56.40, 54.69, 52.39, 52.29, 45.63, 17.37.

**Methyl 3-hydroxy-6-(4-(4-methylpiperazin-1-yl)butyl)picolinate - 161**

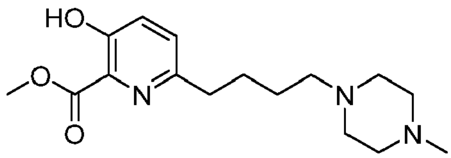

Procedure E on compound **160** (Pearlman's catalyst: 150 mg; MeOH : 200 mL; overnight; no purification). Brown oil (398 mg, quant.). *Rf* = 0.33 (dichloromethane/MeOH, 90/10, v/v). MS (ESI+): *m/z* = 306 $[M+H]^+$, 328 $[M+Na]^+$. $^{1}H$ NMR (300 MHz, $CDCl_3$): δ (ppm) 10.51 (s, 1H), 7.23 (s, 2H), 3.97 (s, 3H), 3.39 (s, 3H), 3.10 - 2.35 (br m, 10H), 1.66 (br s, 4H). $^{13}C$ NMR (75 MHz, $CDCl_3$): δ (ppm) 170.08, 157.35, 129.29, 128.93, 126.89, 57.22, 53.23, 50.65, 50.24, 44.29, 36.99, 27.37.

**3-hydroxy-6-(4-(4-methylpiperazin-1-yl)butyl)picolinaldehyde -162**

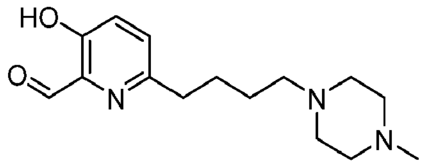

Procedure F on compound **161** (DIBAL-H: 3 equiv.). Brown oil (188 mg, 58% over 3 steps). *Rf* = 0.14 (dichloromethane/MeOH, 90/10, v/v). MS (ESI+): *m/z* (%) = 278 (100) $[M+H]^+$. $^1H$ NMR (300 MHz, $CDCl_3$): δ (ppm) 10.58 (br s, 1H), 9.96 (s, 1H), 7.22 (s, 1H), 7.19 (s, 1H), 2.82 - 2.64 (m, 2H), 2.43 (br s, 7H), 2.37 - 2.27 (m, 3H), 2.24 (s, 3H), 1.68 (dt, *J* = 8.7, 7.3 Hz, 2H), 1.49 (dt, *J* = 10.4, 4.9 Hz, 2H). $^{13}C$ NMR (75 MHz, $CDCl_3$): δ (ppm) 198.31, 156.71, 154.37, 135.52, 129.47, 126.16, 58.03, 54.67, 52.72, 45.64, 36.84, 27.28, 26.08.

**3-hydroxy-6-(4-(4-methylpiperazin-1-yl)butyl)picolinaldehyde oxime - AB639**

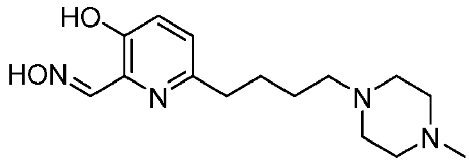

2.2 TFA

Procedure G on compound **162.** Reverse phase purification (method F). White off powder (200.84 mg, 54 %). $^1H$ NMR (300 MHz, $CD_3OD$) δ (ppm) 8.32 (s, 1H), 7.62 (d, *J* = 8.7 Hz, 1H), 7.44 (d, *J* = 8.7 Hz, 1H), 3.50 (s, 8H), 3.12 (s, 2H), 2.88 (s, 5H), 1.73 (s, 4H). $^{13}C$ NMR (75 MHz, $CD_3OD$): δ (ppm) 162.64 (d, *J* = 35.9 Hz), 154.47, 151.41, 145.67, 134.51, 131.04, 127.30, 119.69, 115.83, 57.42, 51.79, 50.03, 43.47, 34.37, 27.53, 24.58. HPLC (method A): $t_R$ = 8.70 (97.68%). HRMS (ESI+): *m/z* calcd. for C15H25N4O2 293.1978; found 293.1975.

**Methyl 3-(benzyloxy)-6-(5-(4-methylpiperazin-1-yl)pent-1-yn-1-yl)picolinate - 163**

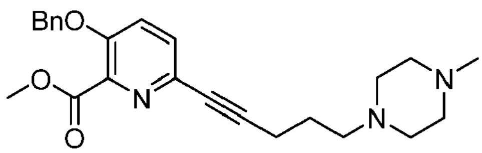

Procedure C using methyl 3-(benzyloxy)-6-(5-((methylsulfonyl)oxy)pent-1-yn-1-yl)picolinate and 1-methylpiperazine as subtrats. Purification: dichloromethane/MeOH + 2% triethylamine, 100/0/2 to 95/5/2, v/v/v. Orange Oil (1.73 g, 76%). *Rf* = 0.18 (dichloromethane/MeOH + 2%triethylamine, 95/5/2, v/v/v). MS (ESI+): m/z (%)= 408 (100). $^1H$ NMR (300 MHz, $CDCl_3$): δ (ppm) 7.65 - 7.20 (m, 7H), 5.23 (s, 2H), 3.98 (s, 3H), 2.84 - 2.40 (m, 12H), 2.35 (s, 4H), 1.91 - 1.72 (m, 2H). $^{13}C$ NMR (75 MHz, $CDCl_3$): δ (ppm) 164.77, 152.84, 139.83, 135.43, 135.31, 129.97, 128.66, 128.16, 126.83, 121.71, 89.89, 79.39, 70.68, 57.26, 54.90, 53.47, 52.80, 52.64, 45.80, 25.50, 17.27.

**Methyl 3-hydroxy-6-(5-(4-methylpiperazin-1-yl)pentyl)picolinate** - **164**

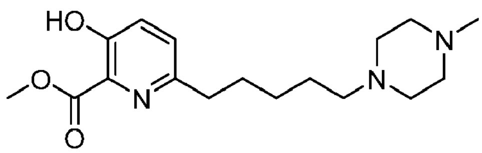

Procedure D on compound **163** (Perlman catalyst in MeOH). Brown solid (1.19 g, 97 %). MS (ESI+): *m/z* = 322 $[M+H]^+$. $^1H$ NMR (300 MHz, $CD_3OD$): δ (ppm) 7.44 (d, *J* = 8.7 Hz, 1H), 7.37 (d, *J* = 8.6 Hz, 1H), 4.03 (s, 3H), 2.86 - 2.70 (m, 3H), 2.52 (d, *J* = 24.6 Hz, 5H), 2.41 (dd, *J* = 9.0, 6.7 Hz, 3H), 2.32 (s, 3H), 1.71 (dt, *J* = 15.4, 7.6 Hz, 2H), 1.63 - 1.45 (m, 2H), 1.45 - 1.26 (m, 2H). $^{13}C$ NMR (75 MHz, $CD_3OD$): δ (ppm) 198.84, 186.25, 183.42, 158.87, 158.20, 156.30, 87.47, 83.47, 81.61, 81.47, 77.16, 73.95, 65.83, 59.19, 56.26, 55.28.

**3-hydroxy-6-(5-(4-methylpiperazin-1-yl)pentyl)picolinaldehyde - 165**

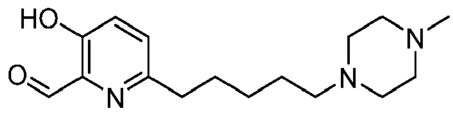

Procedure E on compound **164:** 2,6-lutidine as base, DIBAL-H = 3.0 equiv. Purification = silica gel, dichloromethane/MeOH/triethylamine, 92.5/2.5/2 (v/v/v). Brown redish oil (577 mg, 61% over 3 steps). *Rf* = 0.33 (dichloromethane/MeOH/triethylamine, 95/5/2, v/v/v). MS (ESI+): *m/z* = 295 $[M+H]^+$. $^1H$ NMR (300 MHz, $CDCl_3$): δ (ppm) 10.34 (s, 1H), 10.01 (s, 1H), 7.30 (d, *J* = 8.7 Hz, 1H), 7.26 (d, *J* = 8.8 Hz, 1H), 2.85 - 2.66 (m, 2H), 2.49 (s, 8H), 2.41 - 2.31 (m, 2H), 2.29 (s, 3H), 1.73 (dt, *J* = 15.4, 7.6 Hz, 2H), 1.63 - 1.44 (m, 2H), 1.35 (td, *J* = 14.8, 7.6 Hz, 2H). $^{13}C$ NMR (75 MHz, $CDCl_3$): δ (ppm) 198.02, 156.51, 154.36, 135.41, 129.27, 125.98, 58.01, 54.52, 52.67, 52.57, 45.62, 45.51, 36.81, 29.16, 26.75, 26.19.

**AB-069 - 147**

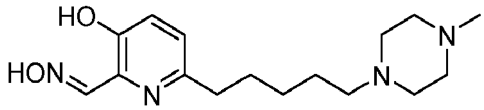

Compound **165** (422 mg, 1.09 mmol, 1.0 equiv.) was dissolved in dried MeOH (11 mL). Then, sodium acetate (369 mg, 4.46 mmol, 4.1 equiv.) was introduced followed by hydroxylamine hydrochloride (304 mg, 4.36 mmol, 4.0 equiv.). The mixture was stirred at room temperature overnight. Concentration under reduced pressure and purification by flash chromatography on silica gel (dichloromethane/MeOH/30% aq $NH_4OH$, 98:2:1 v/v/v) gave **AB-069** as a white-off solid (201 mg, 46%). *Rf* = 0.21 (dichloromethane/MeOH/triethylamine, 97.5/2.5/2, v/v/v). $^1H$ NMR (300 MHz, $CD_3OD$): δ (ppm) 8.28 (s, 1H), 7.26 (d, *J* = 8.5 Hz, 1H), 7.14 (d, *J* = 8.5 Hz, 1H, H), 2.79 - 2.52 (br m, 8H), 2.46 (dd, *J* = 9.0, 6.7 Hz, 2H), 2.38 (s, 3H), 1.79 - 1.64 (m, 2H), 1.64 - 1.47 (m, 2H), 1.46 - 1.26 (m, 4H). $^{13}C$ NMR (75 MHz, $CD_3OD$): δ (ppm) 154.6, 153.8, 152.9, 136.2, 126.0, 125.3, 59.0, 55.0, 53.1, 45.5, 37.7, 30.9, 27.9, 26.9. MS (ESI+): *m/z* = 307 (100%) $[M+H]^+$. HPLC (method A): $t_R$ = 19.32 min (purity: 96 %).

**AB-069 HCl**

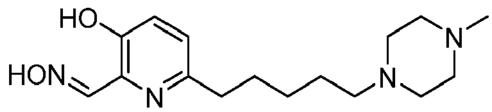

2HCl, $0.5H_2O$

**AB-069** (57.1 mg, 0.186 mmol, 1.0 equiv.) was dissolved in methanol (2.2 mL). Aqueous HCl (650 µL, 0.650 mmol, 3.5 equiv., 1.01 N) was added at rt. After 2 h, concentration to dryness and storage in vacuum desiccator gave the title compound as white off solid (53 mg, 75%). Elemental analysis calculated: N% 15.92, C% 54.61, H% 8.02; found N% 17.03, C% 58.78 H% 6.85. HPLC (method A): $t_R$ = 14.27 (purity : 96.68 %).

**Methyl 6-bromo-3-(methoxymethoxy)picolinate - 4b**

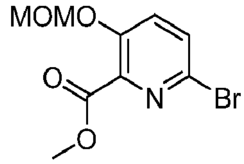

To a solution of **9a** (4.84 g, 20.87 mmol) in acetone (100 mL) was slowly added potassium carbonate (5.8 g, 2 equiv.) and MOMCl (20 mL, 41.74 mmol, 2 equiv.) under argon at 0 °C. The mixture was stirred heated to reflux overnight. Salts were removed by filtration and the crude was concentrated under vacuum. The desired product was obtained after purification by flash chromatography on silica gel (Petroleum ether/EtOAc, 60/40, v/v) as a colourless oil (1.70 g, 94%).

To a solution of **9a** (1 g, 6.5 mmol) in DMF (65 mL) was slowly added NaH (230 mg, 9.75 mmol, 1.2 equiv.) and MOMCl (3 mL, 6.5 mmol, 1.0 equiv.) under argon at 0 °C. The mixture was stirred 1 h and quenched with aq. $NH_4Cl$. The cooled reaction mixture was washed extracted with dichloromethane, washed with water, dried under magnesium sulfate and concentrated under reduced pressure. The desired product was obtained without further purification as a colourless oil (5.18 g, 95%). *Rf* = 0.25 (Petroleum ether/EtOAc 60/40, v/v). $^1H$ NMR (300 MHz, $CDCl_3$) δ (ppm) 7.53 (d, *J* = 8.8 Hz, 1H), 7.49 (d, *J* = 8.8 Hz, 1H), 5.25 (s, 2H), 3.95 (s, 3H), 3.50 (s, 3H). $^{13}C$ NMR (75 MHz, $CDCl_3$) δ (ppm) 52.7, 56.6, 95.1, 127.1, 131.3, 132.1, 140.1, 152.4, 163.9. MS (ESI+): *m/z* (%): 276 (85) $[M+H]^+$ and 278 (100).

**Methyl 6-(4-hydroxybut-1-yn-1-yl)-3-(methoxymethoxy)picolinate** - **23b**

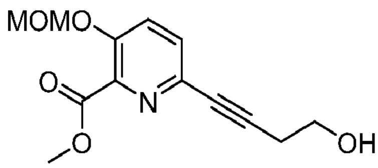

To a degassed solution of **4b** (1.25 g, 4.55 mmol, **1** equiv.) in dry dichloromethane/triethylamine (2/1, v/v, 0.1M) and but-3-yn-1-ol (345 μL, 4.55 mmol, 1.0 equiv) were added $Pd(PPh_3)_4$ (263 mg, 0.23 mmol 0.05 equiv.) CuI (87 mg, 0.46 mmol, 0.1 equiv.) and the solution was stirred under argon at rt overnight in absence of light. The reaction mixture was concentrated under reduced pressure and purifided by flash chromatography on silica gel (Petroleum ether/EtOAc, 60/40 to 30/70, v/v) to give the desired product as a yellow solid (1.13 g, 94%). *Rf* = 0.25 (Petroleum ether/EtOAc, 40/60, v/v). $^1H$ NMR (300 MHz, $CDCl_3$) δ (ppm) 7.54 (d, *J* = 8.8 Hz, 1H), 7.46 (d, *J* = 8.7 Hz, 1H), 5.26 (s, 2H), 3.96 (s, 3H), 3.83 (dd, *J* = 11.8, 5.9 Hz, 2H), 3.51 (s, 3H), 2.70 (t, *J* = 6.3 Hz, 2H), 1.92 (s, 1H). $^{13}C$ NMR (75 MHz, $CDCl_3$) δ (ppm) 164.9, 151.8, 140.5, 136.1, 130.2, 124.2, 95.1, 87.1, 81.0, 77.1, 60.9, 56.7, 52.9, 23.9. MS (ESI+): *m/z* (%): 266 (100) $[M+H]^+$.

**Methyl 6-(4-hydroxybutyl)-3-(methoxymethoxy)picolinate - 24b**

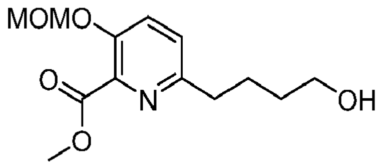

To a solution **of 23b** (550 mg, 1.97 mmol) in dry DMF (20 mL) were added imidazole (410 mg, 7.03 mmol, 3.5 equiv.) and TBDMSCl (460 mg, 3 mmol, 1.5 equiv.). The mixture was stirred at rt overnight. The DMF was removed and

the product was dissolved in EtOAc. The organic layer was washed with brine and dried over magnesium sulfate. Concentration under reduced pressure furnished the desired product as orange oil (775 mg, quant.). $^1$H NMR (300 MHz, CDCl3) δ (ppm) 7.52 (d, J = 8.8 Hz, 1H), 7.42 (d, J = 8.7 Hz, 1H), 5.26 (s, 2H), 3.95 (s, 3H), 3.72 (t, J = 6.0 Hz, 2H), 3.50 (s, 3H), 2.50 (t, J = 7.2 Hz, 2H), 1.92 - 1.68 (m, 2H), 0.90 (s, 9H), 0.06 (s, 6H).

To a solution of silyl ether in degazed MeOH was added Perlman's catalyst (155mg, 20% w/w). The hetogeneous solution was degazed with argon, and hydrogene gas was bubled into the slution. The reaction was stirred at rt, under hydrogene gas atm (1 atm) during 2 h. Subsequent filtration on celite® gave the crude product as an orangeous oil (658 mg, 84%) that was used without further purification in the next step. To a solution of crude product in dry THF was added TBAF (2.2 mL, 1M in dichloromethane, 1.1 equiv) at 0 °C. The mixture was stired at rt overnight. After concentration under vacuum, the crude product was purified using flash chromatography on silica gel (EtOAc/PE, 60/40 to 90/10, v/v) to give the desired compound as yellow oil that crystallized upon standing (413 mg, 78% over 3 steps). *Rf* = 0.5 (EtOAc/PE, 90/10, v/v). $^1$H NMR (300 MHz, $CDCl_3$) δ (ppm) 7.50 (d, *J* = 8.7 Hz, 1H), 7.22 (d, *J* = 8.7 Hz, 1H), 5.22 (s, 2H), 3.95 (s, 3H), 3.65 (t, *J* = 6.3 Hz, 2H), 3.50 (s, 3H), 2.89 - 2.76 (m, 2H), 1.91 (s, 1H), 1.86 - 1.71 (m, 2H), 1.70 - 1.53 (m, 2H). $^{13}$C NMR (75 MHz, $CDCl_3$) δ (ppm) 165.8, 155.1, 151.0, 139.5, 126.0, 125.1, 95.3, 62.5, 56.5, 52.7, 36.8, 32.2, 25.9. MS (ESI+): *m/z* (%): 270 (100) $[M+H]^+$.

**Methyl 6-(4-(4-ethylpiperazin-1-yl)butyl)-3-(methoxymethoxy)picolinate -166**

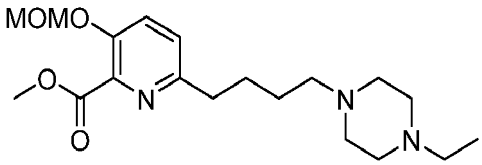

Primary alcohol 24b (1.2 g, 2.23 mmol, 1.0 equiv.) was dissolved in dry dichloromethane (0.1 M) and the resulting mixture was cooled down to 0°C. triethylamine (675 μL, 4.69 mmol, 2.1 equiv.) followed by mesyl chloride (190 μL, 2.45 mmol, 1.1 equiv.) were added dropwise in this order. The resulting mixture was stirred 3 h at rt. The crude mixture is diluted in dichloromethane, washed with water and brine. Organic layer was dried over magnesium sulfate and evaporated under reduced pressure. The residue was dissolved in dry MeCN (0.1M). N-ethylpiperazine (314 μL, 2.45 mmol, 1.1 equiv.) and potassium carbonate (619 mg, 4.46 mmol, 2 equiv.) were added in this order. The resulting heterogenous mixture was reflux overnight. Salt were removed by filtration and solvent was evaporated under reduced pressure. The crude product was purified (silica gel, dichloromethane/MeOH, 95/5, v/v) to give the title compound as a brown oil (253 mg, 31%). *Rf* = 0.21 (dichloromethane/MeOH, 95/5, v/v). MS (ESI+): *m/z* = 366 $[M+H]^+$, 388 $[M+Na]^+$. $^1$H NMR (300 MHz, $CDCl_3$): δ (ppm) 7.43 (d, *J* = 8.6 Hz, 1H), 7.15 (d, *J* = 8.6 Hz, 1H), 5.15 (s, 2H), 3.89 (s, 3H), 3.43 (s, 3H), 2.79 - 2.60 (m, 2H), 2.59 - 2.19 (m, 10H), 1.76 - 1.55 (m, 2H), 1.47 (dt, *J* = 15.1, 7.4 Hz, 2H), 1.01 (t, *J* = 7.2 Hz, 3H). $^{13}$C NMR (75 MHz, $CDCl_3$): δ (ppm) 165.81, 155.12, 150.74, 139.64, 125.73, 124.89, 95.23, 58.38, 56.41, 53.03, 52.64, 52.62, 52.26, 37.20, 27.90, 26.38, 11.83.

**Methyl 6-(4-(4-ethylpiperazin-1-yl)butyl)-3-hydroxypicolinate** - **167**

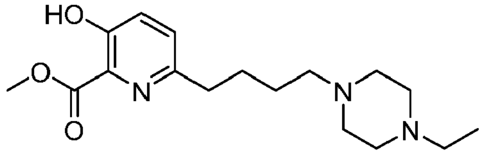

Compound **166** (252 mg, 0.69 mmol, 1 equiv.) in dry dichloromethane (14 mL, 0.05 M) and TFA (3.8 mL, 49 mmol, 72 equiv.) was added at 0°C and the reaction was stirred at this temperature during 2h. The crude was evaporated until dryness and purified on silica gel (dichloromethane/MeOH, 100/0 to 90/10, v/v) to give the title product as an orange oil (47 mg, 22 %). *Rf* = 0.5 (dichloromethane/MeOH, 90/10, v/v). MS (ESI+): *m/z* = 322 $[M+H]^+$, 344 $[M+Na]^+$. $^1$H NMR (300 MHz, $CDCl_3$): δ (ppm) 10.50 (s, 1H), 7.22 (s, 2H), 3.97 (s, 3H), 2.93 - 2.60 (m, 2H), 2.60 - 2.08 (m, 12H), 1.73 - 1.57 (m, 2H), 1.57 - 1.40 (m, 2H), 1.04 (t, *J* = 7.2 Hz, 3H). $^{13}$C NMR (75 MHz, $CDCl_3$): δ (ppm) 170.18, 157.26, 153.82, 129.20, 128.86, 126.71, 58.36, 53.50, 53.19, 52.86, 52.53, 52.30, 37.46, 27.94, 26.36, 11.73.

**AB-588 - 148**

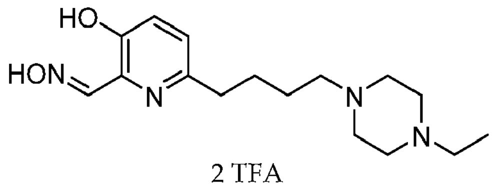

2 TFA

**AB-588** oxime was synthetized using procedure G on compound **167** and was purified using reverse phase purification (method D) to afford the title compound as white off powder (45.4 mg, 75%). $^1H$ NMR (300 MHz, $CD_3OD$): δ (ppm) 8.32 (s, 1H), 7.58 (d, *J* = 8.7 Hz, 1H), 7.41 (d, *J* = 8.7 Hz, 1H), 3.53 (br s, 8H), 3.24 - 3.10 (m, 4H), 2.86 (br s, 2H), 1.74 (br s, 4H), 1.30 (t, *J* = 7.3 Hz, 3H). $^{13}C$ NMR (75 MHz, $CD_3OD$): δ (ppm) 162.86 (q, *J* = 35.3 Hz), 154.40, 151.71, 146.73, 134.74, 130.37, 127.04, 123.71, 119.84, 115.98, 112.11, 57.47, 53.10, 49.99, 49.62, 34.70, 27.56, 24.55, 9.49. HPLC (method B): $t_R$ = 24.31 min (purity: 98.90%). HRMS (ESI+): *m/z* calcd. for C16H27N4O2 307.2134; found 307.2132.

**Methyl 3-(benzyloxy)-6-(5-(4-ethylpiperazin-1-yl)pent-1-yn-1-yl)picolinate - 168**

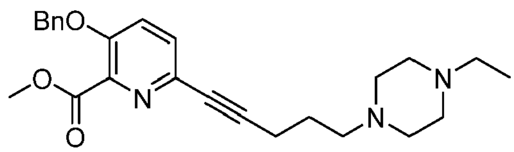

Procedure D was used on methyl 3-(benzyloxy)-6-(5-(tosyloxy)pent-1-yn-1-yl)picolinate using 1-ethylpiperazine (purification: silica gel, dichloromethane/MeOH/triethylamine, 95/5/1, v/v/v) to give the title compound as a brown oil (1.12 g, 92%). *Rf* = 0.29 (dichloromethane/MeOH/triethylamine, 95/5/1, v/v/v). MS (ESI+): *m/z* = 422 $[M+H]^+$. $^1H$ NMR (300 MHz, $CDCl_3$): δ (ppm) 7.55 - 7.25 (m, 7H), 5.23 (s, 2H), 3.98 (s, 3H), 2.68 - 2.39 (br m, 12H), 1.92 - 1.73 (m, 2H), 1.33 (t, *J* = 7.3 Hz, 2H), 1.15 (t, *J* = 7.2 Hz, 1H). $^{13}C$ NMR (75 MHz, $CDCl_3$): δ (ppm) 164.93, 152.98, 140.19, 135.63, 135.58, 130.03, 128.82, 128.32, 127.01, 126.00, 121.90, 90.05, 79.59, 70.95, 57.46, 52.85, 52.75, 52.72, 52.39, 46.16, 25.67, 17.46, 11.75, 9.29.

**Methyl 6-(5-(4-ethylpiperazin-1-yl)pentyl)-3-hydroxypicolinate** - **169**

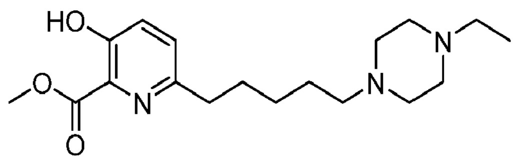

Procedure E (Pearlman's catalyst, MeOH) was used on compound **168** and the crude product was purified using silica gel (dichloromethane/MeOH, 95/5 to 90/10 v/v) to give the title compound as an orange oil (446 mg, 49%). *Rf* = 0.38 (dichloromethane/MeOH, 90/10 v/v). MS (ESI+): *m/z* = 336 $[M+H]^+$. $^1H$ NMR (300 MHz, $CDCl_3$): δ (ppm) 10.52 (s, 1H), 7.22 (s, 2H), 3.97 (s, 3H), 2.98 - 2.53 (m, 12H), 2.53 - 2.32 (m, 2H), 1.79 - 1.42 (m, 4H), 1.42 - 1.23 (m, 2H), 1.23 - 1.04 (m, 3H). $^{13}C$ NMR (75 MHz, $CDCl_3$): δ (ppm) 170.65, 157.74, 154.30, 129.69, 129.34, 129.30, 127.22, 126.45, 58.28, 53.69, 52.59, 51.89, 37.98, 30.28, 27.42.

**AB-557 - 149**

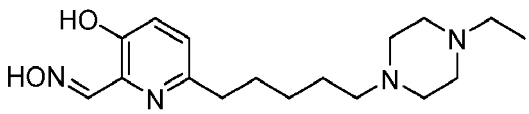

1.9 TFA

Procedure F (DIBAL = 3 equiv.) and procedure G (Reverse phase purification, method D) were used on compound **169** to give the title compound as a brown solid (69.9 mg, 10 % over 4 steps). $^1$H NMR (300 MHz, $CD_3OD$): δ (ppm) 8.31 (s, 1H), 7.58 (d, *J* = 8.6 Hz, 1H), 7.40 (d, *J* = 8.4 Hz, 1H), 3.25 - 3.08 (m, 8H), 2.91 - 2.73 (m, 2H), 1.71 (s, 8H), 1.38 (s, 2H), 1.28 (t, *J* = 7.0 Hz, 3H). $^{13}$C NMR (75 MHz, $CD_3OD$): δ (ppm) 161.63 (q, *J* = 35.4 Hz), 154.31, 134.52, 130.51, 127.08, 119.91, 116.06, 57.68, 53.08, 35.08, 32.83, 30.22, 29.55, 26.90, 26.72, 24.84. HPLC (method B): $t_R$ = 14.52 min (96.1%). HRMS (ESI+): *m/z* calcd. for C17H29N4O2 321.2291; found 321.2287.

**1-(but-3-yn-1-yl)-4-phenylpiperazine - 170**

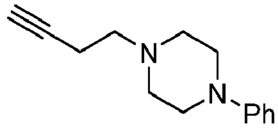

Procedure D was used on but-3-yn-1-yl 4-methanesulfonate (1 equiv.) with N-phenylpiperazine (1 equiv.) and potassium carbonate (1.1 equiv.), normal phase purification (Petroleum ether/EtOAc, 100/0 to 1/1, v/v) gave the title compound as a yellow oil (829 mg, 78%). *Rf* = 0.28 (Petroleum ether/EtOAc, 80/40, v/v). $^1$H NMR (300 MHz, $CD_2Cl_2$): δ (ppm) 7.23 (dd, *J* = 8.7, 7.3 Hz, 2H), 6.90 (d, *J* = 8.0 Hz, 2H), 6.82 (t, *J* = 7.3 Hz, 1H), 3.24 - 3.08 (m, 4H), 2.70 - 2.58 (m, 6H), 2.41 (td, *J* = 7.4, 2.4 Hz, 2H), 2.01 (t, *J* = 2.7 Hz, 1H). $^{13}$C NMR (75 MHz, $CD_2Cl_2$): δ (ppm) 152.06, 129.55, 119.91, 116.40, 83.40, 71.09, 69.25, 68.06, 57.50, 53.49, 49.58, 38.06, 20.26, 17.37.

**Methyl 3-(benzyloxy)-6-(4-(4-phenylpiperazin-1-yl)but-1-yn-1-yl)picolinate - 171**

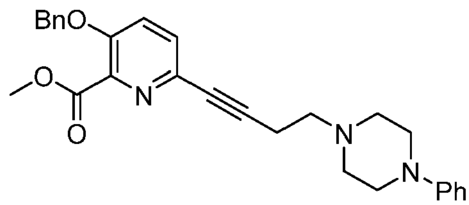

Procedure A was used with compounds **170** and **4a** (purification on silica gel, Petroleum ether/EtOAc/triethylamine, 70/30/1 to 50/50/1, v/v/v), affording the title product as an orange oil (505 mg, 54%, trace of $PPh_3$). *Rf* = 0.28 (Petroleum ether/EtOAc/triethylamine, 50/50/1, v/v/v). $^1$H NMR (300 MHz, $CD_2Cl_2$): δ (ppm) 7.31 - 7.05 (m, 9H), 6.76 (d, *J* = 8.6 Hz, 2H), 6.67 (t, *J* = 7.3 Hz, 1H), 4.98 (s, 2H), 3.76 (s, 3H), 3.11 - 2.94 (m, 4H), 2.49 - 2.40 (m, 4H), 2.36 (t, *J* = 7.1 Hz, 4H), 1.75 - 1.60 (m, 2H). $^{13}$C NMR (75 MHz, $CD_2Cl_2$): δ (ppm) 165.35, 153.25, 152.02, 140.62, 136.28, 135.69, 132.46, 132.38, 132.33, 130.31, 129.46, 129.10, 129.07, 128.91, 128.66, 127.59, 122.10, 119.68, 116.21, 90.23, 79.89, 71.19, 60.67, 57.68, 53.68, 52.82, 49.48, 26.33, 21.27, 17.56, 14.53.

**Methyl 3-hydroxy-6-(4-(4-phenylpiperazin-1-yl)butyl)picolinate** - **172**

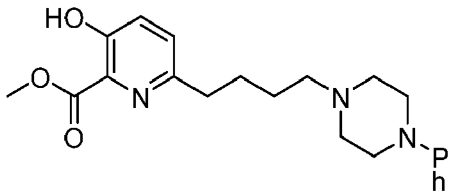

Procedure E (Perlmaan 0.4 equiv. in methanol, 0.005M) was used on compound **172** to give the title compound without purification needed as a light orange oil (178 mg, 99%). $^1H$ NMR (300 MHz, $CDCl_3$): δ (ppm) 10.53 (s, 1H), 7.19 (dd, *J* = 11.0, 4.9 Hz, 4H), 6.91 - 6.67 (m, 3H), 3.96 (s, 3H), 3.23 - 3.05 (m, 4H), 2.79 - 2.69 (m, 2H), 2.66 - 2.53 (m, 4H), 2.50 - 2.36 (m, 2H), 1.76 - 1.59 (m, 2H), 1.60 - 1.41 (m, 2H). $^{13}C$ NMR (75 MHz, $CDCl_3$): δ (ppm) 170.15, 157.29, 153.67, 151.07, 129.26, 129.21, 128.87, 128.68, 128.52, 126.80, 120.07, 116.28, 58.26, 53.20, 53.06, 48.81, 37.32, 29.75, 27.83, 25.94.

**3-hydroxy-6-(4-(4-phenylpiperazin-1-yl)butyl)picolinaldehyde - 173**

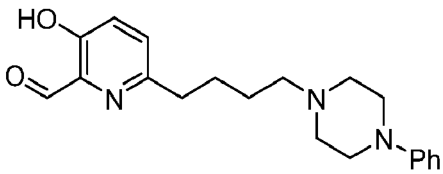

Procedure F (DIBAL = 3 equiv.) was used on compound **172.** The title compound was isolated as an orange oil (67 mg, 38% over 3 steps) using normal phase purification (dichloromethane/MeOH, 100/0 to 85/15, v/v) . *Rf* = 0.62 (dichloromethane/MeOH, 90/10). $^1H$ NMR (300 MHz, $CDCl_3$): δ (ppm) 10.08 (s, 1H), 7.42 - 7.19 (m, 4H), 6.97 (d, *J* = 8.1 Hz, 2H), 6.89 (t, *J* = 7.3 Hz, 1H), 3.37 - 3.14 (m, 4H), 2.87 (t, *J* = 7.6 Hz, 2H), 2.77 - 2.57 (m, 4H), 2.57 - 2.42 (m, 2H), 1.96 - 1.72 (m, 2H), 1.72 - 1.55 (m, 2H). $^{13}C$ NMR (75 MHz, $CDCl_3$): δ (ppm) 198.81, 157.07, 154.78, 151.36, 135.78, 129.81, 129.16, 126.45, 119.75, 116.09, 58.49, 53.35, 49.15, 37.22, 27.65, 26.49.

**AB-277 - 150**

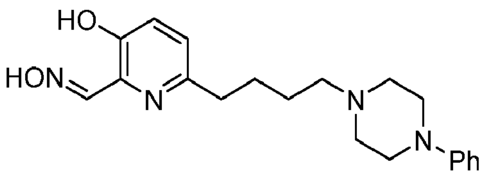

Procedure G was used on compound **173**; purification using silica gel (dichloromethane/MeOH, 95/5 to 90/10, v/v) gave the title product as light orange solid (59 mg, 92 %). *Rf* = 0.5 (dichloromethane/MeOH, 90/10). $^1H$ NMR (300 MHz, $CD_2Cl_2$): δ (ppm) 9.19 (br s, 2H), 8.27 (s, 1H), 7.25 (t, *J* = 8.0 Hz, 2H), 7.14 (d, *J* = 8.5 Hz, 1H), 7.02 (d, *J* = 8.5 Hz, 1H), 6.96 - 6.82 (m, 3H), 3.43 - 3.24 (m, 4H), 3.04 - 2.83 (m, 3H), 2.75 (t, *J* = 6.1 Hz, 4H), 1.85 - 1.65 (m, 4H), 1.26 (t, *J* = 6.0 Hz, 3H). $^{13}C$ NMR (75 MHz, $CD_2Cl_2$): δ (ppm) 154.05, 153.09, 151.15, 135.61, 129.71, 125.43, 124.85, 124.49, 120.91, 116.97, 58.22, 52.94, 48.24, 36.77, 27.58, 24.86, 21.52. HPLC (method A): $t_R$ = 18.85 (purity = 96.45 %). MS (ESI+): *m*/*z* = 355 $[M+H]^+$

**AB-745 - 150a**

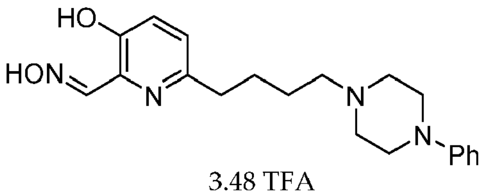

3.48 TFA

**AB-277** (58 mg, 0.164 mmol, 1.0 equiv.) was dissolved in dry methanol (1 mL, 0.1 M), and TFA (1 mL) was added. Concentration until dryness and reverse phase purification gave the title compound as golden white off solid (57.25 mg, 46%). $^1$H NMR (300 MHz, $CD_3OD$): δ (ppm) 8.42 (s, 1H), 7.75 (d, *J* = 8.7 Hz, 1H), 7.56 (d, *J* = 8.7 Hz, 1H), 7.35 - 7.23 (m, 2H), 7.01 (d, *J* = 7.9 Hz, 2H), 6.93 (t, *J* = 7.3 Hz, 1H), 3.98 - 3.48 (m, 4H), 3.29 - 2.86 (m, 8H). $^{13}$C NMR (75 MHz, $CD_3OD$): δ (ppm) 162.48 (d, *J* = 35.9 Hz), 154.46, 151.30, 151.12, 145.60, 134.44, 131.11, 130.33, 127.32, 122.36, 119.80, 117.98, 115.94, 57.44, 53.06, 48.02, 34.32, 27.59, 24.36. HPLC (method B): $t_R$ = 19.42 min (purity: 96.91 %). MS (ESI+): *m/z* = 355 $[M+H]^+$, 386 [M+K]+. HRMS (ESI+): *m/z* calcd. for C20H27N4O2 355.2134; found 355.2148.

**1-(pent-4-yn-1-yl)-4-phenylpiperazine -174**

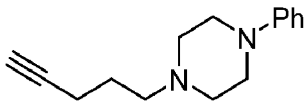

Procedure D was used on but-3-yn-1-yl 4-methanesulfonate (1 equiv.) with N-phenylpiperazine (1 equiv.) and potassium carbonate (1.1 equiv.); normal phase purification (Petroleum ether/EtOAc, 100/0 to 1/1, v/v) gave the title compound as a yellow oil (989 mg, 87%). *Rf* = 0.25 (Petroleum ether/EtOAc, 80/40, v/v). $^1$H NMR (300 MHz, $CD_2Cl_2$): δ (ppm) 7.23 (dd, *J* = 8.7, 7.3 Hz, 2H), 6.90 (d, *J* = 8.0 Hz, 2H), 6.81 (t, *J* = 7.3 Hz, 1H), 3.22 - 3.07 (m, 4H), 2.62 - 2.51 (m, 4H), 2.46 (t, *J* = 7.1 Hz, 2H), 2.26 (td, *J* = 7.1, 2.6 Hz, 2H), 1.98 (t, *J* = 2.7 Hz, 1H), 1.72 (p, *J* = 7.1 Hz, 2H). $^{13}$C NMR (75 MHz, $CD_2Cl_2$): δ (ppm) 152.15, 129.53, 119.79, 116.32, 84.89, 69.95, 69.11, 68.67, 57.61, 53.80, 49.63, 37.68, 28.44, 26.47, 16.75.

**Methyl 3-(benzyloxy)-6-(5-(4-phenylpiperazin-1-yl)pent-1-yn-1-yl)picolinate** - 175

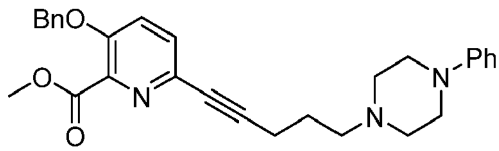

Procedure A was used with compounds **174** and **4a**, purification on silica gel (Petroleum ether/EtOAc/triethylamine, 70/30/1 to 50/50/1, v/v/v) afforded the title product as an orange oil (283 mg, 32%, trace of $PPh_3$). *Rf* = 0.28 (Petroleum ether/EtOAc/triethylamine, 50/50/1, v/v/v). $^1$H NMR (300 MHz, $CD_2Cl_2$): δ (ppm) 7.52 - 7.19 (m, 9H), 7.00 - 6.79 (m, 3H), 5.15 (s, 2H), 3.94 (s, 3H), 3.32 - 3.15 (m, 4H), 2.71 - 2.57 (m, 4H), 2.53 (t, *J* = 7.1 Hz, 4H), 1.85 (p, *J* = 7.2 Hz, 2H), 1.38 - 1.17 (m, 1H). $^{13}$C NMR (75 MHz, $CD_2Cl_2$): δ (ppm) 165.35, 153.25, 152.02, 140.62, 136.28, 135.69, 132.46, 132.38, 132.33, 130.31, 129.46, 129.10, 129.07, 128.91, 128.66, 127.59, 122.10, 119.68, 116.21, 90.23, 79.89, 71.19, 57.68, 53.68, 52.82, 49.48, 26.33, 17.56.

**Methyl 3-hydroxy-6-(5-(4-phenylpiperazin-1-yl)pentyl)picolinate -176**

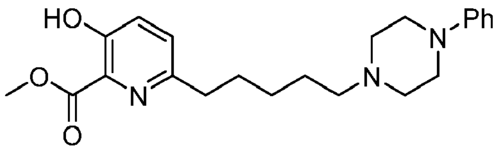

Procedure E (Pearlman's catalyst: 0.4 equiv., methanol as solvent, 0.006 M) was used on compound **175** to give the title compound without purification needed as an orange oil (173 mg, quant.). $^1H$ NMR (300 MHz, $CDCl_3$): δ (ppm) 7.17 (dd, *J* = 8.3, 7.6 Hz, 4H), 6.84 (d, *J* = 8.2 Hz, 2H), 6.76 (t, *J* = 7.2 Hz, 1H), 3.94 (s, 3H), 3.25 - 3.02 (m, 4H), 2.82 - 2.62 (m, 2H), 2.62 - 2.42 (m, 4H), 2.42 - 2.20 (m, 2H), 1.65 (dt, *J* = 15.5, 7.7 Hz, 2H), 1.49 (dt, *J* = 15.0, 7.4 Hz, 2H), 1.32 (dt, *J* = 14.6, 7.4 Hz, 2H). $^{13}C$ NMR (75 MHz, $CDCl_3$): δ (ppm) 170.18, 157.45, 153.95, 151.41, 132.21, 132.08, 129.13, 128.63, 128.47, 126.79, 119.66, 116.04, 58.67, 53.35, 53.18, 49.18, 37.68, 30.10, 27.38, 26.84.

**3-hydroxy-6-(5-(4-phenylpiperazin-1-yl)pentyl)picolinaldehyde - 177**

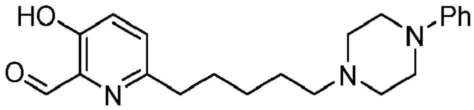

Procedure F (2,6-lutidine, DIBAL = 3 equiv.) was used on compound **176** that was isolated using normal phase purification (dichloromethane/MeOH, 100/0 to 90/10, v/v) to give the title compound as a white off solid (40 mg, 50% over 3 steps). *Rf* = 0.55 (dichloromethane/MeOH, 90/10). $^1H$ NMR (300 MHz, $CDCl_3$): δ (ppm) 10.38 (s, 1H), 10.00 (s, 1H), 7.31 - 7.15 (m, 4H), 6.93 - 6.86 (m, 2H), 6.82 (dd, *J* = 11.4, 4.1 Hz, 1H), 3.33 - 3.08 (m, 4H), 2.85 - 2.67 (m, 2H), 2.67 - 2.46 (m, 4H), 2.37 (dd, *J* = 8.7, 6.6 Hz, 2H), 1.73 (dt, *J* = 15.5, 7.6 Hz, 2H), 1.57 (dt, *J* = 15.2, 7.3 Hz, 2H), 1.46 - 1.30 (m, 2H). $^{13}C$ NMR (75 MHz, $CDCl_3$): δ (ppm) 198.89, 157.09, 155.04, 151.44, 135.82, 132.28, 132.15, 129.84, 129.20, 128.69, 128.53, 126.47, 119.77, 116.12, 58.71, 53.40, 49.21, 37.41, 29.77, 27.35, 26.83.

**AB-269- 151**

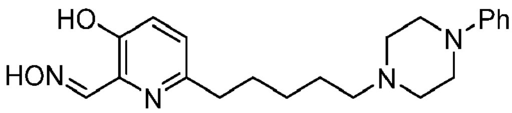

Procedure G was used for compound **177** and the title product was purified by flash chromatography on silica gel (dichloromethane/MeOH, 100/0 to 90/10, v/v) as a a white off solid (29 mg, 70%). *Rf* = 0.2 (dichloromethane/MeOH, 95/5, v/v). $^1H$ NMR (300 MHz, $CD_2Cl_2$): δ (ppm) 10.60 (s, 2H), 8.24 (s, 1H), 7.15 (dd, *J* = 8.5, 7.4 Hz, 2H), 7.06 (d, *J* = 8.4 Hz, 1H), 6.91 (d, *J* = 8.4 Hz, 1H), 6.82 (d, *J* = 8.0 Hz, 2H), 6.75 (t, *J* = 7.3 Hz, 1H), 3.28 - 3.07 (m, 4H), 2.80 - 2.55 (m, 6H), 2.53 - 2.36 (m, 2H), 1.72 - 1.43 (m, 4H), 1.37 - 1.24 (m, 2H). $^{13}C$ NMR (75 MHz, $CD_2Cl_2$): δ (ppm) 153.79, 153.73, 153.04, 151.54, 135.78, 129.63, 124.86, 124.26, 120.48, 116.73, 58.70, 53.32, 48.81, 37.40, 30.24, 27.42, 26.14. HPLC (method A): $t_R$ = 19.63 (98.60%)

**AB-744 - 151a**

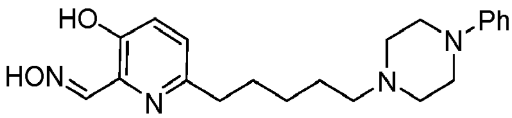

2.93 TFA

Same procedure used for **AB-745** on **AB-269**; white off solid (57.4 mg, 49%). $^1$H NMR (300 MHz, $CD_3OD$): δ (ppm) 8.29 (s, 1H), 7.72 (d, *J* = 8.8 Hz, 1H), 7.51 (d, *J* = 8.8 Hz, 1H), 7.13 (t, *J* = 8.0 Hz, 2H), 6.86 (d, *J* = 8.0 Hz, 2H), 6.77 (t, *J* = 7.3 Hz, 1H), 3.66 (d, *J* = 12.1 Hz, 2H), 3.51 (d, *J* = 9.7 Hz, 2H), 3.13 - 2.73 (m, 8H), 1.78 - 1.54 (m, 4H), 1.45 - 1.22 (m, 2H). $^{13}$C NMR (75 MHz, $CD_3OD$): δ (ppm) 160.5 (q, J = 36 Hz), 153.17, 149.84, 149.75, 141.68, 132.15, 131.51, 128.95, 126.59, 121.01, 116.62, 56.27, 51.67, 46.68, 32.48, 28.75, 25.55, 23.24. HPLC (method B): $t_R$ = 20.00 (95.30%). MS (ESI+): *m*/*z* = 369 (100) [M+H], 401 (5) [M+K]+. HRMS (ESI+): *m*/*z* calcd. for C21H29N4O2 369.2291; found 369.2291.

**Methyl 3-(benzyloxy)-6-(5-(4-benzylpiperazin-1-yl)pent-1-yn-1-yl)picolinate - 173**

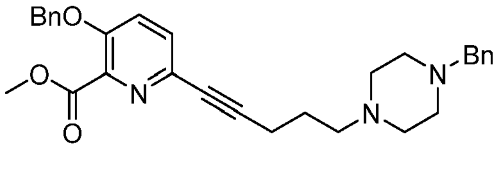

Procedure D was used for mesylate **57a** and *N*-benzylpiperazine, purification by flash chromatography on silica gel (dichloromethane/MeOH/triethylamine, 100/0/1 to 95/5/1, v/v/v) to give the title compound as an orange oil (870 mg, 68%). *Rf* = 0.68 (dichloromethane/MeOH/triethylamine, 90/10/1, v/v/v). $^1$H NMR (300 MHz, $CDCl_3$): δ (ppm) 7.53 - 7.10 (m, 13H), 5.13 (s, 2H), 3.92 (s, 3H), 3.49 (s, 2H), 2.53 - 2.31 (m, 12H), 1.91 - 1.67 (m, 2H). $^{13}$C NMR (75 MHz, $CDCl_3$): δ (ppm) 164.46, 152.44, 139.59, 137.83, 135.19, 135.01, 129.63, 128.78, 128.32, 127.82, 126.62, 126.51, 121.36, 89.69, 79.13, 70.28, 62.69, 57.11, 52.84, 52.75, 52.25, 25.36, 17.00. MS (ESI+): *m*/*z* = 484 (100) $[M+H]^+$.

**Methyl 6-(5-hydroxypent-1-yn-1-yl)-3-(methoxymethoxy)picolinate - 23a**

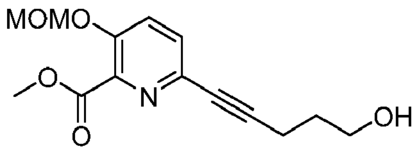

Procedure A was used on 4-pentyn-1-ol and **4b.** Purification by flash chromatography on silica gel (Petroleum ether/EtOAc, 75/25 to 20/80, v/v) gave the title compound as a yellow solid (1.13 mg, 80%). *Rf* = 0.15 (Petroleum ether/EtOAc, 60/40, v/v). $^1$H NMR (300 MHz, $CDCl_3$): δ (ppm) 7.52 (d, *J* = 8.7 Hz, 1H), 7.43 (d, *J* = 8.8 Hz, 1H), 5.26 (s, 2H), 3.95 (s, 3H), 3.80 (dd, *J* = 11.6, 6.0 Hz, 2H), 3.50 (s, 3H), 2.55 (t, *J* = 7.0 Hz, 2H), 1.98 - 1.77 (m, 2H), 1.56 (s, 1H), 1.43 (t, *J* = 5.3 Hz, 1H), 1.27 (d, *J* = 8.5 Hz, 1H), 0.84 (d, *J* = 7.1 Hz, 1H). $^{13}$C NMR (75 MHz, $CDCl_3$): δ (ppm) 164.91, 151.89, 140.51, 136.11, 130.22, 124.20, 95.13, 87.10, 81.07, 77.36, 60.95, 56.76, 52.90, 23.96. MS (ESI+) : *m*/*z* = 280 $[M+H]^+$.

**Methyl 6-(5-hydroxypentyl)-3-(methoxymethoxy)picolinate - 24a**

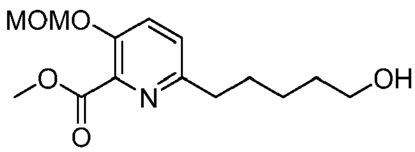

To a solution **of 23b** (562 mg, 2.01 mmol, 1 equiv.) in dry DMF (20 mL, 0.1 M) were added imidazole (411 mg, 7.05 mmol, 3.5 equiv.) and TBDMSCl (463 mg, 3.02 mmol, 1.5 equiv.). The mixture was stirred at rt overnight. The DMF was removed and the product was dissolved in EtOAc. The organic layer was washed with brine and dried over magnesium sulfate. Concentration under reduced pressure furnished the intermediate silyl ether as orange oil that was used without further purification.

To a solution of silyl ether in degassed MeOH was added Perlman's catalyst (158 mg, 20% w/w). The heterogeneous solution was degassed with argon, and hydrogen gas was bubbled into the solution. The reaction was stirred at rt, under hydrogen gas atm (1 atm) during 2 h. Subsequent filtration on celite® gave the crude product reduced product as an orange oil that was used without further purification in the next step.

To a solution of reduced silyl ether in dry THF was added TBAF (2.2 mL, 1 M in dichloromethane, 1.1 equiv.) at 0 °C. The mixture was stirred at rt overnight. After concentration under vacuum, the crude product was purified using flash chromatography on silica gel (EtOAc/PE, 60/40 to 90/10, v/v) to give the desired compound as light-yellow oil (342 mg, 60% over 3 steps).. *Rf* = 0.25 (Petroleum ether/EtOAc,40/60, v/v). $^{1}H$ NMR (300 MHz, $CDCl_3$): δ (ppm) 7.49 (d, *J* = 8.7 Hz, 1H), 7.21 (d, *J* = 8.7 Hz, 1H), 5.20 (s, 2H), 3.92 (s, 3H), 3.58 (t, *J* = 6.5 Hz, 2H), 3.47 (s, 3H), 2.87 - 2.69 (m, 2H), 1.81 - 1.61 (m, 2H), 1.61 - 1.49 (m, 2H), 1.49 - 1.32 (m, 2H). $^{13}C$ NMR (75 MHz, $CDCl_3$): δ (ppm) 165.47, 154.89, 150.21, 139.17, 125.42, 124.52, 94.77, 61.76, 55.95, 52.16, 36.81, 32.06, 29.39, 25.11. MS (ESI+): *m/z* = 284 [M+H]+.

**Methyl 6-(5-(4-benzylpiperazin-1-yl)pentyl)-3-(methoxymethoxy)picolinate - 179**

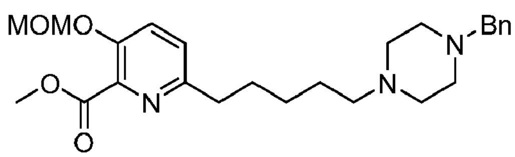

Procedure B was used on **24a** to generate the mesylate that was used without purification on the next steps (procedure D) with *N*-benzyl morpholine (1.2 equiv.) to give the title compound after purification by flash chromatography on silica gel (dichloromethane/MeOH/triethylamine, 100/0/2 to 90/10/2, v/v/v) as an orangeous oil (446 mg, 86%). *Rf* = 0.55 (dichloromethane/MeOH/triethylamine, 90/10/2, v/v/v). $^{1}H$ NMR (300 MHz, $CDCl_3$): δ (ppm) 7.54 (d, *J* = 8.6 Hz, 1H), 7.36 - 7.23 (m, 6H), 5.25 (s, 2H), 3.98 (s, 3H), 3.53 (s, 3H), 2.86 - 2.73 (m, 3H), 2.53 (br s, 10H), 2.44 - 2.31 (m, 2H), 1.86 - 1.66 (m, 2H), 1.63 - 1.52 (m, 2H). $^{13}C$ NMR (75 MHz, $CDCl_3$): δ (ppm) 166.29, 155.50, 150.57, 140.52, 138.56, 132.31, 129.46, 128.99, 128.54, 127.40, 125.82, 125.16, 95.71, 62.96, 58.37, 56.59, 53.22, 52.63, 46.23, 37.45, 30.00, 27.30, 26.28. MS (ESI+) : *m/z* = 442 (100%) $[M+H]^+$.

**Methyl 6-(5-(4-benzylpiperazin-1-yl)pentyl)-3-hydroxypicolinate** - **180**

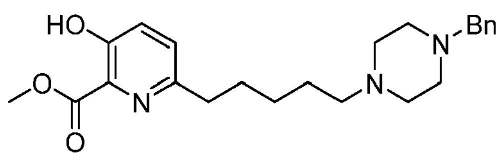

Compound **179** (404 mg, 0.915 mmol, 1 equiv.) was dissolved in dry dichloromethane and TFA was added at 0 °C. The mixture was stirred at rt during 2 h, then excess of TFA was removed under vacuum. The residue was dissolved in dichloromethane and washed with saturated aqueous $Na_2CO_3$. The organic phase was dried with magnesium sulfate and removing of volatiles gave the desired product as an orange oil (233 mg, 64%). *Rf* = 0.57 (dichloromethane/MeOH/triethylamine, 95/5/2, v/v/v). $^{1}H$ NMR (300 MHz, $CDCl_3$): δ (ppm) 7.47 - 7.20 (m, 7H), 4.07 (s, 2H), 3.56 (s, 3H), 2.96 - 2.71 (m, 2H), 2.71 - 2.26 (m, 10H), 1.92-1.69 (m, 2H), 1.69-1.51 (m, 2H), 1.51-1.34 (m, 2H), 1.06-0.81 (m, 1H). MS (ESI+) : 398 $[M+H]^+$.

**6-(5-(4-benzylpiperazin-1-yl)pentyl)-3-hydroxypicolinaldehyde - 181**

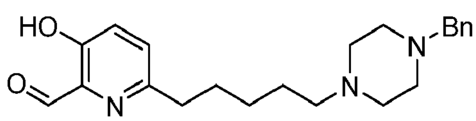

Procedure F was used on compound **180** (2,6-lutidine as base; DIBAL-H = 3 equiv.) and after a normal phase purification (dichloromethane/MeOH, 100/0 to 95/5, v/v/v) the title compound was obtained as yellow oil (94 mg, 43% over 3 steps). $^1$H NMR (300 MHz, $CDCl_3$): δ (ppm) 9.85 (s, 1H), 7.13 (dd, *J* = 7.0, 3.0 Hz, 7H), 3.39 (s, 2H), 2.71-2.22 (m, 12H), 1.62 - 1.42 (m, 4H), 1.26 - 1.15 (m, 3H). $^{13}$C NMR (75 MHz, $CDCl_3$) δ (ppm) 198.74, 157.00, 154.71, 137.36, 135.71, 129.83, 129.28, 128.35, 127.35, 126.42, 62.64, 58.11, 52.76, 51.78, 37.09, 29.36, 26.90, 25.63. HRMS (ESI+): *m*/*z* calcd. for C22H30N3O2 368.2338; found 368.2338.

**AB-153-153**

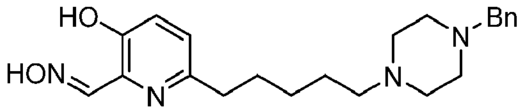

Procedure G was used on compound **181** to give after normal phase purification (dichloromethane/MeOH, 90/10, v/v) the title product as a yellow oil that crystalize upon standing (78.2 mg, 82%). $^1$H NMR (300 MHz, $CD_3OD$): δ (ppm) 8.08 (s, 1H), 7.16 - 7.03 (m, 6H), 6.94 (d, *J* = 8.5 Hz, 1H), 3.45 (s, 2H), 2.94 (s, 4H), 2.74 (dd, *J* = 9.5, 6.8 Hz, 2H), 2.64-2.48 (m, 6H), 1.75 (s, 4H), 1.60-1.45 (m, 4H), 1.26-1.13 (m, 2H). $^{13}$C NMR (75 MHz, $CD_3OD$): δ (ppm) 154.28, 153.77, 152.75, 137.43, 136.27, 130.49, 129.53, 128.83, 126.04, 125.37, 62.73, 57.91, 52.90, 51.11, 37.43, 30.56, 27.22, 25.15, 22.38. MS (ESI+): *m*/*z* (%) = 383 (100) $[M+H]^+$. HPLC (method A): $t_R$ = 17.87 min (98.4%).

**Methyl 3-(benzyloxy)-6-(4-(4-benzylpiperazin-1-yl)but-1-yn-1-yl)picolinate -152**

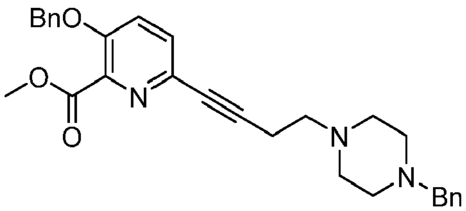

Procedure D was used on mesylate **57b** (1.1 equiv.), potassium carbonate (2.2 equiv.) and N-benzylpiperazine (1 equiv.), purification by chromatography on silica gel (dichloromethane/MeOH, 100/0 to 95/5, v/v) gave the title product as an orange oil (714 mg, 46 %). *Rf* = 0.28 (dichloromethane/MeOH, 96/4, v/v). $^1$H NMR (300 MHz, $CDCl_3$): δ (ppm) 7.45 - 7.19 (m, 12H), 5.20 (s, 2H), 3.95 (s, 3H), 3.51 (s, 2H), 2.74 - 2.64 (m, 2H), 2.64 - 2.27 (m, 10H). $^{13}$C NMR (75 MHz, $CDCl_3$): δ (ppm) 164.67, 152.80, 139.97, 137.98, 135.41, 135.14, 132.00, 131.87, 131.82, 129.83, 129.05, 128.59, 128.48, 128.32, 128.09, 126.91, 126.78, 121.66, 88.31, 79.92, 70.66, 62.89, 56.65, 52.85, 52.69, 52.52, 17.46. MS (ESI+): *m*/*z* = 470 $[M+H]^+$, 492 $[M+Na]^+$, 502 $[M+K]^+$.

**Methyl 6-(4-(4-benzylpiperazin-1-yl)butyl)-3-hydroxypicolinate -183**

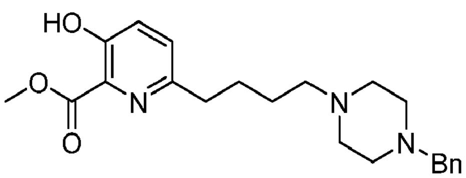

Procedure E was used on compound **182**. After normal phase purification, the title compound was obtained as a brown oil (335 mg, 58%, trace of toluene). *Rf* = 0.5 (dichloromethane/MeOH, 90/10, v/v). $^1$H NMR (300 MHz, $CDCl_3$): δ (ppm) 7.32 - 7.20 (m, 7H), 4.00 (s, 3H), 3.49 (s, 3H), 2.88 - 2.67 (m, 2H), 2.62 - 2.23 (m, 10H), 1.79 - 1.63 (m, 2H), 1.60 - 1.45 (m, 2H). $^{13}$C NMR (75 MHz, $CDCl_3$): δ (ppm) 170.17, 157.26, 153.80, 137.94, 132.21, 129.28, 128.84, 128.64,

128.27, 127.15, 63.00, 58.37, 53.18, 53.13, 52.75, 37.42, 27.94, 26.23. MS (ESI+): *m/z* = 384 $[M+H]^+$.

**6-(4-(4-benzylpiperazin-1-yl)butyl)-3-hydroxypicolinaldehyde -184**

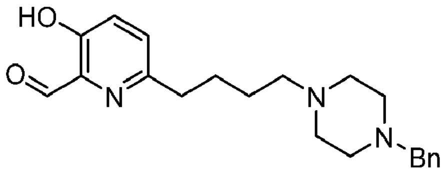

Procedure F (triethylamine as base, DIBAL-H = 3 equiv.) was used on compound **183** and the title compound was isolated after normal phase purification (dichloromethane/MeOH, 100/0 to 90/10, v/v) as a brown oil (216 mg, 70% over 3 steps. TLC-MS (ESI+) : *m/z* = 354 $[M+H]^+$, 376 $[M+Na]^+$; *Rf* = 0.33; . $^1$H NMR (300 MHz, $CDCl_3$): δ (ppm) 10.49 (br s, 1H), 9.95 (s, 1H), 7.37 - 7.03 (m, 7H), 3.44 (s, 2H), 2.86 - 2.62 (m, 2H), 2.60 - 2.18 (m, 8H), 1.78 - 1.57 (m, 2H), 1.57 - 1.36 (m, 2H). $^{13}$C NMR (75 MHz, $CDCl_3$): δ (ppm) 198.53, 156.86, 154.53, 137.82, 135.61, 129.62, 129.11, 128.10, 126.96, 126.26, 62.87, 58.21, 53.02, 52.70, 36.97, 27.44, 26.14.

**AB-641 - 152**

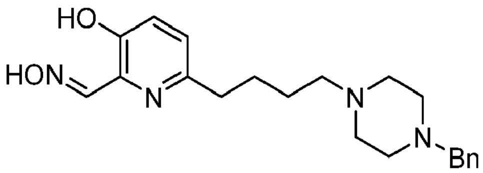

3.2 TFA

Procedure G was used on compound **184**, reverse phase purification (method D) gave the title compound as white off powder (189.84 mg, 42%). $^1$H NMR (300 MHz, $CD_3OD$): δ (ppm) 8.29 (s, 1H), 7.66 (d, *J* = 8.7 Hz, 1H), 7.46 (d, *J* = 8.8 Hz, 1H), 7.41 - 7.25 (m, 5H), 4.17 (s, 2H), 3.44 (s, 4H), 3.35 (d, *J* = 4.1 Hz, 4H), 3.10 (t, *J* = 7.2 Hz, 2H), 2.85 (t, *J* = 6.9 Hz, 2H), 1.81 - 1.56 (m, 4H).$^{13}$C NMR (75 MHz, $CD_3OD$): δ (ppm) 162.33 (q, *J* = 36.5 Hz), 154.59, 150.88, 144.19, 134.08, 131.99, 131.25, 130.96, 130.28, 127.67, 61.55, 57.33, 50.47, 49.71, 33.81, 27.42, 24.37. HPLC (method B): $t_R$ = 17.28 min (purity: 97 %). HRMS (ESI+): *m/z* calcd. for C21H19N4O3 369.2291; found 329.2287.

**Methyl 6-(4-(4-benzhydrylpiperazin-1-yl)but-1-yn-1-yl)-3-(benzyloxy)picolinate - 185**

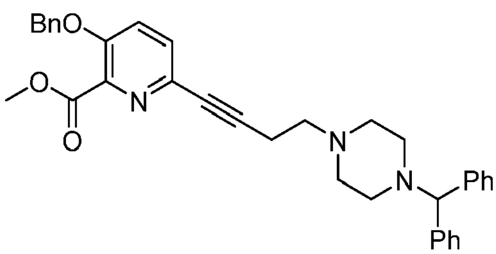

Procedure D was used on mesylate **57b** (1.1 equiv.), potassium carbonate (2 equiv) and N-benzhydrylpiperazine (1 equiv.); purification by chromatography on silica gel (dichloromethane/MeOH, 100/0 to 95/5, v/v) gave the title product as brown foaming solid (767 mg, 43%). TLC-MS (ESI+): *m/z* = 546 $[M+H]^+$; *Rf* = 0.4 (dichloromethane/MeOH, 96/4, v/v); $^1$H NMR (300 MHz, $CDCl_3$): δ (ppm) 7.44 - 7.13 (m, 17H), 5.19 (s, 2H), 4.21 (s, 1H), 3.95 (s, 3H), 2.75-2.63 (br m, 2H), 2.63 - 2.22 (m, 10H). $^{13}$C NMR (75 MHz, $CDCl_3$): δ (ppm) 164.60, 152.70, 142.53, 139.88, 135.35, 135.02, 133.04, 131.91, 131.78, 131.75, 131.66, 129.76, 128.50, 128.41, 128.29, 128.25, 127.99, 127.71, 126.73, 126.70, 121.56, 88.26,

79.87, 75.99, 70.52, 56.55, 52.89, 52.42, 51.61, 17.41.

**Methyl 6-(4-(4-benzhydrylpiperazin-1-yl)butyl)-3-hydroxypicolinate - 186**

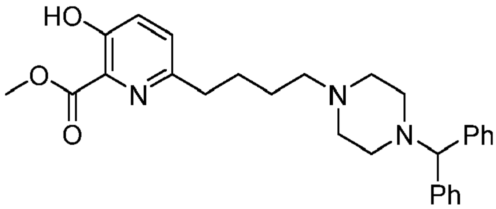

Procedure E (Pearlman's catalyst, MeOH) was used on compound **185** to give after normal phase purification (dichloromethane/MeOH, 100/0 to 90/10) the title compound as a brown oil (574 mg, 89%). *Rf* = 0.52 (dichloromethane/MeOH, 90/10, v/v). $^1H$ NMR (300 MHz, $CDCl_3$): δ (ppm) 7.36 - 7.03 (m, 12H), 4.12 (s, 1H), 3.90 (s, 3H), 2.76 - 2.58 (m, 2H), 2.58 - 2.06 (m, 10H), 1.72 - 1.52 (m, 2H), 1.52 - 1.31 (m, 2H). $^{13}C$ NMR (75 MHz, $CDCl_3$): δ (ppm) 170.07, 157.14, 153.77, 142.73, 132.11, 131.98, 131.92, 131.89, 129.12, 128.74, 128.60, 128.55, 128.41, 127.88, 126.91, 126.85, 126.58, 76.22, 70.96, 58.37, 53.45, 53.07, 51.78, 37.38, 37.08, 27.94, 26.33.

**6-(4-(4-benzhydrylpiperazin-1-yl)butyl)-3-hydroxypicolinaldehyde - 187**

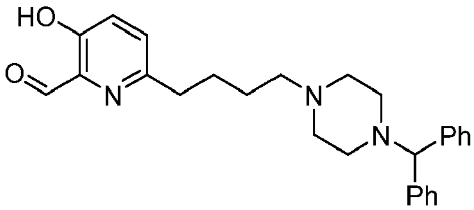

Procedure F (triethylamine as base, DIBAL-H = 3 equiv.) was used on compound **186**, normal phase purification (dichloromethane/MeOH, 100/0 to 90/10, v/v, gave the title compound as an orange oil (499.7 mg, 93% over 3 steps). TLC-MS (ESI+): *m/z* = 430 $[M+H]^+$; *Rf* = 0.5 (dichloromethane/MeOH, 90/10, v/v); .$^1H$NMR (300 MHz, $CDCl_3$): δ (ppm) 10.52 (s, 1H), 9.92 (s, 1H), 7.35 - 7.02 (m, 12H), 4.12 (s, 1H), 2.77-2.58 (m, 2H), 2.51 - 1.89 (m, 10H), 1.73 - 1.53 (m, 2H), 1.53 - 1.35 (m, 2H). $^{13}C$ NMR (75 MHz, $CDCl_3$): δ (ppm) 198.43, 156.80, 154.47, 142.57, 131.97, 131.84, 131.79, 129.56, 128.61, 128.44, 128.31, 128.28, 127.75, 126.90, 126.76, 126.18, 76.04, 58.17, 53.35, 53.30, 51.56, 36.91, 27.40, 26.12.

**AB-643 - 154**

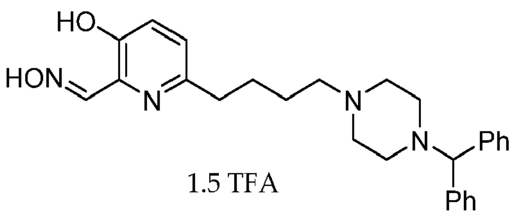

Procedure G was used on compound **187**, reverse phase purification (method A) gave the title compound as a white off powder (444.1 mg, 62%). $^1H$ NMR (300 MHz, $CD_3OD$,): δ (ppm) 8.42 (s, 1H), 7.78 (t, *J* = 10.6 Hz, 1H), 7.58 (t, *J* = 8.9 Hz, 1H), 7.53 - 7.39 (m, 5H), 7.33 (t, *J* = 7.3 Hz, 4H), 7.24 (t, *J* = 7.3 Hz, 2H), 4.52 (d, *J* = 8.9 Hz, 1H), 3.51 - 3.25 br (m, 6H), 3.19 (br s, 3H), 2.98 (br s, 3H), 2.78 (br s, 4H). $^{13}C$ NMR (75 MHz, $CD_3OD$): δ (ppm) 161.65 (q, *J* = 38.1 Hz), 154.59, 150.88, 144.21, 142.20, 134.07, 132.04, 129.93, 128.89, 128.77, 127.65, 119.37, 115.52, 76.37, 57.26,

53.08, 49.87, 33.87, 27.52, 24.38. HPLC (method B): $t_R$ = 24.55 min (purity : 97.99 %). HRMS (ESI+): *m/z* calcd. for C27H33N4O2 445.2604; found 445.2611.

**Methyl 6-(5-(4-benzhydrylpiperazin-1-yl)pent-1-yn-1-yl)-3-(benzyloxy)picolinate - 188**

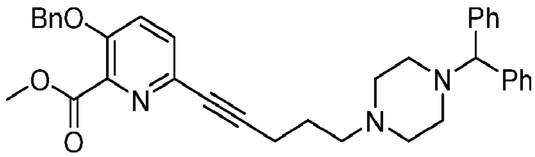

Procedure D was used on mesylate **57a** (1.1 equiv.), potassium carbonate (2 equiv.) and *N*-benzhydrylpiperazine (1.0 equiv.), purification on silica gel (dichloromethane/MeOH, 100/0 to 95/5, v/v) gave the title product as yellow foaming solid (2.21g, 60%). TLC-MS (ESI+): *m/z* = 560 $[M+H]^+$, 582 $[M+Na]^+$.*Rf*= 0.30 (dichloromethane/MeOH, 98/2, v/v); . $^1$H NMR (300 MHz, $CDCl_3$): δ (ppm) 7.73 - 7.16 (m, 17H), 5.24 (s, 2H), 4.36 (s, 1H), 4.04 (s, 3H), 2.77 - 2.43 (m, 12H), 1.98 - 1.83 (m, 2H). $^{13}$C NMR (75 MHz, $CDCl_3$): δ (ppm) 164.66, 152.63, 142.67, 139.92, 135.41, 135.27, 131.94, 131.80, 131.75, 129.76, 128.52, 128.42, 128.29, 128.01, 127.74, 126.73, 121.58, 89.90, 79.34, 76.08, 70.57, 57.32, 53.37, 53.30, 52.42, 51.76, 25.55, 17.24.

**Methyl 6-(5-(4-benzhydrylpiperazin-1-yl)pentyl)-3-hydroxypicolinate - 189**

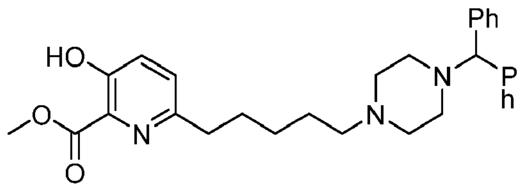

Procedure E (Pearlman's catalyst, MeOH) was used for compound **188** to give the title compound as a yellowish oil (1.69 g, quant.). $^1$H NMR (300 MHz, $CDCl_3$): δ (ppm) 7.34, 7.34, 7.32, 7.22, 7.21, 7.19, 7.19, 7.17, 7.14, 7.13, 7.12, 7.12, 7.10, 4.19, 3.96, 2.73, 2.71, 2.68, 2.60, 2.48, 2.46, 2.43, 2.40, 1.69, 1.66, 1.64, 1.61, 1.59, 1.56, 1.54, 1.51, 1.48, 1.46, 1.34, 1.32, 1.29, 1.27, 1.24, 1.18. $^{13}$C NMR (75 MHz, $CDCl_3$): δ (ppm) 170.21, 157.28, 153.90, 142.50, 141.18, 132.23, 132.10, 129.26, 129.00, 128.87, 128.64, 128.52, 127.91, 127.14, 126.74, 126.13, 76.03, 58.21, 53.21, 50.96, 42.01, 37.52, 29.85, 27.11, 25.83.

**6-(5-(4-benzhydrylpiperazin-1-yl)pentyl)-3-hydroxypicolinaldehyde - 190**

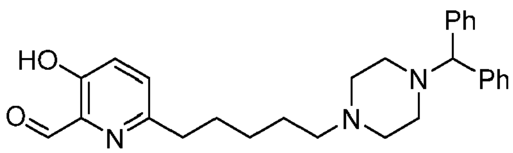

Procedure F (triethylamine as base, DIBAL-H = 3.0 equiv.) was used on compound **189** to give after normal phase purification (dichloromethane/MeOH, 100/0 to 90/10, v/v) the desired product as brown oil (763 mg, 66% over 3 steps). TLC-MS (ESI+): *m/z* = 444 $[M+H]^+$. *Rf* = 0.55 (dichloromethane/MeOH, 90/10, v/v); $^1$H NMR (300 MHz, $CDCl_3$): δ (ppm) 9.94 (s, 1H), 7.95 (br s, 1H), 7.41 - 7.27 (m, 5H), 7.22 - 6.99 (m, 7H), 4.16 (s, 1H), 2.74 - 2.59 (m, 2H), 2.59 - 2.12 (br m, 10H), 1.73 - 1.56 (m, 2H), 1.56 - 1.36 (m, 3H), 1.36 - 1.16 (m, 2H). $^{13}$C NMR (75 MHz, $CDCl_3$): δ (ppm) 198.31, 156.68, 154.50, 142.48, 135.48, 131.89, 131.76, 129.41, 128.35, 128.22, 127.66, 126.66, 126.09, 75.97, 58.23, 53.17, 51.41, 36.91, 29.30, 26.96, 26.19, 25.65.

**AB-555 -155**

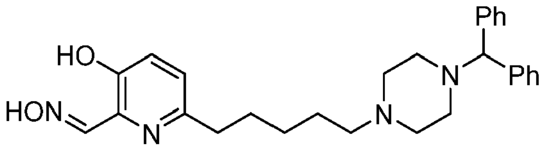

1.46 TFA

Procedure G was used on compound **190**, reverse phase purification (method A) gave the title compound as a yellow solid (416 mg, 39%). $^1$H NMR (300 MHz, $CD_3OD$): δ (ppm) 8.36 (s, 1H), 7.70 (d, *J* = 8.7 Hz, 1H), 7.50 (d, *J* = 8.7 Hz, 1H), 7.43 (dd, *J* = 5.2, 3.4 Hz, 4H), 7.32 - 7.22 (m, 4H), 7.22 - 7.12 (m, 2H), 4.41 (s, 1H), 3.44 - 3.22 (m, 8H), 3.13 - 3.04 (m, 2H), 2.92 - 2.85 (m, 2H), 1.80 - 1.62 (m, 4H), 1.51 - 1.31 (m, 2H). $^{13}$C NMR (75 MHz, $CD_3OD$): δ (ppm) 161.92 (q, *J* = 37.3 Hz), 154.45, 151.74, 144.70, 142.54, 134.04, 131.75, 129.88, 128.88, 128.67, 127.53, 123.40, 119.54, 115.70, 76.34, 57.56, 53.24, 49.89, 49.00, 34.45, 30.16, 26.95, 24.67. HPLC (method B) : $t_R$ 24.75 (98.08%). HRMS (ESI+): *m/z* calcd. for C28H35N4O4 459.2760; found 459.2774.

**Methyl 3-(methoxymethoxy)-6-(4-(4-(pyridin-2-yl)piperazin-1-yl)butyl)picolinate - 191**

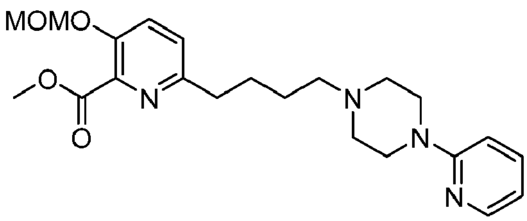

Procedure B was applied to compound **24b** to generate the activated mesylate quantitatively that was used without purification. Then, procedure D was used on the mesylate (1 equiv.) and the 1-(pyridin-2-yl)piperazine (1.1 equiv.) with potassium carbonate (2 equiv.). Normal phase purification (dichloromethane/MeOH, 100/0 to 90/10, v/v) gave the title product as brown oil (428 mg, 46% over 2 steps). TLC-MS (ESI+): *m/z* = 415 [M+H]$^+$, 437 [M+Na]$^+$. *Rf* = 0.37 (dichloromethane/MeOH, 90/10, v/v), . $^1$H NMR (300 MHz, $CDCl_3$) δ (ppm) 8.12 (dd, *J* = 4.9, 1.1 Hz, 1H), 7.47 - 7.35 (m, 2H), 7.18 (d, *J* = 8.7 Hz, 1H), 6.63 - 6.51 (m, 2H), 5.17 (s, 2H), 3.90 (s, 3H), 3.49 (dd, *J* = 10.2, 5.3 Hz, 4H), 3.45 (s, 2H), 2.81 - 2.70 (m, 2H), 2.48 (dd, *J* = 9.3, 4.3 Hz, 4H), 2.42 - 2.30 (m, 2H), 1.78 - 1.60 (m, 2H), 1.60 - 1.43 (m, 2H). $^{13}$C NMR (75 MHz, $CDCl_3$) δ (ppm) 165.82, 159.53, 155.08, 150.70, 147.90, 139.70, 137.42, 128.43, 125.69, 124.87, 113.22, 95.21, 58.48, 56.40, 54.83, 53.47, 53.03, 52.63, 45.12, 37.20, 27.89, 26.38.

**Methyl 3-hydroxy-6-(4-(4-(pyridin-2-yl)piperazin-1-yl)butyl)picolinate-192**

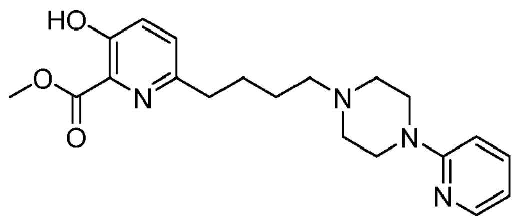

Compound **191** (428 mg, 1.03 mmol, 1.0 equiv.) was dissolved in dry dichloromethane (20 mL, 0.05 M). TFA (5 mL) was added dropwise at 0 °C and the mixture was stirred for 2 h at rt. Concentration to dryness and subsequent normal phase purification (dichloromethane/MeOH, 100/0 to 90/10, v/v) gave the title compound as a brown oil (116 mg, 31%). TLC-MS (ESI+) : *m/z* = 371 [M+H]$^+$, 393[M+Na]$^+$. *Rf* =0.59 (dichloromethane/MeOH, 90/10, v/v); . $^1$H NMR

(300 MHz, $CDCl_3$): δ (ppm) 10.46 (br s, 1H), 8.10 (dd, *J* = 4.8, 1.2 Hz, 1H), 7.49 - 7.30 (m, 1H), 7.22 (s, 2H), 6.63 - 6.41 (m, 2H), 3.95 (s, 3H), 3.53 - 3.40 (m, 4H), 2.83 - 2.66 (m, 2H), 2.56 - 2.42 (m, 4H), 2.42 - 2.22 (m, 2H), 1.77 - 1.60 (m, 2H), 1.60 - 1.42 (m, 2H). $^{13}C$ NMR (75 MHz, $CDCl_3$): δ (ppm) 170.14, 159.51, 157.23, 153.80, 147.96, 137.46, 129.19, 128.83, 126.69, 113.32, 107.07, 58.47, 53.16, 53.03, 45.09, 37.43, 27.93, 26.33.

**AB-589 - 156**

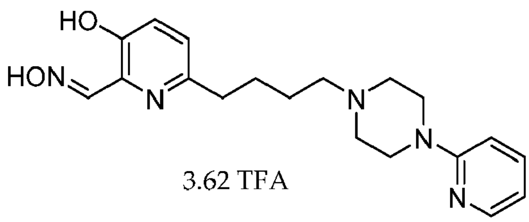

3.62 TFA

Procedure F (triethylamine, DIBAL-H = 4 equiv.) was applied to compound **192** to generate the 3-hydroxy-6-(4-(4-(pyridin-2-yl)piperazin-1-yl)butyl)picolinaldehyde that was isolated with impurities using normal phase purification. TLC-MS(ESI+): *m/z* = 341 $[M+H]^+$, 363 $[M+Na]^+$. *Rf* = 0.33 (dichloromethane/MeOH, 90/10, v/v). The crude aldehyde was used without further purification in the oxime generation (procedure G). Subsequent reverse phase purification (method A) gave the title product as a tan solid (71.21 mg, 27% over 4 steps). $^1H$ NMR (300 MHz, $CD_3OD$): δ (ppm) 8.42 (s, 1H), 8.13 (dd, *J* = 5.9, 1.2 Hz, 1H), 8.02 (ddd, *J* = 9.0, 7.2, 1.8 Hz, 1H), 7.73 (d, *J* = 8.7 Hz, 1H), 7.55 (d, *J* = 8.7 Hz, 1H), 7.34 (d, *J* = 9.1 Hz, 1H), 7.07 (dd, *J* = 9.6, 3.4 Hz, 1H), 4.01 (s, 4H), 3.52 (s, 4H), 3.32 - 3.24 (m, 2H), 2.99 (t, *J* = 7.0 Hz, 2H), 1.98 - 1.73 (m, 4H). $^{13}C$ NMR (75 MHz, $CD_3OD$): δ (ppm) 161.13 (q, *J* = 37.6 Hz), 154.10, 153.12, 149.87, 144.15, 142.94, 140.03, 133.03, 129.87, 125.98, 114.50, 111.55, 56.20, 50.63, 43.00, 32.87, 26.13, 22.90. HRMS (ESI+): *m/z* calcd. for C19H26N5O2 356.2087; found 356.2088. HPLC (method B) : $t_R$ = 15.58 min (purity : 97.09 %).

**Methyl 3-(benzyloxy)-6-(5-(4-(pyridin-2-yl)piperazin-1-yl)pent-1-yn-1-yl)picolinate - 193**

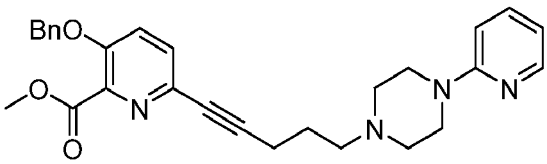

Procedure D was used on tosylate **59a** (1.0 equiv.) and the 1-(pyridin-2-yl)piperazine (1.1 equiv.) with potassium carbonate (2.0 equiv.). Normal phase purification (dichloromethane/MeOH, 99/1 to 85/15, v/v) gave the title compound as a brown oil (1.04g, 72%). TLC-MS (ESI+): *m/z* = 471 [M+H]+, 493 [M+Na]+; *Rf* = 0.29 (dichloromethane/MeOH, 95/5, v/v). $^1H$ NMR (300 MHz, $CDCl_3$): δ (ppm) 8.25 - 8.13 (m, 1H), 7.54 - 7.19 (m, 8H), 6.71 - 6.51 (m, 2H), 5.18 (s, 2H), 3.95 (s, 3H), 3.59 - 3.48 (m, 4H), 2.63 - 2.52 (m, 4H), 2.52 - 2.44 (m, 4H), 1.97 - 1.71 (m, 2H).

**Methyl 3-hydroxy-6-(5-(4-(pyridin-2-yl)piperazin-1-yl)pentyl)picolinate -194**

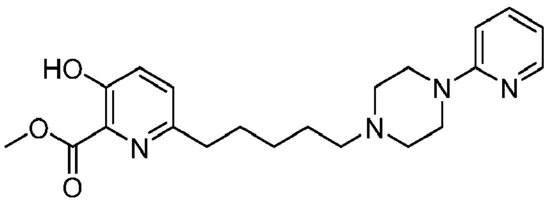

Procedure E (Pearlman's catalyst, MeOH) was used on compound **193** to give the title compound without purification as a brown oil (796 mg, 95%). $^1$H NMR (300 MHz, $CDCl_3$): δ (ppm) 10.36 (br s, 1H), 8.18 (d, *J* = 3.7 Hz, 1H), 7.52 (t, *J* = 7.3 Hz, 1H), 7.31 (s, 2H), 6.82 - 6.53 (m, 2H), 4.04 (s, 3H), 3.91 (br s, 4H), 3.06 (br s, 2H), 2.92 - 2.69 (m, 4H), 2.62 (br s, 2H), 1.87 (br s, 2H), 1.83 - 1.71 (m, 2H), 1.48 - 1.33 (m, 2H). $^{13}$C NMR (75 MHz, $CDCl_3$): δ (ppm) 169.85, 160.30, 158.13, 156.96, 153.24, 147.74, 137.67, 129.06, 126.48, 114.18, 107.24, 57.42, 52.92, 51.56, 43.00, 36.98, 29.20, 26.32, 24.06, 20.22, 18.78.

**3-hydroxy-6-(5-(4-(pyridin-2-yl)piperazin-1-yl)pentyl)picolinaldehyde - 195**

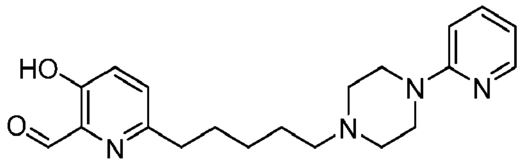

Procedure F (triethylamine as base and DIBAL = 4 equiv.) was applied to compound **194** to give after normal phase purification (dichloromethane/MeOH, 100/0 to 90/10, v/v) the title compound as a brown oil (311 mg, 44% over 3 steps, traces of TBAF remaining). *Rf* = 0.56 (dichloromethane/MeOH, 90/10, v/v). $^1$H NMR (300 MHz, $CDCl_3$): δ (ppm) 9.96 (s, 1H), 8.08 (dd, *J* = 4.9, 1.3 Hz, 1H), 7.37 (ddd, *J* = 7.2, 5.7, 2.0 Hz, 1H), 7.31 - 7.12 (m, 2H), 6.66 - 6.41 (m, 2H), 3.57 - 3.36 (m, 4H), 2.81 - 2.62 (m, 2H), 2.60 - 2.40 (m, 4H), 2.40 - 2.23 (m, 2H), 1.65 - 1.52 (m, 2H + TBAF), 1.37 (dt, *J* = 14.7, 7.4 Hz, 2H + TBAF). $^{13}$C NMR (75 MHz, $CDCl_3$): δ (ppm) 197.72, 159.13, 156.69, 154.40, 147.54, 137.16, 135.58, 129.39, 126.20, 112.99, 106.80, 58.22, 52.63, 44.69, 36.92, 29.28, 26.84, 26.16, 19.43.

**AB-451- 157**

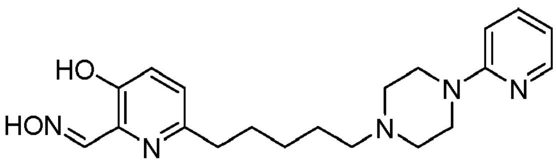

3.5 TFA

Procedure G was used on compound **195** and reverse phase purification gave the title compound as a brown oil (6.51 mg, 1%). $^1$H NMR (300 MHz, $CD_3OD$): δ (ppm) 8.38 (s, 1H), 8.14 (dd, *J* = 5.5, 1.1 Hz, 1H), 8.00 - 7.82 (m, 1H), 7.65 (d, *J* = 8.7 Hz, 1H), 7.48 (d, *J* = 8.7 Hz, 1H), 7.19 (d, *J* = 8.9 Hz, 1H), 7.06 - 6.85 (m, 1H), 3.46 (s, 4H), 3.22 - 3.15 (m, 2H), 2.97 - 2.80 (m, 2H), 1.89 - 1.74 (m, 4H), 1.57 - 1.39 (m, 2H), 1.39 - 1.21 (m, 4H). $^{13}$C NMR (75 MHz, $CD_3OD$:) δ (ppm) 161.13 (q, *J* = 37.8 Hz), 157.00, 154.34, 152.29, 146.40, 144.10, 142.66, 134.56, 130.60, 127.12, 116.04, 111.71, 57.88, 52.28, 49.00, 44.26, 35.08, 30.25, 26.95, 24.67. HRMS (ESI+): *m/z* calcd. for C20H28N5O2 370.2235; found 370.2243. HPLC (method B) : $t_R$ = 16.15 min (purity = 95.5%).

**Methyl 3-(benzyloxy)-6-(4-(4-(pyrimidin-2-yl)piperazin-1-yl)but-1-yn-1-yl)picolinate - 196**

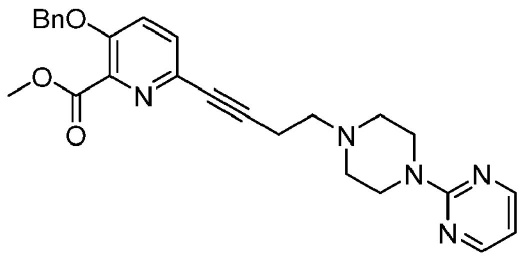

Procedure D was used with mesylate **57b** (1.2 equiv.) and 2-(piperazin-1-yl)pyrimidine (1.0 equiv.) with potassium carbonate (2.0 equiv.). Normal phase purification (dichloromethane/MeOH, 100/0 to 90/10, v/v) gave the title compound as a brown oil (658 mg, 48%). TLC-MS (ESI+): *m/z* = 458 $[M+H]^+$, 480 $[M+Na]^+$. *Rf* = 0.5 (dichloromethane/MeOH, 90/10, v/v). $^1H$ NMR (300 MHz, $CDCl_3$): δ (ppm) 8.34 (d, *J* = 4.7 Hz, 2H), 7.52 - 7.23 (m, 8H), 6.51 (t, *J* = 4.7 Hz, 1H), 5.22 (s, 2H), 3.99 (s, 3H), 3.92 - 3.80 (m, 4H), 2.80 - 2.72 (m, 2H), 2.72 - 2.64 (m, 2H), 2.64 - 2.50 (m, 4H). $^{13}C$ NMR (75 MHz, $CDCl_3$): δ (ppm) 164.62, 161.43, 157.51, 152.77, 139.91, 135.35, 135.02, 131.79, 129.81, 128.53, 128.44, 128.28, 128.04, 126.74, 121.62, 109.70, 88.12, 79.96, 70.60, 56.65, 52.52, 52.46, 43.42, 17.48.

**Methyl 3-hydroxy-6-(4-(4-(pyrimidin-2-yl)piperazin-1-yl)butyl)picolinate - 197**

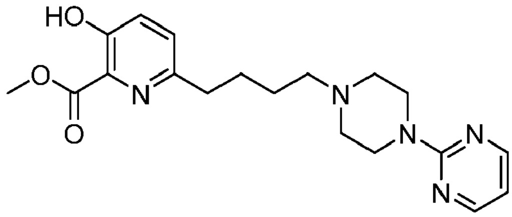

Procedure E (Pearlman's catalyst, MeOH) was applied to compound **196** that was isolated using normal phase purification (dichloromethane/MeOH, 100/0 to 90/10, v/v) to obtain the title compound as a brown oil (104 mg, 19%). TLC-MS (ESI+): *m/z* = 372 $[M+H]^+$, 394 $[M+Na]^+$*Rf* = 0.5 (dichloromethane/MeOH, 90/10, v/v). $^1H$ NMR (300 MHz, $CDCl_3$): δ (ppm) 8.32 (d, *J* = 4.7 Hz, 2H), 7.33 (s, 2H), 6.49 (t, *J* = 4.7 Hz, 1H), 4.06 (s, 3H), 3.93 - 3.76 (m, 4H), 2.91 - 2.78 (m, 2H), 2.56 - 2.49 (m, 4H), 2.49 - 2.36 (m, 2H), 1.87 - 1.69 (m, 2H), 1.69 - 1.51 (m, 2H). $^{13}C$ NMR (75 MHz, $CDCl_3$): δ (ppm) 170.10, 161.64, 157.68, 157.19, 153.77, 129.13, 126.65, 109.79, 58.46, 53.13, 53.06, 43.57, 37.41, 27.91, 26.34.

**3-Hydroxy-6-(4-(4-(pyrimidin-2-yl)piperazin-1-yl)butyl)picolinaldehyde - 198**

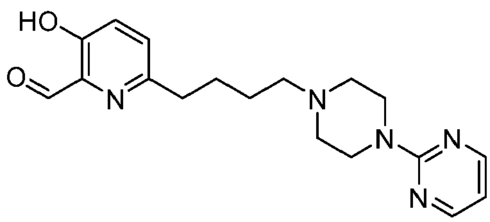

Procedure F (triethylamine as base, DIBAL-H = 3 equiv.) was used on compound **197**. Purification by flash chromatography on silica gel (dichloromethane/MeOH, 100/0 to 90/10, v/v) gave the title compound as light-yellow solid (93 mg, 98 % over 3 steps). TLC-MS (ESI+): *m/z* = 342 $[M+H]^+$, 364 $[M+Na]^+$; *Rf* = 0.5 (dichloromethane/MeOH, 90/10, v/v). $^1H$ NMR (300 MHz, $CDCl_3$): δ (ppm) 10.33 (s, 1H), 9.96 (s, 1H), 8.22 (d, *J* = 4.7 Hz, 2H), 7.24 (d, *J* = 8.7 Hz, 1H), 7.21 (d, *J* = 8.8 Hz, 1H), 6.40 (t, *J* = 4.7 Hz, 1H), 3.90 - 3.65 (m, 4H), 2.75 (t, *J* = 7.6 Hz, 2H), 2.56 - 2.41 (m, 4H), 2.41 - 2.27 (m, 2H), 1.81 - 1.63 (m, 2H), 1.54 (dt, *J* = 9.7, 6.9 Hz, 2H). $^{13}C$ NMR (75 MHz, $CDCl_3$): δ (ppm) 198.80, 161.71, 157.78, 157.08, 154.74, 135.80, 129.82, 126.48, 109.95, 58.55, 53.17, 43.61, 37.19, 27.61, 26.37.

**AB-647 - 158**

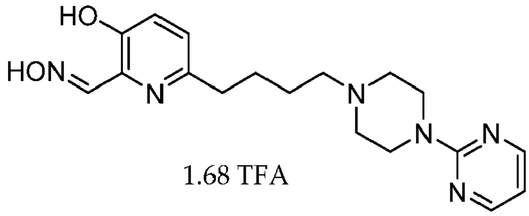

1.68 TFA

Procedure G was applied to compound **198**, reverse phase purification (method A) gave the title product as a white off solid (67.31 mg, 45%). $^1H$ NMR (300 MHz, $CD_3OD$): δ (ppm) 8.16 (s, 1H), 8.12 (d, *J* = 4.8 Hz, 2H), 7.59 (d, *J* = 8.8 Hz, 1H), 7.38 (d, *J* = 8.8 Hz, 1H), 6.44 (t, *J* = 4.8 Hz, 1H), 4.61 (br s, 2H), 3.34 (br s, 4H), 3.00 - 2.85 (m, 3H), 2.83 - 2.64 (m, 3H), 1.73 - 1.42 (m, 4H). $^{13}C$ NMR (75 MHz, $CD_3OD$): δ (ppm) 162.12, 159.24, 154.59, 150.89, 144.36, 133.98, 132.01, 127.66, 112.71, 57.56, 52.70, 42.02, 33.84, 27.51, 24.29. HRMS (ESI+): *m/z* calcd. for C18H25N6O2 357.2039; found 357.2040. HPLC (method B) : $t_R$ = 17.23 min (purity = 95.6%).

**Methyl 3-(benzyloxy)-6-(5-(4-(pyrimidin-2-yl)piperazin-1-yl)pent-1-yn-1-yl)picolinate - 199**

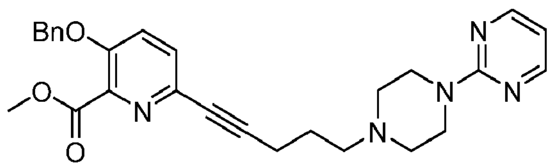

Procedure D was used with mesylate **57a** (1.0 equiv.) and 2-(piperazin-1-yl)pyrimidine (1.0 equiv.) with potassium carbonate (2 equiv.). Normal phase purification (dichloromethane/MeOH, 100/0 to 90/10, v/v) gave the title compound as a brown oil (4.15 g, 78%). *Rf* = 0.4 (dichloromethane/MeOH, 95/5, v/v). $^1H$ NMR (300 MHz, $CD_2Cl_2$): δ (ppm) 8.28 (d, *J* = 4.7 Hz, 2H), 7.53 - 7.22 (m, 7H), 6.46 (t, *J* = 4.7 Hz, 1H), 5.16 (s, 2H), 3.92 (s, 3H), 3.86 - 3.68 (m, 4H), 2.56 - 2.42 (m, 8H), 1.92 - 1.73 (m, 2H). $^{13}C$ NMR (75 MHz, $CD_2Cl_2$): δ (ppm) 165.39, 162.36, 158.14, 153.33, 140.68, 136.32, 135.79, 130.34, 129.17, 128.74, 127.67, 122.17, 110.22, 90.25, 79.86, 71.34, 57.82, 53.60, 52.89, 44.23, 26.32, 17.59.

**Methyl 3-hydroxy-6-(5-(4-(pyrimidin-2-yl)piperazin-1-yl)pentyl)picolinate - 200**

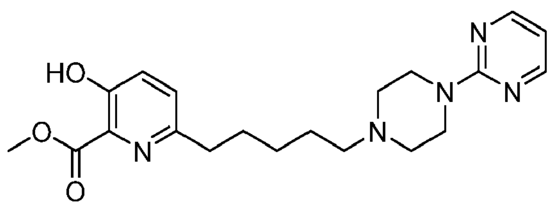

Procedure E was applied on compound **199** to give without purification the title compound as a brown oil (3.4 g, quant.). $^1H$ NMR (300 MHz, $CD_2Cl_2$): δ (ppm) 8.30 (d, *J* = 4.7 Hz, 2H), 7.33 (d, *J* = 8.6 Hz, 1H), 7.29 (d, *J* = 8.6 Hz, 1H), 6.49 (br s), 6.49 (t, *J* = 4.7 Hz, 2H), 4.03 (s, 3H), 3.87 - 3.73 (m, 4H), 2.84 - 2.72 (m, 2H), 2.49 - 2.44 (m, 4H), 2.40 - 2.33 (m, 2H), 1.80 - 1.62 (m, 3H), 1.62 - 1.48 (m, 2H), 1.48 - 1.23 (m, 3H). $^{13}C$ NMR (75 MHz, $CD_2Cl_2$): δ (ppm) 170.80, 162.39, 158.16, 157.71, 154.35, 129.57, 129.51, 126.91, 110.20, 59.07, 53.68, 53.37, 44.26, 37.98, 30.49, 27.72, 27.24.

**3-Hydroxy-6-(5-(4-(pyrimidin-2-yl)piperazin-1-yl)pentyl)picolinaldehyde - 201**

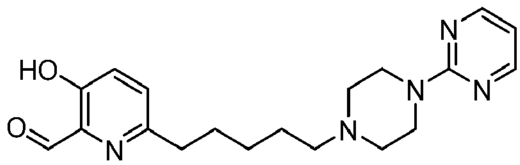

Procedure F (triethylamine as base, DIBAL-H = 4 equiv.) was applied to compound **200**. Normal phase purification (dichloromethane/MeOH, 100/0 to 90/10, v/v) gave the desired product as a yellow solid (1.46 g, 40 % over 3 steps). $^1$H NMR (300 MHz, $CDCl_3$): δ (ppm) 10.04 (s, 1H), 9.73 (s, 1H), 8.30 (d, *J* = 4.7 Hz, 2H), 7.32 (d, *J* = 8.7 Hz, 1H), 7.28 (d, *J* = 8.8 Hz, 1H), 6.47 (t, *J* = 4.7 Hz, 1H), 3.94 - 3.76 (m, 4H), 2.90 - 2.72 (m, 2H), 2.63 - 2.44 (m, 4H), 2.44 - 2.31 (m, 2H), 1.87- 1.69 (m, 2H), 1.69- 1.50 (m, 2H), 1.50 - 1.31 (m, 2H). $^{13}$C NMR (75 MHz, $CDCl_3$): δ (ppm) 198.47, 161.42, 157.46, 156.68, 154.62, 135.51, 129.46, 126.12, 109.58, 58.44, 52.92, 43.40, 37.02, 29.38, 26.98, 26.43.

**AB-508 - 159**

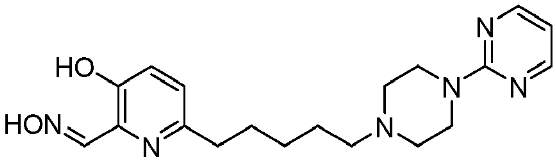

Procedure G was applied to compound **201**. Normal phase purification (dichloromethane/MeOH, 100/0 to 90/10, v/v) gave the title product as a white off powder (1.01 g, 66%). *Rf* = 0.5 (dichloromethane/MeOH, 90/10, v/v). $^1$H NMR (300 MHz, $CDCl_3$): δ (ppm) 9.82 (s, 2H), 8.42 (s, 1H), 8.34 (d, *J* = 4.8 Hz, 2H), 7.29 (s, 1H), 7.20 (d, *J* = 8.5 Hz, 1H), 7.03 (d, *J* = 8.5 Hz, 1H), 6.53 (t, *J* = 4.8 Hz, 1H), 4.02 - 3.79 (m, 4H), 2.83 - 2.69 (m, 2H), 2.69-2.53 (m, 4H), 2.53 - 2.36 (m, 2H), 1.85 - 1.53 (m, 4H), 1.53 - 1.29 (m, 2H). $^{13}$C NMR (75 MHz, $CDCl_3$): δ (ppm) 161.67, 157.89, 153.89, 153.44, 152.68, 135.20, 124.61, 123.98, 110.17, 58.73, 53.05, 43.34, 37.22, 30.03, 27.19, 26.30. HRMS (ESI+): *m/z* calcd. for $C_{19}H_{27}N_6O_2$ 371.2195; found 371.2191. HPLC (method B): $t_R$ = 17.88 min (purity = 97.25%).

**2-(5-bromothiophen-2-yl)-1,3-dioxolane - 202**

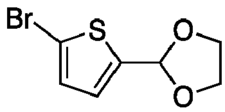

5-Bromo-2-thiophene-carboxaldehyde (3.76 g, 19.68 mmol, 1.0 equiv.), ethylene glycol (2.75 mL, 49.2 mmol, 2.5 equiv.), and a catalytic amount of *p*-toluenesulfonic acid (19 mg, 0.1 mmol, 0.005 equiv.) were dissolved in 28 mL of toluene in a 100 mL roundbottom flask equipped with a Dean-Stark apparatus. The reaction mixture was refluxed at 140 °C for 20 h. The reaction mixture was extracted by ethyl ether and followed by washing with saturated $Na_2CO_3$, $H_2O$, and saturated brine solution. The combined organic phase was dried with anhydrous $Na_2SO_4$. After solvent removal under reduced pressure, the residue was further purified by distillation to give the title compound as a yellow liquid (4.07 g, 82 %). (Under reduced pressure (20-50 mbar, 210 °C). $^1$HNMR (300 MHz, $CDCl_3$): δ (ppm) 6.94 (d, *J* = 3.8 Hz, 1H), 6.90 (dd, *J* = 3.8, 0.7 Hz, 1H), 6.02 (d, *J* = 0.7 Hz, 1H), 4.15 - 4.04 (m, 2H), 4.04 - 3.94 (m, 2H).

**4-(3-(5-(1,3-dioxolan-2-yl)thiophen-2-yl)prop-2-yn-1-yl)morpholine - 203**

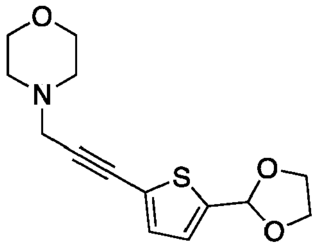

Bromo compound **202** (0.57 g, 2.43 mmol, 1.0 equiv.) and 4-(prop-2-yn-1-yl)morpholine (336 mg, 2.68 mmol, 1.1 eq.) was dissolved in 12 mL of triethylamine and 24 mL of dichloromethane. This mixture was degassed with argon for 5 min, then $Pd(PPh_3)_4$ (141 mg, 0.05 equiv.) and CuI (46 mg, 0.1 equiv.) were added and the mixture was stirred for 18 hours under argon in absence of light at rt. Solvents were removed; the crude was purified by flash chromatography on silica gel (0 to 5% MeOH in dichloromethane over 40 min, 24G, 30 micron, 20 mL/min) to afford an orange oil (442 mg, 65%). *Rf* = 0.36 (DCM/MeOH 95/5, v/v, vanillin). $^1$H NMR (300 MHz, $CDCl_3$): δ (ppm) 7.11 (d, *J* = 3.7 Hz, 1H), 7.04 (dd, *J* = 3.7, 0.7 Hz, 1H), 6.10 (s, 1H), 4.21 - 4.10 (m, 2H), 4.10 - 3.98 (m, 2H), 3.83 - 3.77 (m, 4H), 3.56 (s, 2H), 2.69-2.64 (m, 4H). $^{13}$C NMR (75 MHz, $CDCl_3$): δ (ppm) 143.1, 131.7, 125.9, 123.8, 100.0, 88.7, 78.7, 66.9, 65.3, 52.5, 48.3. HRMS (ESI$^+$): m/z calcd for $[C_{14}H_{18}NO_3S]^+$ 280.1007, found 280.1005.

**4-(4-(5-(1,3-dioxolan-2-yl)thiophen-2-yl)but-3-yn-1-yl)morpholine - 204**

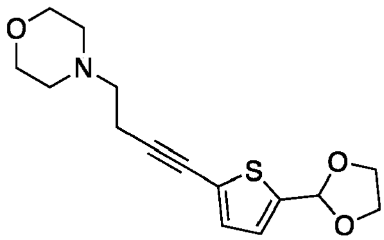

Bromo compound **202** (0.50 g, 2.13 mmol, 1.0 equiv.) and 4-(but-3-yn-1-yl)morpholine (326 mg, 2.34 mmol, 1.1 equiv.) was dissolved in 11 mL of Triethylamine and 21 mL of dichloromethane. This mixture was degassed with argon for 5 min, then $Pd(PPh_3)_4$ (246 mg, 0.213 mmol, 0.1 equiv.) and CuI (41 mg, 0.213 mmol, 0.1 equiv.) were added and the mixture was stirred for 18 hours under argon in absence of light at rt. Solvents were removed; the crude was purified by flash chromatography on silica gel (0 to 5% MeOH in dichloromethane over 40 min, 40G, 30 micron, 30 mL/min) to afford an orange oil (346 mg, 56%). *Rf* = 0.22 (cyclo/EtOAc 6/4, v/v, vanillin). $^1$H NMR (300 MHz, $CDCl_3$), δ (ppm) 7.02 (d, *J* = 3.7 Hz, 1H), 7.00 (d, *J* = 3.8 Hz, 1H), 6.07 (s, 1H), 4.17 - 4.08 (m, 2H), 4.08 - 4.00 (m, 2H), 3.75 (td, *J* = 4.7, 1.8 Hz, 5H), 2.73 - 2.57 (m, 5H), 2.57 - 2.49 (m, 5H). HRMS (ESI$^+$): m/z calcd for $[C_{15}H_{20}NO_3S]^+$ 294.1164, found 294.1161.

**5-(5-(1,3-dioxolan-2-yl)thiophen-2-yl)pent-4-yn-1-ol - 205**

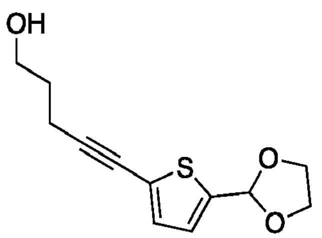

Bromo compound **202** (1 g, 4.25 mmol, 1.0 equiv.) and 4-pentynol (0.44 mL, 4.67 mmol 1.1 equiv.) was dissolved in 21 mL of triethylamine and 42 mL of dichloromethane (0.07 M). This mixture was degassed with argon for 5 min, then $Pd(PPh_3)_4$ (246 mg, 0.21 mmol, 0.05 equiv.) and CuI (81 mg, 0.43 mmol, 0.1 equiv.) were added and the mixture was

stirred overnight under argon, and in absence of light. Solvents were removed; the crude was purified by flash chromatography on silica gel (10 to 50% EtOAc in cyclohexane over 45 min, 40G, 30 micron, 30 mL/min) to afford the alcohol (0.88 g, 87%) as an orange oil. *Rf* = 0.16 (cyclo/EtOAc 7/3, v/v, vanillin). $^1$HNMR (300 MHz, $CDCl_3$): δ (ppm) 6.98 (d, *J* = 3.7 Hz, 1H), 6.96 (dd, *J* = 3.7, 0.6 Hz, 1H), 6.03 (d, *J* = 0.5 Hz, 1H), 4.15 - 4.04 (m, 2H), 4.04 - 3.93 (m, 2H), 3.77 (t, *J* = 6.2 Hz, 2H), 2.53 (t, *J* = 7.0 Hz, 2H), 1. 83 (tt, *J* = 7.0, 6.1 Hz, 2H), 1.72 (s, 1H). $^{13}$C NMR (75 MHz, $CDCl_3$): δ (ppm) 142.2, 130.8, 125.8, 124.9, 100.0, 94.1, 74.2, 65.2, 61.6, 31.2, 16.2. HRMS (ESI$^+$): m/z calcd for $[C_{12}H_{15}O_3S]^+$ 239.0742, found 239.0742.

**4-(3-(5-(1,3-dioxolan-2-yl)thiophen-2-yl)propyl)morpholine - 206**

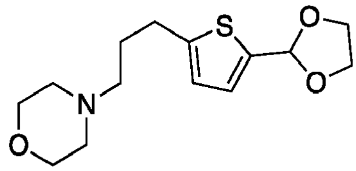

Alkyne **203** (440 mg, 1.57 mmol, 1.0 equiv.) was hydrogenated with Pd on charcoal 10% (335 mg, 0.31 mmol, 0.2 equiv.) in MeOH/EtOAc (10:1, 15.7 mL, 0.1 M) for 4 h at rt then filtered on celite®, rinsed with MeOH and concentrated. The crude was purified by flash chromatography on silica gel (0 to 5% MeOH in dichloromethane over 30 min, 24G, 30 micron, 20 mL/min) to afford the product as a yellowish solid (238 mg, 53%). R*f* = 0.39 (Dichloromethane/MeOH 95/5, v/v, vanillin). $^1$H NMR (300 MHz, $CDCl_3$): δ (ppm) 7.02 (d, *J* = 3.5 Hz, 1H), 6.74 - 6.67 (m, 1H), 6.07 (s, 1H), 4.26 - 4.11 (m, 2H), 4.10 (s, 2H), 3.81 - 3.71 (m, 5H), 2.92 - 2.83 (m, 2H), 2.52 - 2.45 (m, 4H), 2.45 - 2.39 (m, 2H), 1.89 (p, *J* = 7.5 Hz, 3H). $^{13}$C NMR (75 MHz, $CDCl_3$): δ (ppm) 146.5, 138.9, 126.3, 123.8, 100.5, 67.0, 65.2, 57.9, 53.7, 28.4, 27.9. HRMS (ESI$^+$): m/z calcd for $[C_{14}H_{22}NO_3S]^+$ 284.1312, found 284.1320.

**4-(4-(5-(1,3-dioxolan-2-yl)thiophen-2-yl)butyl)morpholine - 207**

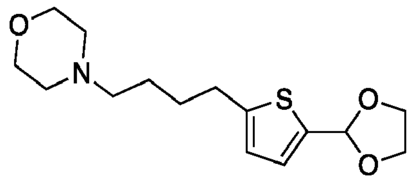

Alkyne **204** (350 mg, 1.18 mmol, 1.0 equiv.) was hydrogenated with Pd on charcoal 10% (251 mg, 0.25 mmol, 0.2 equiv.) in MeOH (11.7 mL, 0.1 M) for 2 h at rt. Filtered on celite® and concentrated. The crude was purified by flash chromatography on silica gel (0 to 5% MeOH in DCM over 40 min, 40G, 15 micron, 30 mL/min). Isolated as a yellowish solid (165 mg, 47%). R*f* = 0.39 (DCM/MeOH 95/5, v/v, vanillin). $^1$H NMR (300 MHz, $CDCl_3$): δ (ppm) 6.93 (d, *J* = 3.5 Hz, 1H), 6.62 (d, *J* = 3.5 Hz, 1H), 5.98 (s, 1H), 4.15 - 4.02 (m, 2H), 4.02 - 3.89 (m, 2H), 3.67 (t, *J* = 4.7 Hz, 4H), 2.78 (t, *J* = 7.4 Hz, 2H), 2.38 (t, *J* = 4.6 Hz, 4H), 2.31 (t, *J* = 7.5 Hz, 2H), 1.66 (p, *J* = 7.2 Hz, 2H), 1.51 (tt, *J* = 9.6, 5.5 Hz, 2H). $^{13}$C NMR (75 MHz, $CDCl_3$): δ (ppm) 146.7, 138.8, 126.2, 123.7, 100.4, 66.9, 65.1, 58.7, 53.7, 30.1, 29.4, 25.9. m.p. = 43-45 °C. HRMS (ESI$^+$): m/z calcd for $[C_{15}H_{24}NO_3S]^+$298.1477, found 298.1486.

**5-(5-(1,3-dioxolan-2-yl)thiophen-2-yl)pentan-1-ol -208**

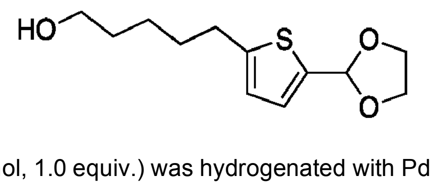

Alkyne **205** (910 mg, 3.81 mmol, 1.0 equiv.) was hydrogenated with Pd on charcoal 10% (227 mg, 25% wt.) in

MeOH (38 mL, 0.1 M). After 2 hours, the reaction mixture was filtered and concentrated. The crude was purified by flash chromatography on silica gel (10 to 50% AcOEt in cyclohexane over 45 min, 40G, 30 micron) to afford the product as an oil (822 mg, 89%). *Rf* = 0.54 (cyclo/EtOAc 1/1, v/v, vanillin). $^{1}H$ NMR (300 MHz, $CDCl_3$): δ (ppm) 7.01 (d, *J* = 3.4 Hz, 1H), 6.70 (dt, *J* = 3.5, 1.0 Hz, 1H), 6.07 (s, 1H), 4.22 - 4.10 (m, 2H), 4.10 - 4.00 (m, 2H), 3.67 (t, *J* = 6.5 Hz, 2H), 2.85 (td, *J* = 7.4, 0.9 Hz, 2H), 1.81 - 1.67 (m, 2H), 1.67 - 1.57 (m, 2H), 1.53 - 1.40 (m, 2H). $^{13}C$ NMR (75 MHz, $CDCl_3$): δ (ppm) 147.0, 138.7, 126.3, 123.7, 100.5, 65.2, 62.8, 32.5, 31.4, 30.2, 25.2. HRMS (ESI$^+$): m/z calcd for $[C_{12}H_{19}O_3S]^+$ 243.1055, found 243.1047.

**5-(5-morpholinopentyl)thiophene-2-carbaldehyde 209**

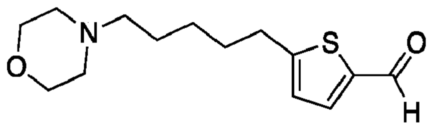

A solution of alcohol **208** (0.56 g, 2.3 mmol, 1.00 equiv.), and triethylamine (1.6 mL, 11.6 mmol, 5.0 equiv.) in dichloromethane (11 mL, 0.2 M) at 0°C. was treated dropwise with methanesulfonyl chloride (0.31 mL, 3.9 mmol, 1.7 equiv.), followed by stirring for 1 hour at this temperature. dichloromethane was added (10 mL), the solution was washed with water (10 mL) and brine (10 mL). Organic layer was dried over $MgSO_4$ and evaporated under reduced pressure. The crude mesylate was directly used in following step without further purification. The crude mesylate was dissolved in THF (11 mL) before morpholine (2.0 mL, 23 mmol, 10 equiv.) was added and the reaction mixture was refluxed overnight. Filtered, concentrated, and purified by flash chromatography (100% dichloromethane to 98% dichloromethane 2% $NH_3$ 7N in MeOH, 24 G, 15 micro, 20 mL/min) to afford the thiophene (410 mg, 57%). *Rf* = 0.42 (DCM/MeOH 7N $NH_3$ 99/1, v/v, vanillin). $^{1}H$ NMR (300 MHz, $CDCl_3$): δ (ppm) 7.01 (d, *J* = 3.5 Hz, 1H), 6.69 (dt, *J* = 3.5, 1.0 Hz, 1H), 6.06 (s, 1H), 4.22 - 4.10 (m, 2H), 4.10 - 3.98 (m, 2H), 3.80 - 3.70 (m, 4H), 2.83 (t, *J* = 7.5 Hz, 2H), 2.52 - 2.39 (m, 4H), 2.41 - 2.30 (m, 2H), 1.81 - 1.65 (m, 2H), 1.64 - 1.46 (m, 2H), 1.48 - 1.35 (m, 2H). $^{13}C$ NMR (75 MHz, $CDCl_3$): δ (ppm) 147.0, 138.7, 126.2, 123.6, 100.5, 67.0, 65.2, 59.0, 53.8, 31.5, 30.2, 26.9, 26.3. HRMS (ESI$^+$): m/z calcd for $[C_{16}H_{26}NO_3S]^+$312.1633, found 312.1640.

The acetal (410 mg, 1.32 mmol, 1.0 equiv.) was stirred for 18 h at rt in a mixture of formic acid and water (1:1, 13 mL, 0.1 M), The reaction mixture was quenched by solid $NaHCO_3$ until neutral and extracted with EtOAc (3 x 50 mL). Dried over $MgSO_4$ and concentrated. Completion monitored by crude NMR. Purified by flash chromatography (0 to 2% MeOH/$NH_3$ in dichloromethane over 45 min, 24G, 20 mL/min) to afford an orange oil (290 mg, 82%). *Rf* = 0.41 (dichloromethane/MeOH $NH_3$ 7M, 98/2, v/v). $^{1}H$ NMR (300 MHz, $CDCl_3$): δ (ppm) 9.81 (s, 1H), 7.60 (d, *J* = 3.8 Hz, 1H), 6.89 (dt, *J* = 3.8, 0.9 Hz, 1H), 3.76 - 3.66 (m, 4H), 2.93 - 2.82 (m, 2H), 2.41 (dd, *J* = 5.7, 3.7 Hz, 4H), 2.35 - 2.28 (m, 2H), 1.80 - 1.65 (m, 2H), 1.53 (dddd, *J* = 14.1, 9.5, 6.5, 3.1 Hz, 2H), 1.45 - 1.33 (m, 2H). $^{13}C$ NMR (75 MHz, $CDCl_3$): δ (ppm) 182.6, 157.4, 141.7, 137.0, 125.9, 67.0, 58.9, 53.8, 31.2, 30.8, 26.9, 26.2. HRMS (ESI$^+$): m/z calcd for $[C_{14}H_{22}NO_2S]^+$268.1371, found 268.1374.

**5-(5-(6,7-dimethoxy-3,4-dihydroisoquinolin-2(1*H*)-yl)pentyl)thiophene-2-carbaldehyde - 210**

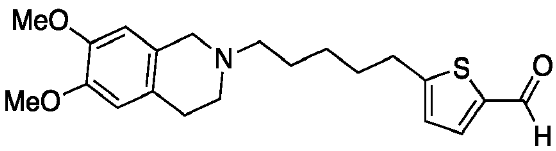

A solution of alcohol **208** (0.79 g, 3.3 mmol, 1.0 equiv.), and triethylamine (2.2 mL, 16.3 mmol, 5.0 equiv.) in dichloromethane (32 mL, 0.1 M) at 0 °C was treated dropwise with methanesulfonyl chloride (0.43 mL, 5.5 mmol, 1.7 equiv.), followed by stirring for 1 hour at this temperature. Dichloromethane was added (10 mL), the solution was washed with $H_2O$ (10 mL) and brine (10 mL). Organic layer was dried over $MgSO_4$ and evaporated under reduced pressure. The crude mesylate was directly used in following step without further purification. Mesylate was dissolved in dry MeCN (16 mL, 0.2 M) under Argon atmosphere and quinoline.HCl (1.5 g, 6.5 mmol, 2.0 equiv.) followed by potassium carbonate (1.8 g, 13.1 mmol, 4.0 equiv.) were added in this order. The resulting heterogenous mixture was reflux overnight. Salt

were removed by filtration, rinsed and solvent was evaporated under reduced pressure. Purified by flash chromatography (0 to 5% MeOH in DCM over 40 min, 24G Interchim, 20 mL/min) to afford the acetal as an orange oil (1.028 g, 78%). $^1$H NMR (300 MHz, $CDCl_3$): δ (ppm) 6.96 (dd, *J* = 3.5, 0.6 Hz, 1H), 6.65 (dt, *J* = 3.5, 0.9 Hz, 1H), 6.58 (s, 1H), 6.52 (s, 1H), 6.02 (d, *J* = 0.5 Hz, 1H), 4.16 - 4.09 (m, 2H), 4.02 - 3.96 (m, 2H), 3.83 (d, *J* = 1.6 Hz, 8H), 3.53 (s, 3H), 2.87 - 2.76 (m, 5H), 2.69 (t, *J* = 5.6 Hz, 3H), 2.53 - 2.42 (m, 2H), 1.77 - 1.65 (m, 3H), 1.65 - 1.54 (m, 3H), 1.48 - 1.38 (m, 2H). $^{13}$C NMR (75 MHz, $CDCl_3$): δ (ppm) 147.5, 147.2, 147.1, 138.7, 126.8, 126.3, 126.2, 123.6, 111.4, 109.6, 100.5, 65.2, 58.3, 56.0, 55.9, 55.9, 51.1, 31.6, 30.2, 28.7, 27.0. HRMS ($ESI^+$): m/z calcd for $[C_{23}H_{32}NO_4S]^+$418.2052, found 418.2047.

The acetal (1.028 g, 2.46 mmol, 1.0 equiv.) was stirred for 18 h at rt in a mixture of formic acid and water (1:1, 33 mL, 0.1 M). Quenched by solid $NaHCO_3$ until neutral and extracted with AcOEt (3 x 150 mL). Dried over $MgSO_4$ and concentrated. Completion monitored by crude NMR. Purified by flash chromatography (0 to 5% MeOH in DCM over 45 min, 24G Interchim, 30 micro, 20 mL/min) to afford an orange oil (832 mg, 90%). *Rf* = 0.64 (DCM/MeOH, 95/5, v/v). $^1$H NMR (300 MHz, $CDCl_3$): δ (ppm) 9.80 (s, 1H), 7.59 (d, *J* = 3.8 Hz, 1H), 6.90 (dt, *J* = 3.8, 0.9 Hz, 1H), 6.58 (s, 1H), 6.51 (s, 1H), 3.83 (s, 3H), 3.82 (s, 3H), 3.56 - 3.48 (m, 2H), 2.92 - 2.85 (m, 2H), 2.81 (t, *J* = 5.8 Hz, 2H), 2.68 (t, *J* = 6.0 Hz, 2H), 2.53 - 2.44 (m, 2H), 1.76 (tt, *J* = 8.4, 7.3 Hz, 2H), 1.69 - 1.56 (m, 2H), 1.44 (tdd, *J* = 10.2, 7.3, 4.1 Hz, 2H). $^{13}$C NMR (75 MHz, $CDCl_3$): δ (ppm) 182.6, 157.5, 147.5, 147.2, 141.7, 137.0, 126.7, 126.2, 125.9, 111.4, 109.5, 58.1, 56.0, 55.9, 55.9, 51.1, 31.2, 30.8, 28.7, 27.0. HRMS ($ESI^+$): m/z calcd for $[C_{21}H_{28}NO_3S]^+$ 374.1790 found 374.1799.

**5-(3-morpholinopropyl)thiophene-2-carbaldehyde oxime** - **211**

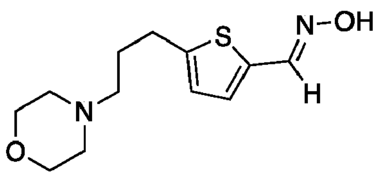

Acetal 206 (226 mg, 0.80 mmol, 1.0 equiv.) was heated for 18 hours at 60 °C in a mixture of formic acid/water (1:1, 16 mL, 0.05 M). The resulting solution was concentrated and quenched by saturated aqueous solution of $NaHCO_3$ until neutral and extracted with EtOAc (3 x 50 mL). Dried over $MgSO_4$ and concentrated. Purified by flash chromatography (0 to 5% MeOH in DCM over 20 min, 12G, 30 micron, 20 mL/min) to afford the aldehyde (145 mg, 79%). *Rf* = 0.48 (DCM/MeOH 95/5, v/v, ninhydrin). $^1$H NMR (300 MHz, $CDCl_3$): δ (ppm) 9.85 (s, 1H), 7.65 (d, *J* = 3.8 Hz, 1H), 6.95 (dd, *J* = 3.8, 0.9 Hz, 1H), 3.77 - 3.72 (m, 4H), 3.00 - 2.92 (m, 2H), 2.46 (dd, *J* = 5.6, 3.7 Hz, 4H), 2.42 (dd, *J* = 8.1, 6.4 Hz, 3H), 1.92 (p, *J* = 7.4 Hz, 2H). $^{13}$C NMR (75 MHz, $CDCl_3$): δ (ppm) 182.7, 156.8, 141.8, 137.1, 126.1, 67.0, 57.5, 53.7, 28.4, 28.0. HRMS ($ESI^+$): m/z calcd for $[C_{12}H_{18}NO_2S]^+$ 240.1058, found 240.1052.

$NaHCO_3$ (155 mg, 1.84 mmol, 3.0 equiv.) and $NH_2OH.HCl$ (107 mg, 1.53 mmol, 2.5 equiv.) added to a mixture of aldehyde (147 mg, 0.61 mmol, 1.0 equiv.) in MeOH (0.1M, 6 mL) and stirred for 24 hours. Filtered through celite®, concentrated and purified by flash chromatography (DCM/MeOH 0 to 10% over 30 min, 24G, 30 micro, 20 mL/min) to afford the oxime (152 mg, 97%). 1M MsOH (961 mg of MsOH in 10 mL of AcOEt) was added to form the mesylate salt. $^1$H NMR (300 MHz, $CDCl_3$): δ (ppm) 8.22 (s, 1H), 7.66 (d, *J* = 4.1 Hz, 0H), 7.19 (d, *J* = 3.7 Hz, OH), 6.98 (d, *J* = 3.6 Hz, 1H), 6.82 (d, *J* = 3.7 Hz, OH), 6.74 (d, *J* = 3.5 Hz, 1H), 3.83 (q, *J* = 4.7 Hz, 4H), 2.91 (dt, *J* = 19.1, 7.7 Hz, 2H), 2.69 - 2.54 (m, 4H), 2.54 - 2.40 (m, 2H), 2.03 (ddt, *J* = 15.4, 10.4, 7.8 Hz, 2H). $^{13}$C NMR (75 MHz, $CDCl_3$): δ (ppm) 150.5, 146.9, 144.3, 134.5, 130.9, 128.9, 124.6, 123.7, 66.5, 66.4, 58.4, 58.2, 53.6, 53.6, 28.3, 28.1, 28.0, 27.9. HRMS ($ESI^+$): m/z calcd for $[C_{12}H_{19}N_2O_2S]^+$ 255.1167, found 255.1167. HPLC (Column Syncronis Aq, 100*2.1 mm, 1.7 μm, 0.1% formic Acid $H_2O$/MeCN, 5 to 100% over 18 min, 0.6 mL/min) : $t_R$ = 1.83 min (Cis), 2.00 min (Trans), purity = 99.1%.

**5-(4-morpholinobutyl)thiophene-2-carbaldehyde oxime - 212**

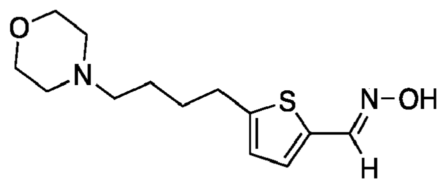

Acetal **207** (157 mg, 0.53 mmol, 1.0 equiv.) was heated for 18 hours at 60 °C in a mixture of formic acid and water (1:1, 0.05 M, 10.5 mL). The resulting solution was concentrated and quenched by saturated aqueous solution of $NaHCO_3$ until neutral and extracted with EtOAc (4 x 100 mL). Dried over $MgSO_4$ and concentrated to give the crude material (130 mg, 97%) that was used without further purification in the next step .
$^1$H NMR (300 MHz, $CDCl_3$): δ (ppm) 9.80 (s, 1H), 7.61 (d, *J* = 3.8 Hz, 1H), 6.91 (dt, *J* = 3.8, 0.9 Hz, 1H), 3.76 - 3.62 (m, 4H), 2.98 - 2.81 (m, 2H), 2.42 (dd, *J* = 5.7, 3.6 Hz, 4H), 2.39 - 2.30 (m, 2H), 1.82 - 1.65 (m, 2H), 1.64 - 1.48 (m, 2H).
NaOAc (126 mg, 1.534 mmol, 3.0 equiv.) and $NH_2OH$.HCl (89 mg, 1.28 mmol, 2.5 equiv.) added to a mixture of aldehyde (130 mg, 0.51 mmol, 1.0 equiv.) in MeOH (5 mL) and stirred for 6 hours. The crude mixture was concentrated and purified by reversed phase (0 to 30 % MeCN in $H_2O$ 0.2% AcOH over 30 min, puriflash C18, 30 x 250 mm, C18AQ-5 micro) as an acetate salt. This was treated with $NaHCO_3$ and extracted with AcOEt, concentrated and isolated as a free amine (108.1 mg, 78%). The free amine was treated with 1.0 equivalent of $MeSO_3H$ (1.0 M in AcOEt), evaporated slowly and isolated as mesylate salt (144.4 mg, 77%) that was dried on heated vacuum and high vacuum. Mixture of two isomers : $^1$H NMR (300 MHz, MeOD): δ (ppm) 8.17 (s, OH), 7.65 (s, 1H), 7.30 (d, *J* = 3.8 Hz, 1H), 7.05 (d, *J* = 3.6 Hz, OH), 6.95 - 6.86 (m, 1H), 6.86 - 6.78 (m, OH), 4.87 (s, 4H), 4.05 (dd, *J* = 13.2, 3.7 Hz, 2H), 3.78 (ddd, *J* = 13.5, 11.9, 2.2 Hz, 2H), 3.57 - 3.42 (m, 2H), 3.25 - 3.15 (m, 2H), 3.10 (dd, *J* = 12.3, 3.7 Hz, 2H), 2.98 (d, *J* = 6.8 Hz, 1H), 2.94 - 2.85 (m, 1H), 2.72 (s, 3H), 1.82 (tt, *J* = 6.0, 3.2 Hz, 4H). $^{13}$C NMR (75 MHz, MeOD): δ (ppm) 150.3, 145.9, 143.8, 140.4, 134.6, 131.6, 129.5, 128.9, 124.8, 124.0, 63.6, 56.8, 51.8, 38.2, 28.9, 28.7, 28.0, 27.9, 22.6, 22.6. HRMS (ESI$^+$): m/z calcd for $[C_{13}H_{21}N_2O_2S]^+$ 269.1324, found 269.1313. HPLC (Column Syncronis Aq, 100*3 mm, 3 μm, $H_2O$ formic acid 0.2%/MeCN, 0 to 100% over 20 min, 0.5 mL/min) : $t_R$ = 11.02 min (Cis), 11.33 min (Trans), purity = 99.1%.

**(E)-5-(5-morpholinopentyl)thiophene-2-carbaldehyde oxime** - **213**

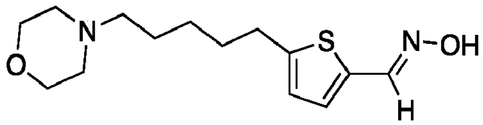

NaOAc (113 mg, 1.38 mmol, 3.0 equiv.) and $NH_2OH$.HCl (80 mg, 1.15 mmol, 2.5 equiv.) added to a mixture of aldehyde (123 mg, 0.46 mmol, 1.0 equiv.) in MeOH (4.6 mL, 0.1 M) and stirred for 4 hours. The resulting mixture was concentrated with celite® and purified by flash chromatography (0 to 10 MeOH in DCM, over 40 min, 20 mL/min, 12G, 15 micro) to give the desired compound as an acetate salt (trans isomer: 46 mg, 36%, cis isomer: 53 mg, 41%).
F1 (Trans isomer, 0.69 AcOH): $^1$H NMR (300 MHz, $CDCl_3$ + MeOD-d6): δ (ppm) 8.09 (d, *J* = 0.9 Hz, 1H), 6.89 (d, *J* = 3.4 Hz, 1H), 6.61 (dd, *J* = 3.5, 1.0 Hz, 1H), 3.71 (t, *J* = 4.7 Hz, 4H), 2.71 (t, *J* = 7.6 Hz, 2H), 2.55 (t, *J* = 4.7 Hz, 4H), 2.45 - 2.33 (m, 2H), 1.95 (d, *J* = 1.0 Hz, 2H), 1.61 (p, *J* = 7.4 Hz, 2H), 1.51 (td, *J* = 11.4, 9.8, 6.1 Hz, 2H), 1.30 (q, *J* = 8.1 Hz, 2H). $^{13}$C NMR (75 MHz, $CDCl_3$ + MeOD-d6): δ (ppm) 176.0, 147.5, 144.5, 133.6, 129.2, 124.4, 65.9, 58.5, 53.0, 31.1, 30.0, 26.6, 25.1, 21.8 (AcOH). HRMS (ESI$^+$): m/z calcd for $[C_{14}H_{23}N_2O_2S]^+$283.1480, found 283.1491. HPLC (Column Syncronis Aq, 100*3 mm, 3 μm, Ammonium Acetate 50 mM/MeCN, 0 to 100% over 20 min, 0.5 mL/min) : $t_R$ = 14.91 min (Cis), 15.43 min (Trans), purity = 100.0%.
F2 (Cis isomer, 0.46 AcOH) : $^1$H NMR (300 MHz, $CDCl_3$ + MeOD-d6): δ (ppm) 7.50 (s, 1H), 7.11 (d, *J* = 3.7 Hz, 1H), 6.69 (dd, *J* = 3.8, 0.9 Hz, 1H), 3.70 (t, *J* = 4.7 Hz, 4H), 2.76 (t, *J* = 7.5 Hz, 2H), 2.50 (t, *J* = 4.7 Hz, 4H), 2.42 - 2.29 (m, 2H), 1.96 (s, 1H), 1.65 (p, *J* = 7.6 Hz, 2H), 1.50 (tt, *J* = 7.8, 6.2 Hz, 2H), 1.31 (qd, *J* = 7.6, 6.6, 3.7 Hz, 2H). $^{13}$C NMR (75 MHz, $CDCl_3$ + MeOD-d6): δ (ppm) 175.8, 151.5, 140.9, 131.4, 129.1, 123.6, 66.2, 58.6, 53.2, 31.2, 29.9, 26.8, 25.4, 21.8. HRMS (ESI$^+$): m/z calcd for $[C_{14}H_{23}N_2O_2S]^+$ 283.1480, found 283.1483. HPLC (Column Syncronis Aq, 100*3 mm, 3 mm, Ammonium Acetate 50 mM/MeCN, 0 to 100% over 20 min, 0.5 mL/min) : $t_R$ = 15.06 min (Cis), 15.42 min (Trans), purity = 99.1%.

**(*E*)-5-(5-(6,7-dimethoxy-3,4-dihydroisoquinolin-2(1H)-yl)pentyl)thiophene-2-carbaldehyde oxime - 214**

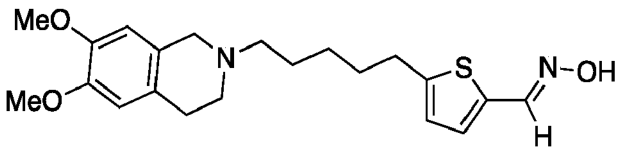

NaOAc (86 mg, 1.04 mmol, 3.0 equiv.) and$NH_2OH.HCl$ (60 mg, 0.887 mmol, 2.5 equiv.) added to a mixture of aldehyde (130 mg, 0.35 mmol, 1.0 equiv.) in MeOH (3.5 mL, 0.1 M) and stirred for 4 hours. The crude mixture was concentrated with celite® and purified by flash chromatography (0 to 10 % MeOH in DCM, over 40 min, 20 mL/min, 24G, 15 micro) to give the desired product as an acetate salt (trans isomer: 72 mg, 53%, cis isomer: 52 mg, 38%).

F1 (Trans isomer) : $^1H$ NMR (300 MHz, $CDCl_3$ + MeOD): δ (ppm) 8.06 (s, 1H), 6.84 (d, *J* = 3.6 Hz, 1H), 6.61 - 6.57 (m, 1H), 6.52 (s, 1H), 6.45 (s, 1H), 3.82 (s, 2H), 3.75 (s, 3H), 3.73 (s, 3H), 2.99 (t, *J* = 6.0 Hz, 2H), 2.91 - 2.82 (m, 2H), 2.69 (dq, *J* = 7.6, 4.7, 4.1 Hz, 4H), 1.76 - 1.65 (m, 2H), 1.65 - 1.55 (m, 2H), 1.33 (h, *J* = 7.4, 6.5 Hz, 2H). $^{13}C$ NMR (75 MHz, $CDCl_3$ + MeOD): δ (ppm) 148.1, 147.7, 147.2, 144.1, 134.1, 129.0, 124.5, 124.4, 122.9, 111.2, 109.5, 56.0, 55.91, 55.89, 53.6, 49.5, 31.1, 30.1, 26.7, 26.0, 25.2. HRMS ($ESI^+$): m/z calcd for $[C_{21}H_{29}N_2O_3S]^+$ 389.1899, found 389.1902. HPLC (Column Syncronis Aq, 100*3 mm, 3 μm, Ammonium Acetate 50 mM/MeCN, 0 to 100% over 20 min, 0.5 mL/min) : $t_R$ = 17.00 min (Cis), 17.48 min (Trans), purity = 96.8%.

F2 (Cis isomer) : $^1H$ NMR (300 MHz, $CDCl_3$ + MeOD): δ (ppm) 7.49 (s, 1H), 7.10 (d, *J* = 3.6 Hz, 1H), 6.68 (d, *J* = 3.7 Hz, 1H), 6.53 (s, 1H), 6.46 (s, 1H), 3.77 (s, 2H), 3.76 (s, 3H), 3.75 (s, 3H), 2.96 (t, *J* = 6.0 Hz, 2H), 2.84 (t, *J* = 6.1 Hz, 2H), 2.76 (t, *J* = 7.5 Hz, 2H), 2.71 - 2.61 (m, 2H), 1.71-1.56 (m, 4H), 1.40 - 1.23 (m, 2H). $^{13}C$ NMR (75 MHz, $CDCl_3$ + MeOD): δ (ppm) 151.4, 148.1, 147.7, 140.8, 131.4, 129.1, 124.6, 123.7, 123.1, 111.2, 109.4, 56.1, 55.9, 55.8, 53.7, 49.6, 31.1, 29.8, 26.7, 26.1, 25.3. HRMS ($ESI^+$): m/z calcd for $[C_{21}H_{29}N_2O_3S]^+$ 389.1899, found 389.1905. HPLC (Column Syncronis Aq, 100*3 mm, 3 μm, Ammonium Acetate 50 mM/MeCN, 0 to 100% over 20 min, 0.5 mL/min) : $t_R$ = 17.08 min (Cis), 17.48 min (Trans), purity = 98.8%.

**Ethyl 5-bromo-1,3,4-thiadiazole-2-carboxylate - 215**

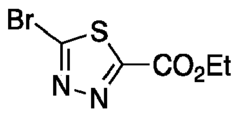

To a stirred solution of ethyl 5-amino-1,3,4-thiadiazole-2- carboxylate (3.66 g, 21.1 mmol, 1.0 equiv.) in acetonitrile (70 mL, 0.6 M) at room temperature was added copper (II) bromide (9.4 g, 42.3 mmol, 2.0 equiv.) and the mixture was stirred for 20 min. Tertiary butyl nitrite (4.3 g, 42.3 mmol, 2.0 equiv.) was then added drop wise for 10 min, and the reaction mixture was heated to 60 °C for 30 min. The reaction mixture was concentrated under reduced pressure, diluted with water (300 mL), and then extracted with ethyl acetate (500 m:). The organic layer was separated and dried over anhydrous sodium sulphate, and evaporated to afford ethyl 5-bromo-1,3,4-thiadiazole-2-carboxylate as a light-yellow solid (2.43 g, 49%).
$^1H$ NMR (300 MHz, $CDCl_3$): δ (ppm) 4.57 (q, *J* = 7.1 Hz, 1H), 1.50 (t, *J* = 7.1 Hz, 2H).

**Ethyl 5-(4-morpholinobut-1-yn-1-yl)-1,3,4-thiadiazole-2-carboxylate** - **216**

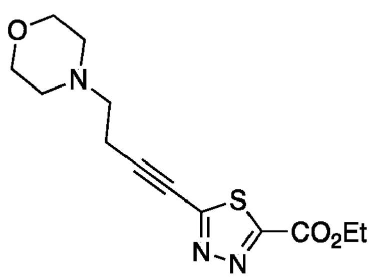

Bromo compound **215** (0.5 g, 2.11 mmol, 1.0 equiv.) and 4-(but-3-yn-1-yl)morpholine (323 mg, 2.32 mmol, 1.1 equiv.) was dissolved in 11 mL of triethylamine and 21 mL of dichloromethane. This mixture was degassed with argon for 5 min, then $Pd(PPh_3)_4$ (244 mg, 0.21 mmol, 0.1 equiv.) and CuI (40 mg, 0.21 mmol, 0.1 equiv.) were added and the mixture was stirred for 18 hours under argon in absence of light at rt. Solvents were removed; the crude was purified by flash chromatography on silica gel (dichloromethane/MeOH 0 to 5% over 45 min, 24G, 30 micron, SIHP, 30 mL/min) to afford the title product as a black oil (381 mg, 61%). R*f* = 0.13 (cyclo/EtOAc 6/4, v/v). $^1H$ NMR (300 MHz, $CDCl_3$): δ (ppm) 4.45 (q, *J* = 7.2 Hz, 2H), 3.71 - 3.58 (m, 4H), 2.65 (d, *J* = 1.0 Hz, 4H), 2.49 - 2.35 (m, 4H), 1.39 (t, *J* = 7.1 Hz, 3H).

$^{13}C$ NMR (75 MHz, $CDCl_3$): δ (ppm) 159.6, 158.2, 154.3, 102.2, 70.8, 66.9, 63.5, 56.2, 53.3, 18.1, 14.2. HRMS ($ESI^+$): m/z calcd for $[C_{13}H_{18}N_3O_3S]^+$ 296.1069, found 296.1068.

**Ethyl 5-(4-morpholinobutyl)-1,3,4-thiadiazole-2-carboxylate - 217**

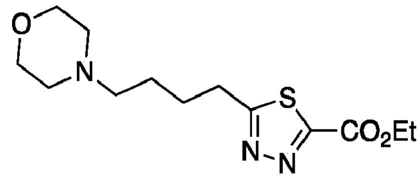

Alkyne **216** (1.27 g, 4.28 mmol, 1.0 equiv.) was dissolved in EtOH (42 mL, 0.1 M) with 10% Pd on charcoal (456 mg, 0.43 mmol, 0.1 equiv.) under hydrogen for 4 h at rt. The crude mixture was filtered through celite®, concentrated and purified by flash chromatography on silica gel (0 to 5% MeOH in DCM over 40 min, 40G, 15 micron, 30 mL/min) to give the desired compound as a Black oil (850 mg, 66%). $^1H$ NMR (300 MHz, $CDCl_3$): δ (ppm) 4.43 (q, *J* = 7.1 Hz, 2H), 3.62 (t, *J* = 4.7 Hz, 4H), 3.14 (t, *J* = 7.6 Hz, 2H), 2.39 - 2.25 (m, 6H), 1.81 (p, *J* = 7.6 Hz, 2H), 1.60 - 1.47 (m, 2H), 1.38 (t, *J* = 7.1 Hz, 3H). $^{13}C$ NMR (75 MHz, $CDCl_3$): δ (ppm) 175.0, 160.2, 158.7, 66.8, 63.1, 58.1, 53.6, 30.1, 27.8, 25.7, 14.1. HRMS ($ESI^+$): m/z calcd for $[C_{13}H_{22}N_3O_3S]^+$ 300.1382, found 300.1388.

**5-(4-morpholinobutyl)-1,3,4-thiadiazole-2-carbaldehyde** - **218**

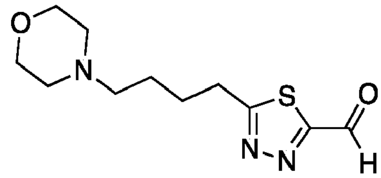

Methyl ester **217** (0.71 g, 2.37 mmol, 1.0 equiv.) was dissolved in dry DCM (15 mL, 0.15 M), the solution was cooled down to -78 °C, DIBAL-H [1 M in DCM] (4.74 mL, 2.60 mmol, 1.1 equiv.) was slowly added. This mixture was stirred at -78 °C for 45 min. Extra 0.9 equiv. of DIBAL-H added and stirred for 15 min. MeOH (10 mL) was slowly added, then the mixture was allowed to warm to rt. The solvent was evaporated and the residue dissolved by dichloromethane (100 mL), washed with saturated solution of NaCl, filtered through celite® and extrated twice by DCM. Combined organic layers were dried over $MgSO_4$ and evaporated under reduced pressure to afford the aldehyde (769 mg, 92%). The crude material was used for the next step without purification. $^1H$ NMR (300 MHz, $CDCl_3$): δ (ppm) 10.23 (s, 1H), 3.83 - 3.62 (m, 4H), 3.28 (t, *J* = 7.6 Hz, 2H), 2.52 - 2.44 (m, 4H), 2.42 (d, *J* = 7.4 Hz, 2H), 2.02 - 1.88 (m, 2H), 1.74 - 1.59 (m, 2H). $^{13}C$ NMR (75 MHz, $CDCl_3$): δ (ppm) 183.4, 175.5, 167.6, 67.0, 58.2, 53.7, 27.8, 25.8. HRMS ($ESI^+$): m/z calcd for $[C_{11}H_{18}N_3O_2S]^+$ 256.1120, found 256.1120.

**(*E*)-5-(4-morpholinobutyl)-1,3,4-thiadiazole-2-carbaldehyde oxime - 219**

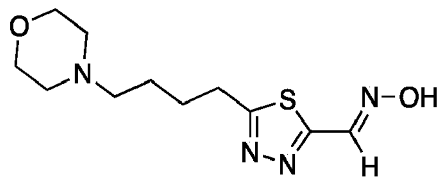

$NH_2OH.HCl$ (406 mg, 5.85 mmol, 2.5 equiv.) and $NaHCO_3$ (589 mg, 7.01 mmol, 3.0 equiv.) added to a mixture of aldehyde **218** (597 mg, 2.34 mmol, 1.0 equiv.) in MeOH (24 mL, 1.2 M) and stirred for 2 hours. Concentrated under reduced pressure, water was added and extracted with DCM (3x20 mL), dried over $MgSO_4$ and finally concentrated.

The crude mixture was purified by flash chromatography (0 to 10% MeOH in DCM over 30 min, 12G, 15microns, 20 mL/min) to afford the title oxime (538 mg, 85%). $^1$H NMR (300 MHz, $CDCl_3$ + MeOH): δ (ppm) 8.34 (s, 0H), 8.02 (s, 1H), 3.71 (t, *J* = 4.7 Hz, 4H), 3.14 (t, *J* = 7.5 Hz, 1H), 3.08 (t, *J* = 7.5 Hz, 1H), 2.48 (t, *J* = 4.7 Hz, 3H), 2.45 - 2.31 (m, 2H), 1.82 (ddt, *J* = 15.1, 10.1, 7.2 Hz, 2H), 1.63 (ddt, *J* = 15.3, 10.1, 5.8 Hz, 2H). $^{13}$C NMR (75 MHz, $CDCl_3$ + MeOH): δ (ppm) 172.5, 170.1, 164.1, 154.8, 141.9, 138.3, 66.3, 58.3, 58.2, 53.4, 29.8, 29.3, 27.7, 27.6, 25.3. HRMS (ESI$^+$): m/z calcd for $[C_{11}H_{19}N_4O_2S]^+$ 271.1229, found 271.1232.

Dissolved in DCM/Methanol, 1 equiv. of MsOH 1M in DCM was added, concentrated and dried in high vacuum. Product crystallized. HPLC (Column Syncronis Aq, 100*3 mm, 3 μm, $H_2O$ formic acid 0.1%/MeCN, 0 to 100% over 20 min, 0.5 mL/min) : $t_R$ = 9.29 min (purity = 95.2%).

**4-(5-(benzyloxy)-6-(1,3-dioxolan-2-yl)pyridin-2-yl)but-3-yn-1-ol - 220**

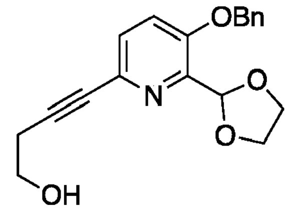

Bromo compound **85** (5 g, 14.87 mmol, 1.0 equiv.) and 3-butyn-1-ol (1.13 mL, 14.97 mmol, 1.0 equiv.) was dissolved in 73 mL of TEA and 148 mL of DCM. This mixture was degassed 15 min, then $Pd(PPh_3)_4$ (0.86 g, 0.74 mmol, 0.05 equiv.) and CuI (283 mg, 1.49 mmol, 0.1 equiv.) were added and the mixture was stirred overnight under argon. Solvents were removed; the crude was purified by flash chromatography on silica gel (Cyclohexane/AcOEt 50:50 to 100% AcOEt over 1 hour, 120G) to afford the title compound (4.53 g, 94%) as an orange oil. R*f* = 0.45 (cyclo/EtOAc 2/8, v/v). $^1$H NMR (300 MHz, $CDCl_3$) δ (ppm) 7.47 - 7.35 (m, 5H, Ph), 7.22 (d, *J* = 8.6 Hz, 1H), 6.29 (s, 1H), 5.16 (s, 2H), 4.29 - 4.18 (m, 2H), 4.13 - 3.99 (m, 2H), 3.86 (t, *J* = 6.3 Hz, 2H), 2.93 (s, 1H), 2.71 (t, *J* = 6.3 Hz, 2H). $^{13}$C NMR (75 MHz, $CDCl_3$): δ (ppm) 152.7, 146.5, 135.7, 134.6, 128.7, 128.36, 128.35, 127.4, 120.2, 100.7, 86.5, 81.5, 70.6, 65.8, 60.8, 23.9. HRMS (ESI$^+$): m/z calcd for $[C_{19}H_{20}NO_4]^+$ 326.1392, found 326.1391.

**4-(5-(benzyloxy)-6-(1,3-dioxolan-2-yl)pyridin-2-yl)butan-1-ol - 221**

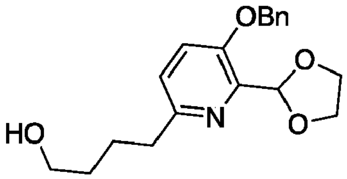

Alkyne **220** (0.95 g, 2.94 mmol) and 2,2'-dipyridyl (181 mg, 1.16 mmol, 0.4 equiv.) was dissolved in MeOH (19 mL, 0.15 M), the solution was degassed with Argon. Pd/C 10% (618 mg, 0.58 mmol, 0.2 equiv.) was added, the mixture was stirred for 8 h at rt under hydrogen at 20 bars. The crude was purified by flash chromatography on silica gel (DCM/MeOH 0 to 7% over 45 min, 40G interchim SIHP, 15 micro, 30 mL/min) to afford a colourless oil (557 mg, 58%). *Rf* = 0.38 (DCM/MeOH 95/5, v/v, vanillin). $^1$H NMR (300 MHz, $CDCl_3$) δ (ppm) 7.51 - 7.32 (m, 5H), 7.22 (d, *J* = 8.5 Hz, 1H), 7.10 (d, *J* = 8.5 Hz, 1H), 6.37 (s, 1H), 5.14 (s, 2H), 4.35 - 4.21 (m, 2H), 4.15 - 4.00 (m, 2H), 3.69 (t, *J* = 6.3 Hz, 2H), 2.85 (t, *J* = 7.4 Hz, 2H), 2.31 (s, 1H), 1.92 - 1.76 (m, 2H), 1.75 - 1.54 (m, 2H). $^{13}$C NMR (75 MHz, $CDCl_3$): δ (ppm) 153.4, 151.6, 145.2, 136.3, 128.6, 128.1, 127.4, 123.7, 121.0, 100.4, 70.7, 65.7, 62.3, 36.3, 32.0, 25.6. HRMS (ESI$^+$): m/z calcd for $[C_{19}H_{24}NO_4]^+$ 330.1705, found 330.1705.

**1-(4-(5-(benzyloxy)-6-(1,3-dioxolan-2-yl)pyridin-2-yl)butyl)-4,4-dimethyl-1,4-azasilinane - 222**

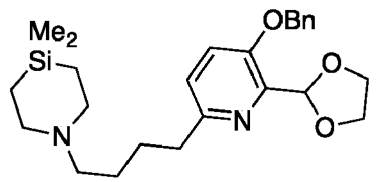

Alcohol **221** (467 mg, 1.42 mmol, 1.0 equiv.) and $Et_3N$ (0.23 mL, 2.84 mmol, 2 equiv.) was dissolved in DCM (5 mL), then MsCl (0.165 mL, 2.12 mmol, 1.5 equiv.) was added at 0 °C. The resulting mixture was stirred for 30 min at rt. After completion, the mixture was quenched with water and extracted with dichloromethane (3 × 20 mL). Dried over $MgSO_4$ and concentrated in vacuo. The obtained crude product was used directly for the next reaction.

4,4-dimethyl-1,4-azasilinane.HCl (470 mg, 2.84 mmol, 2 equiv.) was basified by an aq sat sol of $K_2CO_3$ and extracted with DCM, dried over $MgSO_4$ and concentrated. The crude mesylate was dissolved in MeCN (10 mL) before 4,4-dimethyl-1,4-azasilinane and $K_2CO_3$ (392 mg, 2.84 mmol, 2.0 equiv.) were added and the reaction mixture was heated at 60 ° for 18 hours. The reaction mixture was concentrated and the crude material was purified by flash chromatography on silica gel (MeOH in DCM 0 to 10 % over 40 min, 25G, SIHP, 30 microns, 25 mL/min). R*f* = 0.2 (95/5 DCM/MeOH, v/v, ninhydrin). $^1$H NMR (300 MHz, $CDCl_3$) δ (ppm) 7.39 - 7.25 (m, 5H), 7.09 (d, *J* = 8.5 Hz, 1H), 6.97 (d, *J* = 8.4 Hz, 1H), 6.23 (s, 1H), 5.02 (s, 2H), 4.22 - 4.06 (m, 2H), 4.02 - 3.85 (m, 2H), 2.76 (t, *J* = 6.4 Hz, 4H), 2.71 - 2.65 (m, 2H), 2.48 (t, *J* = 7.6 Hz, 2H), 1.61 (dd, *J* = 14.9, 6.9 Hz, 2H), 1.55 (dd, *J* = 6.8, 3.9 Hz, 2H), 0.78 (t, *J* = 6.3 Hz, 4H), 0.00 (s, 6H). $^{13}$C NMR (75 MHz, $CDCl_3$) δ (ppm) 153.2, 151.5, 145.5, 136.4, 128.6, 128.1, 127.4, 123.5, 120.8, 100.8, 70.7, 65.7, 57.4, 52.3, 40.0, 36.9, 27.7, 25.8, 12.6, -3.3. HRMS (ESI$^+$): m/z calcd for $[C_{25}H_{37}N_2O_3Si]^+$ 441.2573, found 441.2585.

**(*E*)-6-(4-(4,4-dimethyl-1,4-azasilinan-1-yl)butyl)-3-hydroxypicolinaldehyde oxime** - **223**

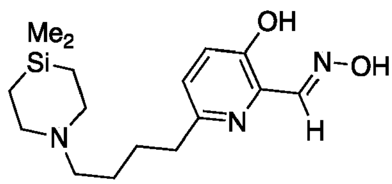

Alkane 222 (360 mg, 0.82 mmol, 1.0 equiv.) was dissolved in MeOH (8 mL) and the solution was degassed with Argon. $Pd(OH)_2/C$ 20% (88 mg, 0.16 mmol, 0.2 equiv.) was added, the mixture was stirred for 2 h at rt under hydrogen. The reaction mixture was filtered over celite, solvents were removed under vacuum. The crude material was used for the next step. A mixture of formic acid and water (16 mL 1:1) was added to acetal and heated for 18 hours at 60 °C. The reaction mixture was neutralized by a saturated aqueous solution of $NaHCO_3$ until neutral and extracted with DCM (4 x 30 mL), dried over $MgSO_4$ and concentrated. The crude material was used for the next step. $NaHCO_3$ (277 mg, 3.29 mmol, 4 equiv.) and NH2OH.HCl (143 mg, 2.06 mmol, 2.5 equiv.) were added to a mixture of crude aldehyde in MeOH and stirred for 18 hours. The reaction mixure was concentrated, and washed with water and extracted with DCM (3x 25 mL), dried over $MgSO_4$ and concentrated. Purified by flash chromatography (10 to 40% MeOH in DCM over 50 min, 12G SiHP, 30 microns, 20mL/min) to afford the title compound as an oil (155 mg, 58%). Dissolved in AcOEt, 0.47 mL of 1M MsOH in AcOEt (961 mg of MsOH in 10 mL of AcOEt) was added, concentrated and dried in high vacuum to afford the mesylate salt.

$^1$H NMR (300 MHz, MeOD) δ 8.11 (s, 1H), 7.14 (d, *J* = 8.5 Hz, 1H), 7.03 (d, *J* = 8.5 Hz, 1H), 3.45 (q, *J* = 9.6, 8.1 Hz, 2H), 2.94 (t, *J* = 7.7 Hz, 2H), 2.63 (td, *J* = 8.4, 7.0, 5.0 Hz, 2H), 2.51 (s, 3H), 1.66 - 1.47 (m, 4H), 0.90 (t, *J* = 5.5 Hz, 4H), 0.16 - -0.16 (m, 6H). $^{13}$C NMR (75 MHz, MeOD) δ 152.6, 152.1, 150.8, 135.0, 125.0, 124.2, 55.6, 52.2, 38.1, 35.3, 26.5, 23.3, 10.0, -4.8, -6.1. HRMS (ESI$^+$): m/z calcd for $[C_{16}H_{28}N_3O_2Si]^+$ 322.1951, found 322.1949. HPLC (Column BEH C18 100*2.1 mm, 1.7 μm, $H_2O$ formic acid 0.1%/MeCN, 5 to 100% over 10 min, 0.5 mL/min) : $t_R$ = 3.80 min (purity = 90.5%).

## Example 2: Biological activity of the compounds of the invention

### Materials and methods

#### 1. *h*BChE and *h*AChE Preparation.

Recombinant *h*BChE and *h*AChE were produced and purified as previously described (Zueva et al., Neuropharmacology, 2019. 155: p. 131-141; Brazzolotto et al., FEBS J, 2012. 279(16): p. 2905-16).

#### 2. Chemicals.

VX, sarin and tabun have been supplied by DGA maîtrise NRBC (Vert le Petit, France). 2-PAM and HI6 were from Pharmacie Centrale des Armées (Orléans, France) and other chemicals were purchased from Sigma-Aldrich.

#### 3. Inhibition of *h*BChE and *h*AChE.

Stock solutions of OPNA at 5 mM in isopropanol were used to inhibit the purified *h*BChE and *h*AChE as previously described (Carletti et al., J Am Chem Soc, 2008. 130(47): p. 16011-20). Briefly, a ten-fold excess of OPNA was used to perform the inhibition of enzymes in a sodium phosphate buffer (100 mM, pH 7.4, 0.1 % BSA) at 25°C. Complete inhibition of enzymes was monitored by measuring the residual activity with a modified Ellman assay as previously described (Ellman et al., Biochem Pharmacol, 1961. 7: p. 88-95) and excess of OPNA were removed by using a desalting PD-10 column (GE Healthcare).

#### 4. IC50 Measurements.

Oximes were dissolved in water to prepare 40 mM stock solutions. Recombinant *h*BChE and *h*AChE activity was measured spectrophotometrically at 25°C, monitoring the absorbance at 412 nm, in 1 mL of Ellman's buffer (0.5 mM DTNB, 0.1% BSA, 0.1 M phosphate, pH 7.4), in the presence of appropriate oxime concentrations. Measurements were performed at least in duplicate for each concentration tested. The oxime concentration producing 50% inhibition was determined by nonlinear fitting with ProFit (Quantumsoft) using the standard IC 50 equation: % activity = $100 \times IC50/(IC50 + [Ox])$.

#### 5. Oxime reactivation of OP-inhibited *h*BChE/*h*AChE.

The ability of oximes to reactivate OP-inhibited *h*BChE and optionnaly *h*AChE were assessed with a modified Ellman assay using a microplate reader (SPARK 10M, Tecan) described previously (Kitz et al.. Biochem Pharmacol, 1965. 14(10): p. 1471-7; Worek et al., Biochem Pharmacol, 2004. 68(11): p. 2237-48) with minor modifications. Briefly, the desired oximes concentrations to be tested were dispensed in a 96-well flat-bottomed polystyrene microplate containing 0.1% BSA phosphate buffer and DTNB. At t=0, OP-inhibited *h*AChE and ATCh or OP-inhibited *h*BChE and BTCh diluted in 0.1 % BSA phosphate buffer were injected in each well containing oximes using the built-in injectors of the microplate reader to a final volume of 200 μL. ATCh or BTCh hydrolysis was continuously monitored over 30 minutes and the increase of absorbance at 412 nm recorded every 10 seconds at 25°C. Activities were individually corrected for oxime-induced hydrolysis of ATCh or BTCh.

Reactivation of OP-inhibited enzyme by oximes proceeds according to scheme 1 and kinetics of oximes reactivation were determined as previously described *(ibid.).* For each oxime concentration, the apparent reactivation rate, kobs, the dissociation constant, $K_D$ and the reactivation rate constant, $k_r$, were calculated by nonlinear fitting with ProFit (Quantumsoft) using the following standard oxime-concentration-dependent reactivation equation (1):

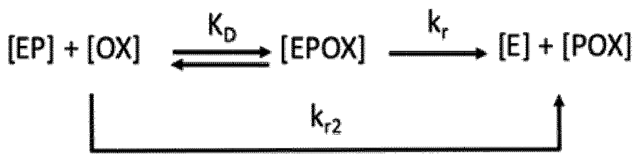


and the following equation:

$$k_{obs} = \frac{k_r[OX]}{K_D + [OX]}$$

When [OX] « $K_D$, Eq (1) simplifies to Eq (2):

$$k_{obs} = \left(\frac{k_r}{K_D}\right)[OX]$$

The second order reactivation rate constant $k_{r2}$, describing the specific reactivity can be derived from Eq (2):

$$k_{r2} = \frac{k_r}{K_D}$$

For the continuous method of recording OP-inhibited *h*AChE/*h*BChE reactivation by oximes, the velocity of substrate hydrolysis ($v$) is proportional to the concentration of the reactivated *h*AChE/*h*BChE and is expressed and derived as equation 4 and 5 respectively. $v_t$ is the velocity at time t and $v_0$ represents the maximum velocity. Equation 5 was used to determine the $k_{obs}$ by non-linear regression analysis for each individual oxime concentration with ProFit (Quantumsoft).

Eq (4): $$v_t = v_0\,(1 - e^{-k_{obs}\,t})$$

Eq (5) $$-d[S] = \int_0^t v\,dt = v_0 t + \frac{v_0}{k_{obs}}\,(e^{-k_{obs}t} - 1)$$

**6. Reactivation kinetics for newly synthesize compounds**

Results are presented in the following tables 1 and 2. HI-6 is asoxime chloride and 2-PAM is pralidoxime, both references outside the invention.

Table 1: Reactivation of OP-inhibited *h*BChE by oximes

| OP | Oxime | $k_r$ (min$^{-1}$) | $K_D$ (μM) | $k_{r2}$ (mM$^{-1}$.min$^{-1}$) |
|---|---|---|---|---|
| VX | HI-6 | 0.03 ± 0.002 | 307 ± 41 | 0,1 |
| | AB-746 | 0,065 ± 0,0009 | 0,4 ± 0,05 | 175 |
| | AB-739 | 0,09 ± 0,0013 | 81 ± 7 | 1 |
| | AB-639 | 0,14 ± 0,01 | 117 ± 22 | 1 |
| | AB-745 | 0,6 ± 0,06 | 253 ± 36 | 2,4 |
| Sarin | HI-6 | 0,3 ± 0,02 | 409 ± 71 | 0,7 |
| | AB-746 | 0,07 ± 0,009 | 0,27 ± 0,04 | 259 |
| | AB-745 | 0,16 ± 0,003 | 30 ± 2 | 5,3 |
| Paraoxon | 2-PAM | 0,35 ± 0,02 | 65 ± 22 | 5,4 |
| | HI-6 | Ø | Ø | Ø |
| | AB-746 | 0,045 ± 0,0006 | 0,15 ± 0,03 | 300 |
| | AB-639 | 0,07 ± 0,001 | 8 ± 1 | 9 |

Table 2: IC50 of oxime on *h*BChE

| Oxime | ICso (μM) |
|---|---|
| 2-PAM | 10 % inhibition at 5 mM |

(continued)

| Oxime | ICso (μM) |
|---|---|
| HI-6 | 6345 ± 515 |
| AB-746 | 19 ± 2 |
| AB-739 | 1297 ± 131 |
| AB-639 | 1776 ± 137 |
| AB-745 | 80 ± 8 |

## Claims

**1.** Compound of following formula (I):

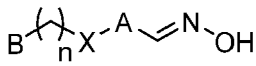
(I)

wherein:

n is an integer from 1 to 6;
at least one of the carbon atoms of

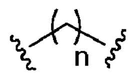

being optionally replaced by an atom chosen from nitrogen and oxygen;
X is a single bond or chosen from -O-, -S- and -NH-;
A is chosen from the group comprising arene diyles and 5 to 6 membered heteroarene diyles, said heteroarene being chosen from the group comprising pyridine, thiophene, thiadiazole, oxathiazole, in particular pyridine;
A is optionally substituted by at least one group R chosen from -OH, $C_1$-$C_6$ alkyl, -O-$C_1$-$C_6$ alkyl, -halogen, notably -Cl, -Br, -F, in particular -OH;
B is chosen from heterocyclic groups with 4 to 10 carbon atoms comprising at least one nitrogen atom, and heteroaryl groups chosen from indole, pyrrazole, imidazole, oxazole, thiazole, oxadiazole and thiadiazole,
the cycle B is optionally fused with at least an arene, in particular a benzene, to form a polycycle B';
the cycle B or B' is optionally substituted by at least one group Z chosen from $C_1$-$C_6$ alkyl, in particular methyl or ethyl; O-$C_1$-$C_6$ alkyl, in particular -OMe; aryl, in particular phenyl; heteroaryl, in particular pyridyl, pyrimidinyl; benzyl; benzhydryl; and -$NR_aR_b$ groups, wherein $R_a$ and $R_b$ are independently chosen from H and $C_1$-$C_6$ alkyls, Ra and Rb being in particular H;
or a stereoisomeric form, a mixture of stereoisomeric forms or a pharmaceutically acceptable salt or solvate form thereof,
for use as a reactivator of human or animal butyrylcholinesterase, in particular human butyrylcholinesterase for the treatment or prevention of an intoxication with at least one organophosphorus nerve agent, said butyrylcholinesterase being prior to treatment or after prevention inhibited by at least one organophosphorus nerve agent.

**2.** Compound for use according to claim 1, of following formula (Ic):

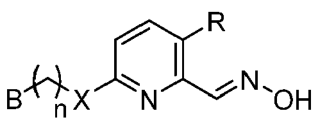
(Ic),

wherein B, n, X and R are as defined in claim 1, R being in particular -OH.

**3.** Compound for use according to any one of preceding claims, wherein n is 4 or 5.

**4.** Compound for use according to any one of preceding claims, wherein cycle B is chosen from pyrrolidine, piperidine, azepane, azocane, azonane, piperazine, thiomorpholine and morpholine, said cycle B being optionally substituted and/or fused as defined in claim 1, in particular substituted as defined in claim 1.

**5.** Compound for use according to any one of claims 1 to 3, wherein cycle B is chosen from indole, pyrrazole, imidazole, oxazole, thiazole, oxadiazole and thiadiazole, said cycle B being optionally substituted and/or fused as defined in claim 1.

**6.** Compound for use according to any one of claims 1 to 3, wherein cycle B is fused with a benzene, to form in particular a tetrahydroisoquinoline, more particularly an unsubstituted tetrahydroisoquinoline.

**7.** Compound for use according to any one of claims 1 to 3, wherein B represents - $NR_1R_2$ being a residue chosen from:

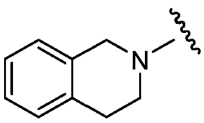, 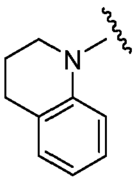, 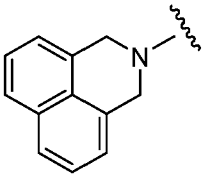, 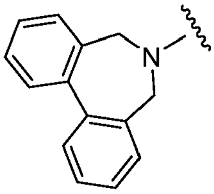, 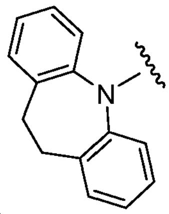,

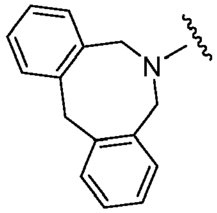, 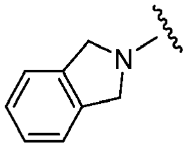, 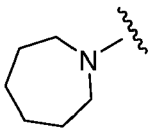, 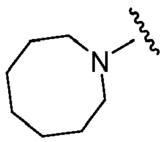, 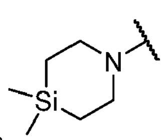, 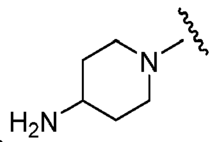,

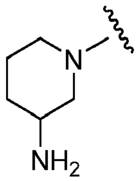, 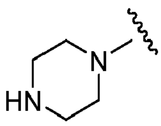, 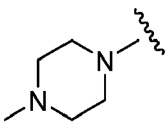, 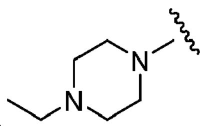, 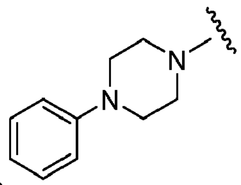,

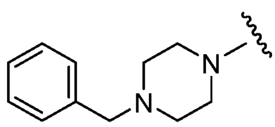, 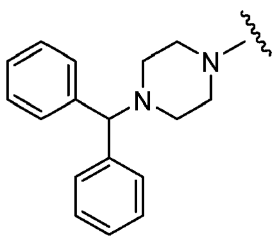, 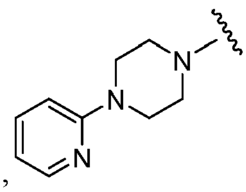 and 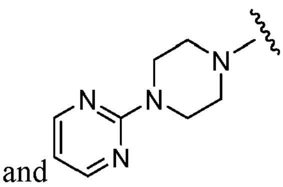.

**8.** Compound for use according to claim 1, wherein said compound is chosen from:

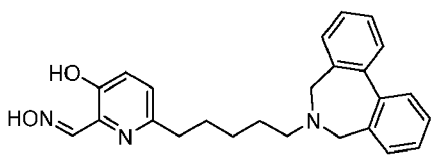, 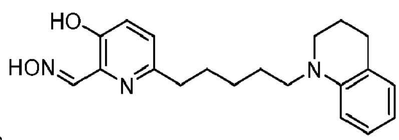,

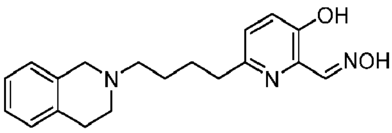, 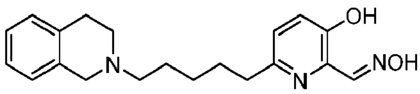,

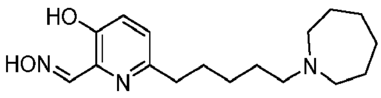,

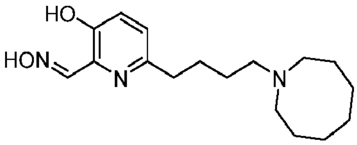, 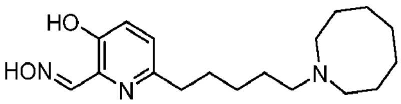,

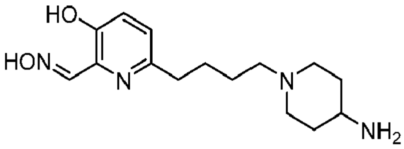, 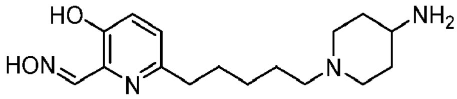,

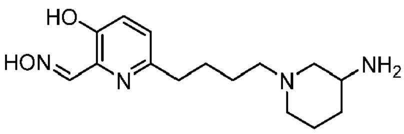, 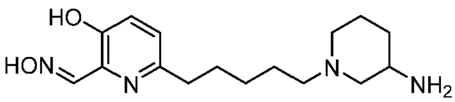,

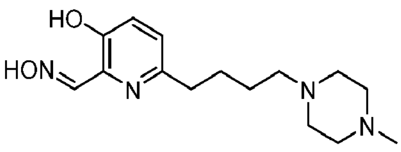, 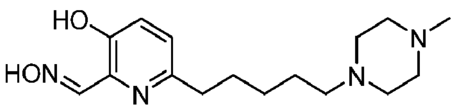,

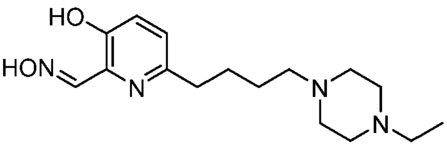, 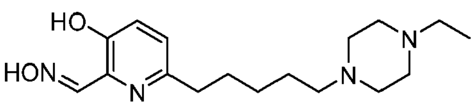,

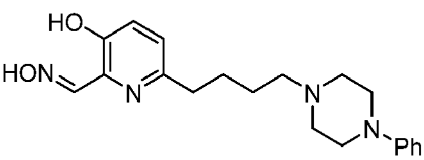, 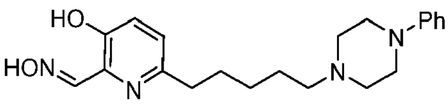,

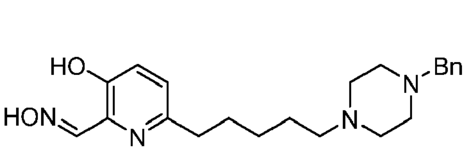, 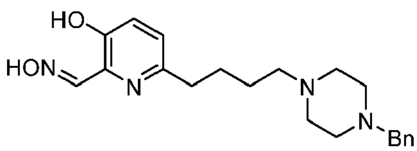,

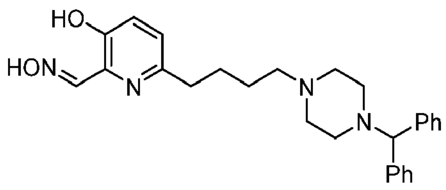,
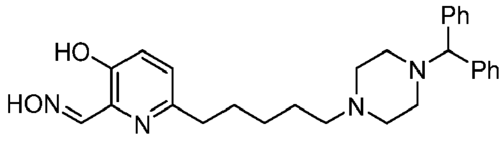,

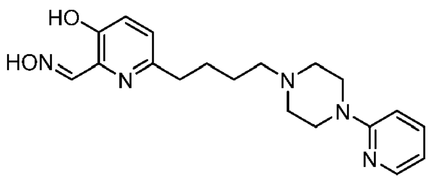,
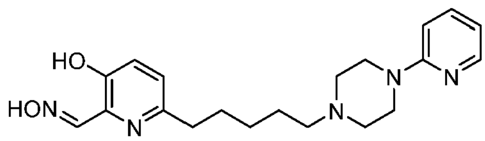,

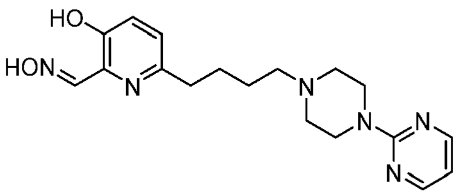,
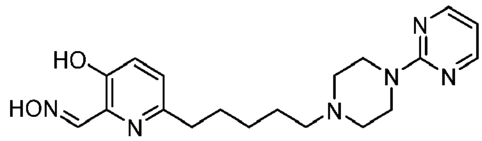

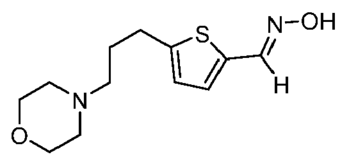,
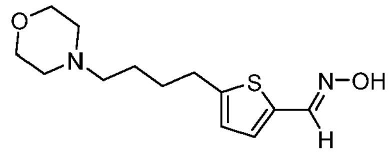,

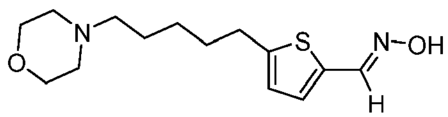,
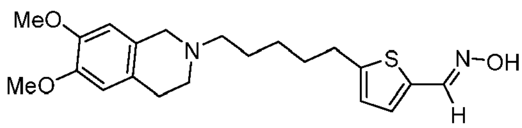,

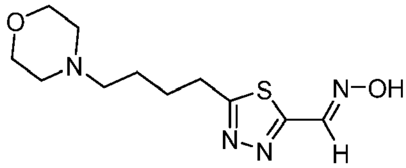,
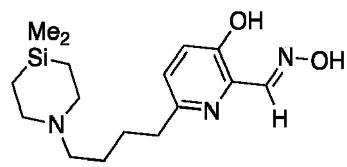,

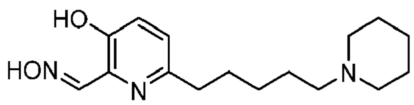,

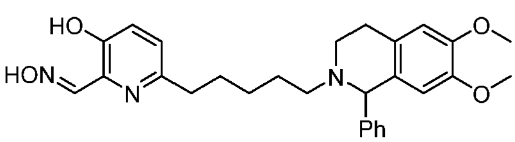,
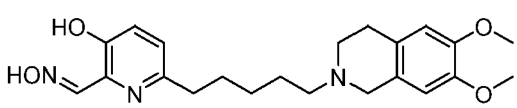,

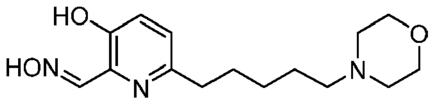 , 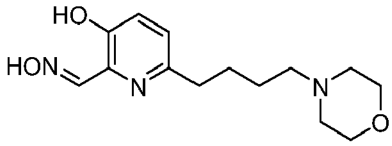 ,

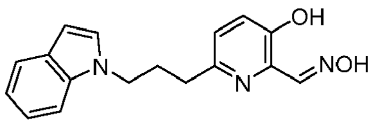 .

**9.** Compound for use according to any one of preceding claims, wherein said organophosphorus nerve agent is selected from warfare agents such as Tammelin esters including *O*-ethyl-*S*-[2-(diisopropylamino)ethyl]methylphosphonothioate (VX), *O*-Ethyl-*S*-2-(diisopropylamino)ethylethylphosphonothiolate (VS), amiton (VG), 2-[ethoxy(ethyl)phosphoryl]sulfanyl-*N*,*N*-diethylethanamine (VE), edemo (VM), *N,N*-diethyl-2-(methyl-(2-methylpropoxy)phosphoryl)sulfanylethanamine (VR) and *O*-cyclopentyl *S*-(2-diethylaminoethyl) methylphosphonothiolate (EA-3148); tabun; sarin; cyclosarin; soman; Novichok agents; and pesticides such as paraoxon, parathion, tetraethyl pyrophosphate (TEPP), dichlorvos, phosmet, malathion, fenitrothion, methyl parathion, tetrachlorvinphos, chlorpyrifos, azamethiphos, diazinon, azinphos-methyl, terbufos.

**10.** Compound of following formula (II):

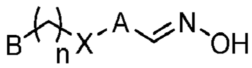 (II)

wherein:

n is an integer from 1 to 6;
at least one of the carbon atoms of

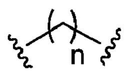

being optionally replaced by an atom chosen from nitrogen and oxygen;
X is a single bond or chosen from -O-, -S- and -NH-;A is chosen from the group comprising arene diyles and 5 to 6 membered heteroarene diyles, said heteroarene being chosen from the group comprising pyridine, thiophene, thiadiazole, oxathiazole, in particular pyridine;
A is optionally substituted by a group R chosen from -OH, $C_1$-$C_6$ alkyl, -O- $C_1$-$C_6$ alkyl, -halogen, notably -Cl, -Br, -F, in particular -OH;
B is chosen from:

- -$NR_1R_2$ groups wherein $R_1$ and $R_2$ form together with the nitrogen to which they are attached a cycle B chosen from the heterocyclic groups with 4 to 10 carbon atoms; and
- heteroaryl groups chosen from pyrrazole, imidazole, oxazole, thiazole, oxadiazole and thiadiazole;

the cycle B is optionally fused with at least an arene, in particular a benzene, to form a polycycle B';
the cycle B or B' is optionally substituted by at least one group Z chosen from $C_1$-$C_6$ alkyl, in particular methyl or ethyl; O-$C_1$-$C_6$ alkyl, in particular -OMe; aryl, in particular phenyl; heteroaryl, in particular pyridyl, pyrimidinyl; benzyl; benzhydryl; and -$NR_aR_b$ groups, wherein $R_a$ and $R_b$ are independently chosen from H and $C_1$-$C_6$ alkyls, Ra and Rb being in particular H;
with the proviso that, in particular when A is a pyridine:

- cycle B is fused and/or substituted when said cycle B is a piperidine;
- cycle B' is not substituted by one or more O-$C_1$-$C_6$ alkyl groups when B' represents a tetrahydroisoquinoline;

or a stereoisomeric form, a mixture of stereoisomeric forms or a pharmaceutically acceptable salt or solvate form thereof,
for use in the treatment or prevention of a nervous and/or respiratory failure due to intoxication with at least one organophosphorus nerve agent.

**11.** A pharmaceutical composition comprising a compound of formula (II) as defined in claim 10 in admixture with at least one pharmaceutically acceptable excipient.

**12.** A compound of formula (II) as defined in claim 10.

**13.** Kit comprising a butyrylcholinesterase, in particular a human butyrylcholinesterase, notably selected from wild-type, recombinent, or synthetic butyrylcholinesterase enzymes, and their variants, incuding peptidomimetics, and a compound of formula (I) as defined in claim 1 or a pharmaceutical composition as defined in claim 11.

**14.** Kit comprising a butyrylcholinesterase, in particular a human butyrylcholinesterase, and a compound of formula (I) as defined in claim 1 or a pharmaceutical composition as defined in claim 11, for simultaneous, sequential or separate use in the treatment or prevention of a nervous and/or respiratory failure due to intoxication with at least one organophosphorus nerve agent.

**15.** Compound of formula (I) as defined in claim 1 or a pharmaceutical composition as defined in claim 11 for use as an in vivo reactivator of human or animal butyrylcholinesterase, in particular human butyrylcholinesterase, said butyrylcholinesterase being prior to use inhibited by at least one organophosphorus nerve agent.

**16.** Use of a compound of formula (I) as defined in claim 1 as an in vitro or ex vivo reactivator of human or animal butyrylcholinesterase, in particular human butyrylcholinesterase, said butyrylcholinesterase being prior to use inhibited by at least one organophosphorus nerve agent.

Europäisches Patentamt
European Patent Office
Office européen des brevets

# EUROPEAN SEARCH REPORT

Application Number
EP 20 30 5860

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/076986 A1 (CENTRE NAT RECH SCIENT [FR]) 25 April 2019 (2019-04-25) * claim 1 * ----- | 1-16 | INV. C07D401/06 C07D401/14 C07D409/06 C07D417/06 A61K31/44 A61K31/425 A61K31/433 A61P25/00 |
| X | ONDREJ SOUKUP ET AL: "In vitro and in silico Evaluation of Non-Quaternary Reactivators of AChE as Antidotes of Organophosphorus Poisoning - a New Hope or a Blind Alley?", MEDICINAL CHEMISTRY, vol. 14, no. 3, 5 April 2018 (2018-04-05), pages 281-292, XP055751419, NL ISSN: 1573-4064, DOI: 10.2174/1573406414666180112105657 * figure 1 * ----- | 1-16 | |
| X | JULIEN RENOU ET AL: "Syntheses and in vitro evaluations of uncharged reactivators for human acetylcholinesterase inhibited by organophosphorus nerve agents", CHEMICO-BIOLOGICAL INTERACTIONS., vol. 203, no. 1, 1 March 2013 (2013-03-01), pages 81-84, XP055220523, IR ISSN: 0009-2797, DOI: 10.1016/j.cbi.2012.09.023 * examples 2,3,4,7 * ----- | 1-16 | TECHNICAL FIELDS SEARCHED (IPC) C07D |
| | -/-- | | |

The present search report has been drawn up for all claims

1

| Place of search | Date of completion of the search | Examiner |
|---|---|---|
| Munich | 19 November 2020 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 30 5860

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JULIEN RENOU ET AL: "Tryptoline-3-hydroxypyridinaldoxime conjugates as efficient reactivators of phosphylated human acetyl and butyrylcholinesterases", CHEMICAL COMMUNICATIONS, vol. 50, no. 30, 1 January 2014 (2014-01-01), pages 3947-3950, XP055751440, ISSN: 1359-7345, DOI: 10.1039/C4CC00561A * the whole document * ----- | 1-16 | |
| X | JULIEN RENOU ET AL: "Synthesis and in vitro evaluation of donepezil-based reactivators and analogues for nerve agent-inhibited human acetylcholinesterase", RSC ADVANCES, vol. 6, no. 22, 1 January 2016 (2016-01-01), pages 17929-17940, XP055751746, GB ISSN: 2046-2069, DOI: 10.1039/C5RA25477A * example 2; table 1 * ----- | 1-16 | TECHNICAL FIELDS SEARCHED (IPC) |
| X | RABINDRANATH LO ET AL: "In Silico Studies in Probing the Role of Kinetic and Structural Effects of Different Drugs for the Reactivation of Tabun-Inhibited AChE", PLOS ONE, vol. 8, no. 12, 2 December 2013 (2013-12-02), page e79591, XP055751743, DOI: 10.1371/journal.pone.0079591 * figure 11 * ----- -/-- | 1-16 | |

The present search report has been drawn up for all claims

1

| Place of search | Date of completion of the search | Examiner |
|---|---|---|
| Munich | 19 November 2020 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 30 5860

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAMARA ZORBAZ ET AL: "Pharmacokinetic Evaluation of Brain Penetrating Morpholine-3-hydroxy-2-pyridine Oxime as an Antidote for Nerve Agent Poisoning", ACS CHEMICAL NEUROSCIENCE, vol. 11, no. 7, 1 April 2020 (2020-04-01), pages 1072-1084, XP055751758, US ISSN: 1948-7193, DOI: 10.1021/acschemneuro.0c00032 * Experimental section; figure 2 * ----- | 1-16 | |
| X | GUILLAUME MERCEY ET AL: "First efficient uncharged reactivators for the dephosphylation of poisoned human acetylcholinesterase", CHEMICAL COMMUNICATIONS, vol. 47, no. 18, 1 January 2011 (2011-01-01), page 5295, XP055099219, ISSN: 1359-7345, DOI: 10.1039/c1cc10787a * figure 3 * ----- | 1-16 | TECHNICAL FIELDS SEARCHED (IPC) |
| X | GUILLAUME MERCEY ET AL: "Phenyltetrahydroisoquinoline-Pyridinaldoxime Conjugates as Efficient Uncharged Reactivators for the Dephosphylation of Inhibited Human Acetylcholinesterase", JOURNAL OF MEDICINAL CHEMISTRY, vol. 55, no. 23, 13 December 2012 (2012-12-13), pages 10791-10795, XP055099217, ISSN: 0022-2623, DOI: 10.1021/jm3015519 * table I * ----- | 1-16 | |

1

The present search report has been drawn up for all claims

| Place of search | Date of completion of the search | Examiner |
|---|---|---|
| Munich | 19 November 2020 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 30 5860

This annex lists the patent family members relating to the patent documents cited in the above-mentioned European search report. The members are as contained in the European Patent Office EDP file on
The European Patent Office is in no way liable for these particulars which are merely given for the purpose of information.

19-11-2020

| Patent document cited in search report | Publication date | Patent family member(s) | Publication date |
|---|---|---|---|
| WO 2019076986 A1 | 25-04-2019 | EP 3473618 A1 | 24-04-2019 |
| | | EP 3697769 A1 | 26-08-2020 |
| | | US 2020255399 A1 | 13-08-2020 |
| | | WO 2019076986 A1 | 25-04-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015077317 A
- EP 2556057 A


### Non-patent literature cited in the description


- **ELIEL, E. L. ; WILEN, S.H.** Stereochemistry of Organic Compounds. Wiley, 1994
- **JACQUES, J et al.** Enantiomers, Racemates, and Resolutions. Wiley, 1981
- **R.C. LAROCK.** Comprehensive Organic Transformations. Wiley-VCH Publishers, 1999
- **T.W. GREENE ; P. G. M. WUTS.** Protective Groups in Organic Chemistry. John Wiley and Sons, 1999
- **J. F. W. MCOMIE.** Protective Groups in Organic Chemistry. Plenum Press, 1973
- **ZUEVA et al.** *Neuropharmacology,* 2019, vol. 155, 131-141
- **BRAZZOLOTTO et al.** *FEBS J,* 2012, vol. 279 (16), 2905-16
- **CARLETTI et al.** *J Am Chem Soc,* 2008, vol. 130 (47), 16011-20
- **ELLMAN et al.** *Biochem Pharmacol,* 1961, vol. 7, 88-95
- **KITZ et al.** *Biochem Pharmacol,* 1965, vol. 14 (10), 1471-7
- **WOREK et al.** *Biochem Pharmacol,* 2004, vol. 68 (11), 2237-48